# EUROPEAN PATENT SPECIFICATION

(11) **EP 0 452 101 B1**
(45) Date of publication and mention of the grant of the patent: **07.07.1999**
(21) Application number: 91303135.7
(22) Date of filing: 09.04.1991
(51) Int. Cl.: C07D 498/20, C07D 498/10, C07D 513/20, C07D 513/10, A61K 31/435

(54) **Spiro bridged and unbridged heterocyclic compounds**
Ueberbrückte und nicht-überbrückte heterocyclische Spiroverbindungen
Composés spiro hétérocycliques pontés et non pontés

(30) Priority: 10.04.1990 US 507708; 10.04.1990 US 507228
(43) Date of publication of application: 16.10.1991
(73) Proprietor: ISRAEL INSTITUTE FOR BIOLOGICAL RESEARCH, Ness Ziona (IL)
(72) Inventor: Fisher, Abraham, Holon (IL); Segall, Yoffi, Ramat Hasharon (IL); Shirin, Ezra, Tel Aviv (IL); Karton, Yishai, Ness Ziona (IL); Meshulam, Haim, Bat Yam (IL)
(74) Representative: Silverman, Warren

(56) References cited:
- EP-A- 0 205 247
- EP-A- 0 337 547
- EP-A- 0 350 118
- US-A- 3 629 276
- J. CHEM. SOC. (B) no. 6, 1971, pages 1308 - 1315 R.A.Y. JONES ET AL

## Description

The present invention relates to 4- and 5-spiro(1,3-oxazolines and 1,3-thiazolines) in which the ring which is spiro-connected to oxazoline or thiazoline is a saturated heterocyclic bridged or unbridged ring containing one nitrogen atom and to pharmaceutical compositions containing the spiro compounds. These compounds are potentially useful for treating diseases of the central and peripheral nervous system using such spirocompounds or pharmaceutical compositions.

Novel spiro-quinuclidine compounds, in which oxathiolane rings were connected in spiro manner with quinuclidine rings, were described e.g. in European Patent Application No. 0205247 A2, published December 17, 1986, and in U.S. Patents Nos. 4,855,290 (issued August 8, 1989), 4,981,858 (issued January 1, 1991), 4,900,830 (issued February 13, 1990) and 4,876,260 (issued October 24, 1989). These novel compounds were found to possess central nervous system activity. The biological activity of the compound 2-methylspiro(1,3-oxathiolane-5',3)quinuclidine, which exists as geometrical cis- and trans-isomers depending upon whether the 2-methyl group is located on the same side of the oxathiolane ring as the quinuclidine ring nitrogen atom (cis) or on the other side of the quinuclidine ring nitrogen atom (trans), was in particular extensively investigated, and it was found on the basis of pre-clinical tests that the cis- compound (code no. AF102B) was especially promising for the control of senile dementia of Alzheimer's type (SDAT). It is also of interest that each of the cis- and trans-isomers may be optically resolved, and the biological activity of the optical isomers was also investigated in a number of cases.

It is a principal object of the invention to provide novel 4- and 5- spiro-1,3-oxazoline and -1,3-thiazoline compounds, which are distinctive from the aforementioned spiro-oxathiolane/quinuclidine compounds. Further objects of the invention, and especially those which relate to the provision of useful pharmaceutical compositions and the treatment of disease, will be apparent from the description which follows.

The present invention provides novel compounds corresponding with the schematic structural formula (I) and (II) 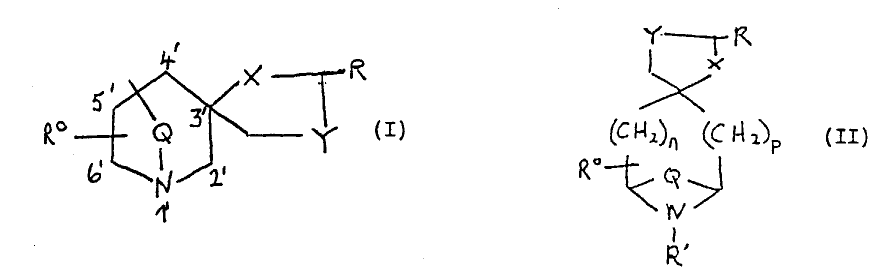 including enantiomers, racemates and acid addition and quaternary salts thereof, wherein in formula (I) Q is -CH₂CH₂- attached to the 1',4' or 1',5' positions of the six-membered ring, and in formula (II) Q is two hydrogen atoms, (CH₂)ₘ or C(CH₃)₂ where m is 1, 2 or 3 and n and p are each independently 0, 1, 2 or 3, provided that n + p = 1-3; R^{o} is hydrogen, methyl or hydroxyl; the moiety 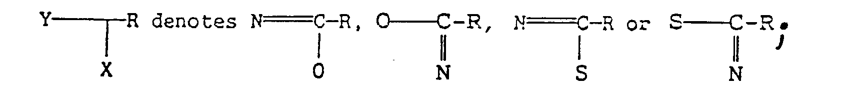 R is selected from hydrogen, C₁₋₆-alkyl, C₂₋₆-alkenyl, C₂₋₆-alkynyl, C₃₋₇- cycloalkyl, C₁₋₆-alkyl substituted by 1-6 halogen atoms, hydroxy- C₁₋₆-alkyl, C₁₋₆-alkoxy, C₁₋₆-alkylthio, C₁₋₆-alkoxy-C₁₋₆-alkyl, carboxy-C₁₋₆-alkyl, (C₁₋₆-alkoxy)carbonyl-C₁₋₆-alkyl, amino-C₁₋₆-alkyl, mono-(C₁₋₆-alkyl)amino-C₁₋₆-alkyl, di-(C₁₋₆-alkyl)amino-C₁₋₆-alkyl, 2-oxo-pyrrolidin-1-yl-methyl, aryl and C₁₋₆-alkyl substituted by one or two aryl groups, with R being additionally selected from NH₂, NH-C₁₋₆ alkyl and N(C₁₋₆alkyl)₂ when R forms part of formula (I); R' is independently selected from the group from which R is selected when R forms part of formula (I), C₁₋₆-alkanoyl and arylcarbonyl; and aryl denotes unsubstituted phenyl or phenyl substituted by 1-3 substituents selected from halogen, C₁₋₆-alkyl, C₁₋₆-alkoxy and CF₃, subject to the provisos (i), (ii) and (iii), namely: (i) in formula II, when Q is two hydrogen atoms, n =p = 1, R^{o} is hydrogen, and R is phenyl, then R' is not tertiary butyl, (ii) in formula II, when Q is two hydrogen atoms, n = p = 1, R^{o} is hydrogen, R is p-chlorophenyl, and R' is tert. butyl, then the moiety 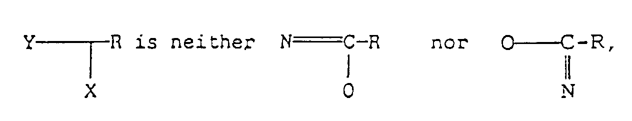 (iii) in formula II, when Q is two hydrogen atoms, n = p = 1, R^{o} is hydrogen, R is 3,5-dichlorophenyl, and R' is tertiary butyl, then the moiety

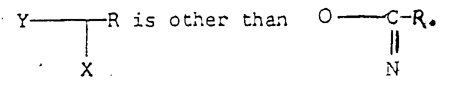

The present spiro compounds, in contrast to those mentioned above, may exhibit optical isomerism, and only in a few cases geometrical isomerism. Optical isomerism will be shown in particular, when R and/or R' is asymmetrical, and/or the spiro-ring containing only a single nitrogen as ring hetero-atom is asymmetrical with respect to the spiro-junction.

Many compounds of formulae (I) and (II) as defined above are new. The definitions of the compounds of formulae (I) and (II) contain a number of provisos to exclude from protection certain compounds which form part of the state of the art. For example US-A-3629276 is known which discloses among others a compound of formula;

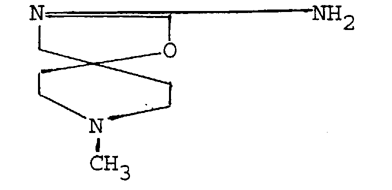

This is the compound of formula (II) in which Q is two hydrogen atoms, n = p = 1, R^{o} is hydrogen, R is NH₂ and R' is methyl, and the moiety 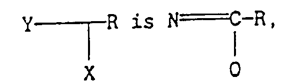 and belongs to a series of compounds tested by Harnden and Rasmussen (J. Med. Chem. 1970, 13: 305-308) for CNS stimulant activity, but was not one of the five compounds selected as being the most active of the series. No information is given to the activity of this compound.

The compounds of formula (II) in which Q is two hydrogen atoms, n = p = 1, R^{o} is hydrogen, R is phenyl, and R' is tertiary butyl, are known, as are those in which Q, n, p, R^{o} and R' have these values and either R is p-chlorophenyl, while the moiety 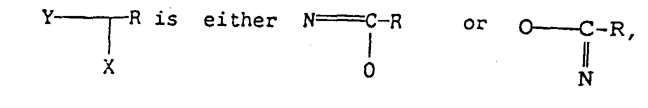 or R is 3,5-dichlorophenyl, while the moiety 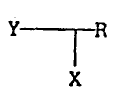 is specifically 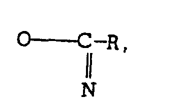 (see Jones et al, J. Chem. Soc. (B) 1308-1315, 1971), but no biological activity was reported for these compounds.

EP 350118 discloses a generic class of spiro-azabicyclic compounds said to be muscarinic agonists, including a sub-class which corresponds with formula (I), above, where, the group Q bridging the 1',4'-position is a methylene group and the moiety 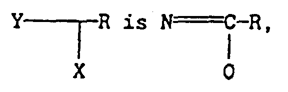 R is hydrogen, C₁₋₄-alkoxy or an optionally substituted saturated or unsaturated hydrocarbon group. EP 350118 does not however describe the chemical or biological properties of any specific member of this sub-class.

It will be appreciated that in the pharmaceutical compositions in accordance with the present invention, the above-stated provisos (i), (ii) and (iii) do not apply.

European Patent Application No. 0337547A1, published October 18, 1989, discloses compounds said to be useful in the treatment of psychotic disorders, presenile and senile dementia, and other physiological conditions, of the following formula: 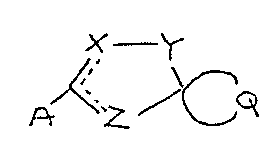 in which the broken line represents an optional bond in one of the two positions, A represents a group of formula 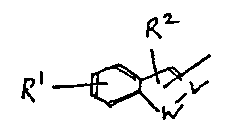 (in which R¹ and R² are independently hydrogen or specified substituents, V is N, 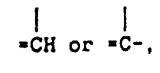 and W is O, S, or NH which may be substituted by specified substituents), Q is the residue of an azacyclic or azabicyclic system, and of X, Y and Z, two are selected independently from O, S and N and the other is C, or Y may be C=O. This reference does not detail results of any biological testing, but states that certain of the disclosed compounds (not specified) demonstrate an affinity for the 5-HT₃ receptors. This reference gives no directions based on biological activity for selecting any particular one of the numerous possible structures represented by permutations of the moieties represented by the symbols Q, X, Y and Z depicted above, in combination with the essential bicyclic radical A.

The bridged-ring in formula (I) is 1-azabicyclo[2,2,2]octane (quinuclidine) or 1-azabicyclo[3,2,1]octane.

Examples of the ring joined in spiro manner to the oxazoline or thiazoline ring in formula (II) are, when Q is two hydrogen atoms, pyrrolidine, piperidine and hexamethyleneimine; and when Q is other than two hydrogen atoms, examples are: 5-azabicyclo[2,1,1]hexane, 6,6,-dimethyl-5-azabicyclo[2,1,1]hexane, 7-azabicyclo[2,2,1]heptane, 8-azabicyclo[3,2,1]octane, 6-aza bicyclo[3,1,1]heptane, 7,7,-dimethyl-6-azabicyclo[3,1,1]heptane, 8-azabicyclo[3,2,1]octane, 9-azabicyclo[3,3,1]nonane, 7-azabicyclo[4,1,1]octane, 8,8,-dimethyl-7-azabicyclo[4,1,1]octane, 9-azabicyclo[4,2,1]nonane, and 10-azabicyclo[4,3,1]decane.

Moreover, as indicated by the symbol R^{o} in formulae (I) and (II), any of these ring systems may be ring-substituted by methyl or OH.

The spiro-compounds provided by the present invention have central and peripheral nervous system activity.

In certain embodiments,each of the symbols in formulae (I) and (II) has the meanings defined above with the following exceptions, namely, that when the moiety

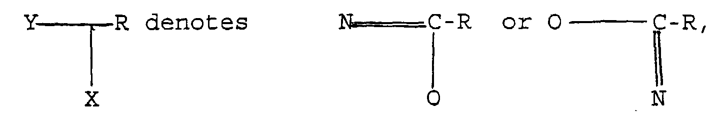

R may not be C₁₋₆-alkoxy or C₁₋₆-alkylthio.

In a presently preferred of compounds of formula (I), R may be hydrogen, NH₂, NH-C₁₋₆alkyl, N(C₁₋₆-alkyl)₂, C₁₋₆-alkyl or aryl, while R' may be hydrogen or methyl. In a presently preferred group of compounds of formula (II), R may be hydrogen or aryl, while R' may be hydrogen or methyl. Without prejudice to the generality of the invention, exemplary of the present compounds are:
2-aminospiro(1,3-oxazoline-5,3')quinuclidine,
2-methylspiro(1,3-ozazoline-5,3')quinuclidine,
2-ethylspiro(1,3-oxazoline-5,3')quinuclidine,
2-phenylspiro(1,3-oxazoline-5,3')quinuclidine,
1-methylpiperidine-4-spiro-4'-(2'-methyl-1',3'-oxazoline),
1-methylpiperidine-4-spiro-4'-(2'-ethyl-1',3'-oxazoline),
2-methylspiro(1,3-oxazoline-5,4')-1'-methylpiperidine,
2-methylspiro(1,3-thiazoline-5,4')-1'-methylpiperidine,
2-ethylspiro(1,3-oxazoline-5,4')-1'-methylpiperidine,
including enantiomers, racemates and acid addition and quaternary salts thereof.

The present invention moreover provides pharmaceutical compositions for use in treating diseases of the central and peripheral nervous system in mammals, which comprise an amount effective for use in treating these diseases, of at least one of the spiro-compounds of the invention. Further, the compounds and pharmaceutical compositions of the invention may be used in methods for treating diseases of the central and peripheral nervous system in mammals, which comprises administering to a mammal an amount effective for use in treating these diseases, of at least one of the spiro-compounds of the invention. As indicated above, provisos (i), (ii) and (iii) do not apply to such compositions and methods.

### DETAILED DESCRIPTION OF THE INVENTION

### Preparation of the oxazoline compounds of formula (I)

The above spiro-compounds of formula (I) may be prepared by a reaction which effects formation of the oxazoline or thiazoline ring. By way of example, suitable starting materials for such cyclization reactions may be prepared in accordance with reaction schemes A1 and B1, below.

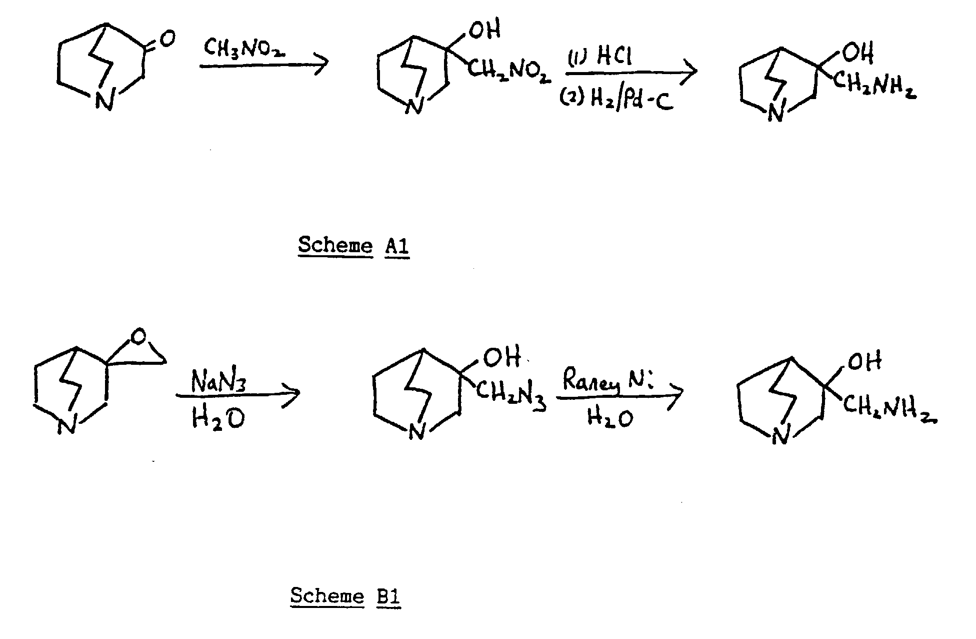

It should be noted that in Scheme B1, reaction of sodium azide with the epoxide of 3-methylenequinuclidine in aqueous solution resulted in formation of a single product, 3-azidomethylquinuclidin-3-ol; addition to the latter of wet active Raney Nickel until the evolution of nitrogen ceased resulted in the formation of the desired compound. The major advantages of the Scheme B1 approach are:
a) there is no need to isolate any intermediates; and
b) no hydrogenation apparatus is needed since the hydrogen content of the catalyst is sufficient.

It has now surprisingly been found that the 3-aminomethyl product of either of these reaction schemes may be readily condensed with a carboxylic acid RCOOH to give the spiro-products, whereas it was previously believed that formation of the oxazoline ring by condensation of amino-alcohol and carboxylic acid would proceed smoothly only when the amino-alcohol is completely substituted on the carbon atom to which the NH₂ group is connected [see "Oxazolines, their Preparation, Reactions and Applications", J.A. Frunp, Chem. Rev. 71, 483-505 (1971)]. The corresponding imidate RC(:NH)-O-alkyl can be alternatively used, in place of the carboxylic acid RCOOH. The reactions affording compounds of formula I when the bridged-ring is, e.g., quinuclidine, may be represented as follows:

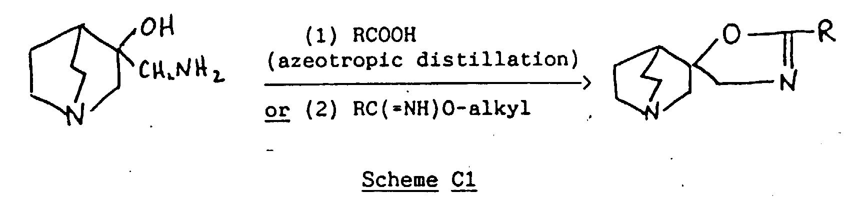

In order to obtain the spiro-quinuclidine compound, for example, when R = NH₂, it is however preferred to react cyanogen bromide with 3-aminomethylquinuclidin-3-ol.

It will be appreciated that while the foregoing methods will be effective to prepare those compounds of the invention in which X is O and Y is N, in order to apply similar methods for the purpose of preparing the corresponding sub-group (a) compounds of the invention in which X is N and Y is O, a suitable starting material would be (illustratively, where the ring spiro-connected to oxazoline is quinuclidine) 3-amino-3-hydroxymethylquinuclidine. This starting material may be prepared, e.g., according to Scheme D1:

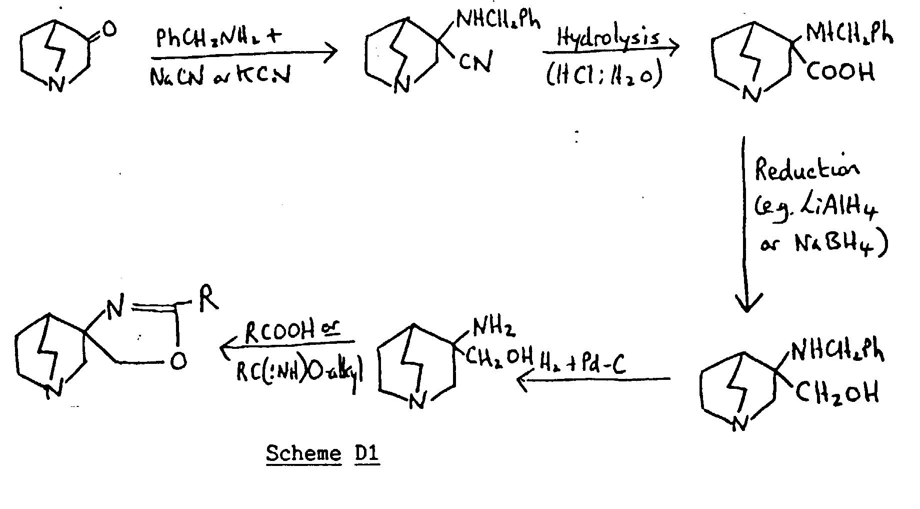

### Preparation of the oxazoline compounds of formula (II)

The above spiro-compounds of formula (II), may be prepared by reacting suitably substituted compounds containing the saturated nitrogen-only ring system with a reactant which will effect formation of the oxazoline ring. Thus, for example, a suitable starting material is 4-aminomethyl-1-methylpiperidin-4-ol, which may be prepared either from 4-nitromethyl-1-methylpiperidin-4-ol e.g. as the HCl salt, as in Scheme A2, or from 4-azidomethyl-1-methylpiperidin-4-ol as in Scheme B2.

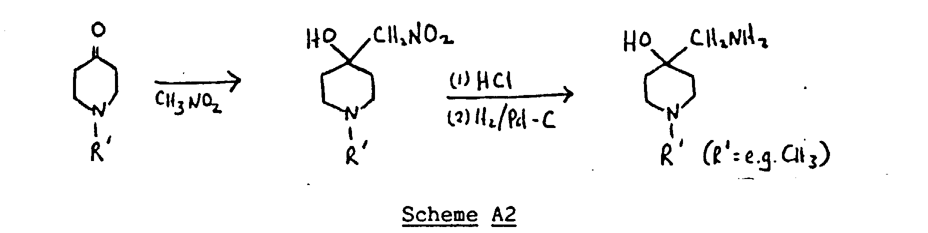

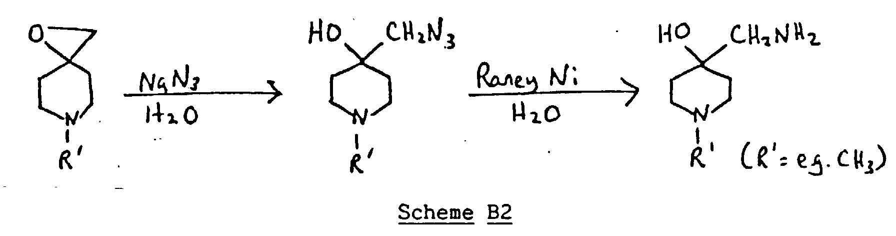

It should be noted that in Scheme B2, reaction of sodium azide with the epoxide of 4-methylene-1-methylpiperidine in aqueous solution results in formation of a single product, 4-azidomethyl-1-methylpiperidin-4-ol, while addition to the latter of wet active Raney Nickel until the evolution of nitrogen ceases results in the formation of the desired compound. The major advantages of the Scheme B2 approach have been noted in connection with Scheme B1, above.

It has now surprisingly been found that the 3-aminomethyl product of either of these reaction schemes may be readily condensed with a carboxylic acid RCOOH to give the spiro-products, whereas it was previously believed that formation of the oxazoline ring by condensation of amino-alcohol and carboxylic acid would proceed smoothly only when the amino-alcohol is completely substituted on the carbon atom to which the NH₂ group is connected [see "Oxazolines, their Preparation, Reactions and Applications", J.A. Frunp, Chem. Rev. 71, 483-505 (1971)]. The corresponding imidate RC(:NH)-O-alkyl can be alternatively used, in place of the carboxylic acid RCOOH.
The reactions affording compounds of formula II may be illustrated (e.g. in the case of spiro oxazoline/piperidines) as follows:

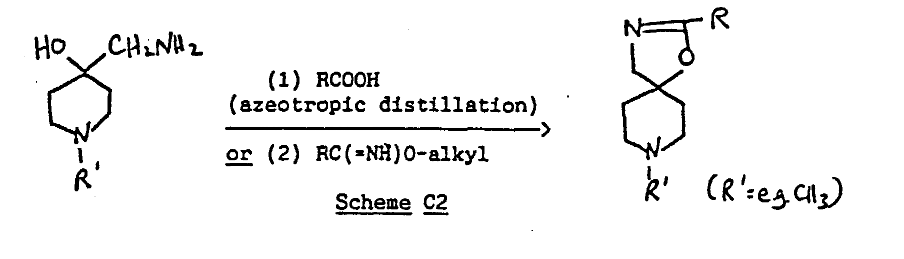

It will be appreciated that while the foregoing methods will be effective to prepare those compounds of the invention in which 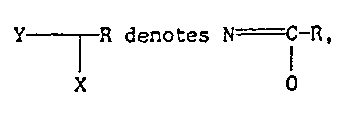 in order to apply similar methods for the purpose of preparing compounds of the invention in which 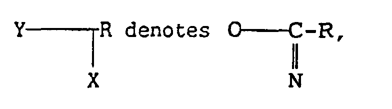 a suitable starting material would be (illustratively, where the ring spiro-connected to oxazoline is piperidine) 4-amino-4-hydroxymethyl-1-methylpiperidine. This starting material and the final product may be prepared, e.g., according to Scheme D2 or E:

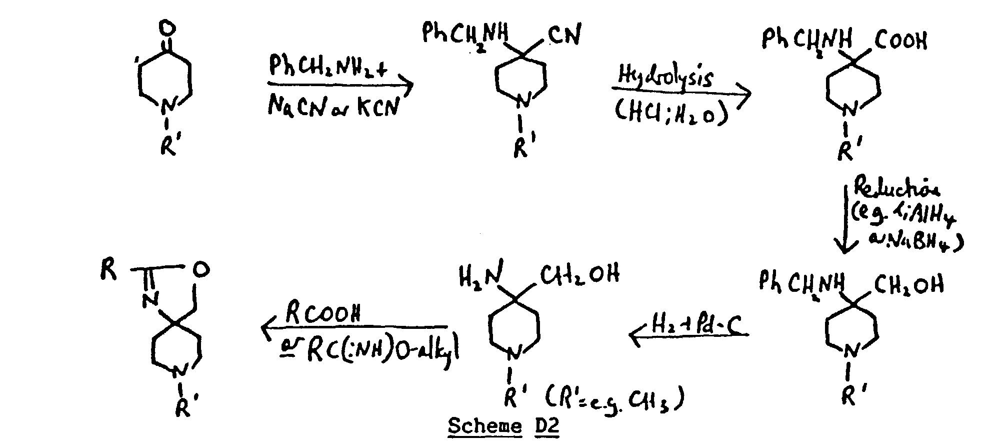

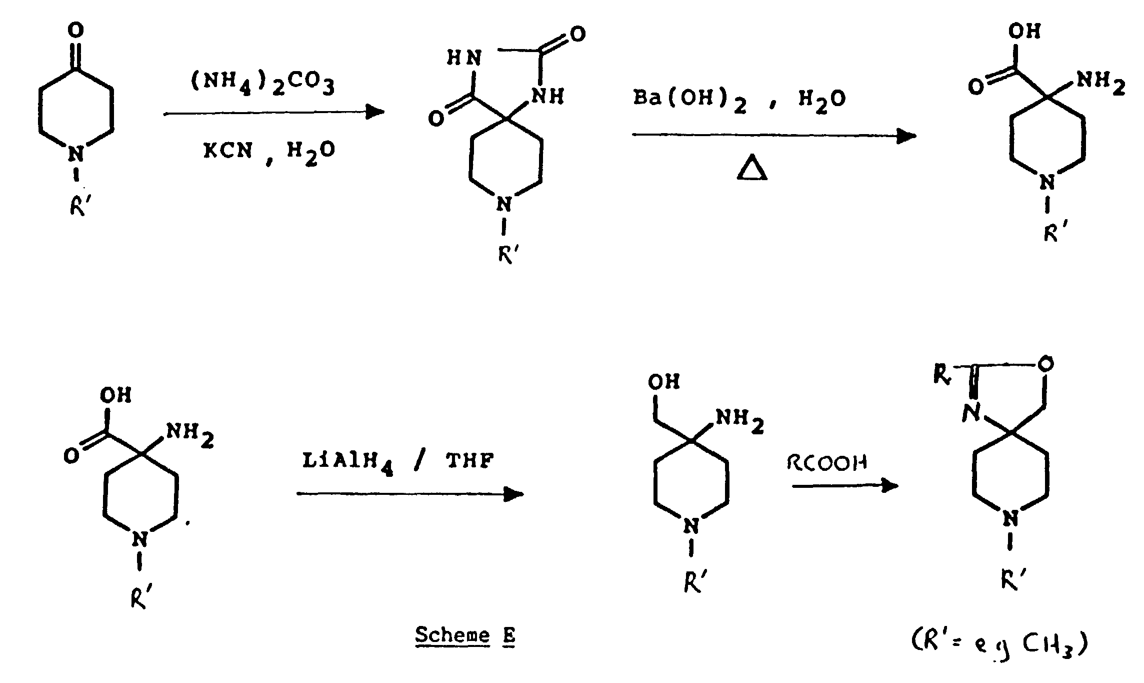

### Preparation of the oxazoline and thiazoline compounds (I) & (II)

When 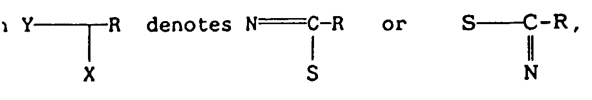 these compounds and the oxazoline analogs can be prepared by the following Schemes F and G, e.g., when the ring spiro connected to thiazoline or oxazoline is piperidine or quinuclidine:

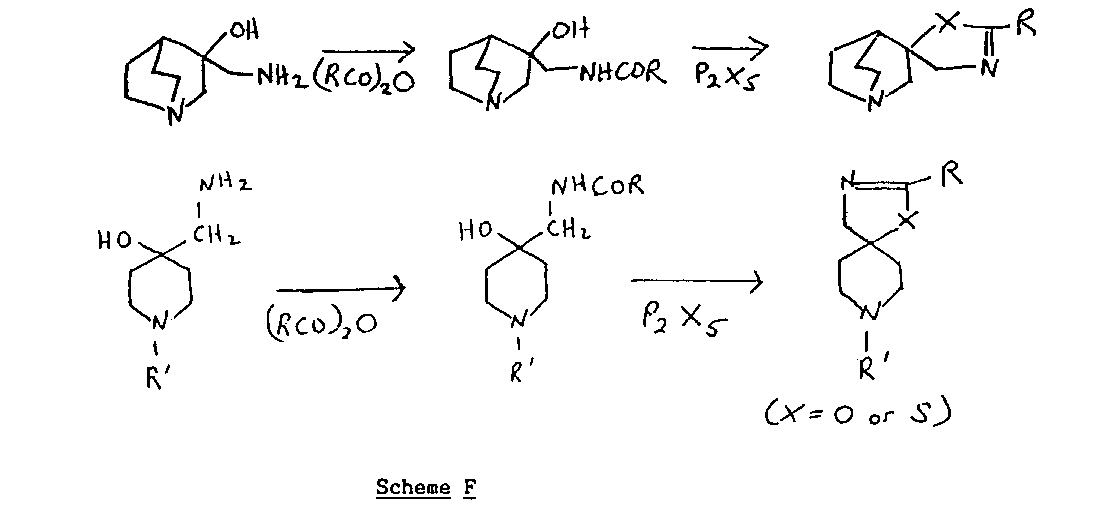

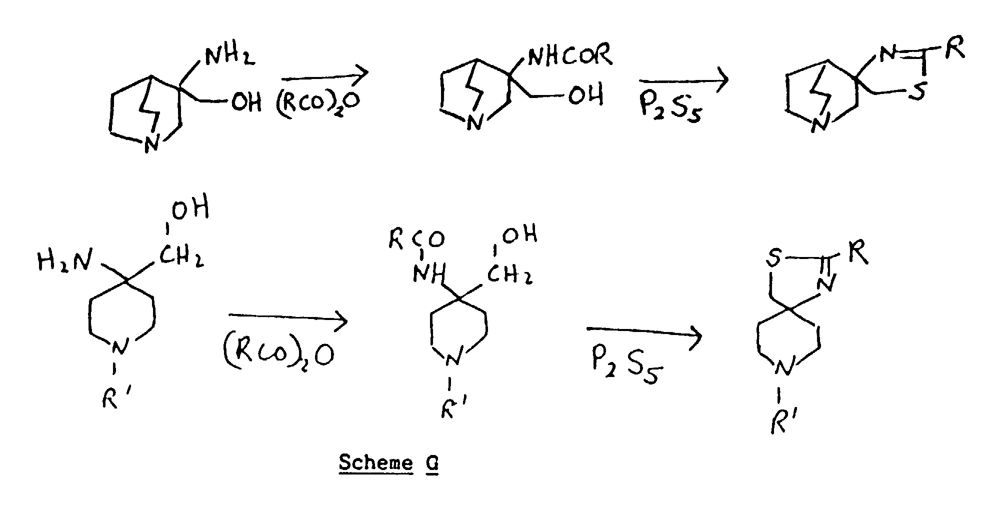

Additional methods include preparation of the corresponding epoxide or thioepoxide and reacting it with an appropriate nitrile, as in Scheme H, where the ring spiro-connected to the oxazoline or thiazoline ring is e.g., piperidine or quinuclidine.

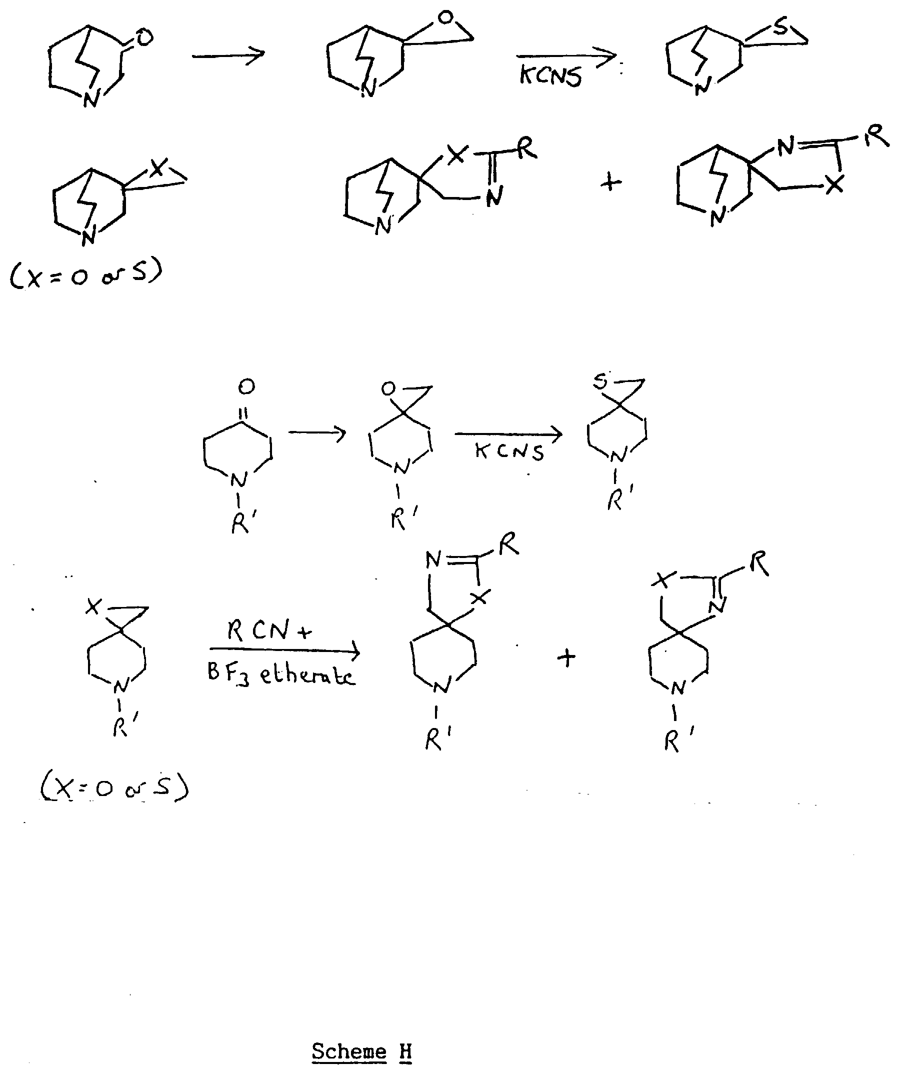

The compounds of formula (I) and (II), whether forming per se an embodiment of the invention, or contained in the pharmaceutical compositions of the invention, or utilized in the methods of the invention, include addition and quaternary salts of these compounds. By way of example, acid addition salts for pharmaceutical use include those formed from such acids as hydrochloric, hydrobromic, fumaric, maleic, oxalic, malonic, malic, acetic, citric, tartaric, dibenzoyl D- and L-tartaric, ditoluoyl D- and L-tartaric, salicylic, carbonic, aspartic and glutamic acids. In those cases where the spiro-compounds of the invention exhibit optical activity, the racemates can be resolved by use of optically active acids such as dibenzoyl-L- or D-tartaric acid, ditolyl-L- or D-tartaric acid.

At least those spiro-compounds of the present invention where R is methyl are centrally active muscarinic agonists. Due to their pharmacological properties, these compounds can activate central cholinergic functions under conditions where the cholinergic system is hypofunctional.

The spiro-compounds of the invention are in general potentially useful for the treatment of presenile and senile dementia, senile dementia of Alzheimer's type (SDAT), atypical Alzheimer's disease (Perry et al, Advances in Neurology, eds. R.J. Wurtman et al., 51:41, 1990), combined multiinfract dementia and Alzheimer's disease, age-associated memory impairments (AAMI), acute confusion disorders, emotional and attention disorders, mania, tardive-dyskinesia, hyperkinesia, mixed Alzheimer's and Parkinson's disease, aphasia, hallucinatory-states, post encephalitic amnesic syndrome, alcohol withdrawal symptoms, Huntington's chorea, Pick's disease, Friedrick's ataxia, Gilles de la Tourette disease and Down syndrome, because all of these disease states are disturbances in which a central cholinergic hypofunction has been implicated at least to a certain extent. The spiro-compounds of this invention are also potentially analgesic agents and therefore may be useful in the treatment of severe painful conditions such as rheumatism, arthritis and terminal illness. The spiro-compounds of the invention in which R is methyl, would appear to be of particular potential value for the treatment of SDAT and related disorders.

The spiro-compounds of the present invention may be used in combination with acetylcholinesterase inhibitors such as physostigmine or tetrahydroaminoacridine; in combination with acetylcholine precursors such as choline or lecithin; in addition to "nootropic" drugs such as piracetam, aniracetam, oxiracetam, or pramiracetam; in addition to compounds that interact with Ca²⁺ channels such as 4-aminopyridine or 3,4-diaminopyridine; or in addition to peptides that can have modulatory effects on acetylcholine release, such as somatostatin; in combination with a peripheral autimuscarinic agent (such as pirenzepine, N-methylatropine, N-butylscopolamine, propantheline, methantheline, glycopyrrolate, or tropenzilium) to counteract peripheral adverse effects that might be expected at high doses, such as salivation, diarrhea, gastric secretion or vomiting, or in combination with transdermal scopolamine such as Scopoderm^{(R)} to counteract nausea and/or vomiting; in combination with antidepressants such as nortriptyline, amitriptyline, imipramine, minaprine in order to alleviate both the cognitive impairments and depressive symptoms associated sometimes with SDAT, AAMI, mixed SDAT/Parkinson's disease (PD); in combination with M2-antimuscarinic drugs such as secoverine, AFDX-116(c.f. Hammer et al, Life Sci. 38: 1653, 1986) in order to counteract peripheral adverse side effects that might be expected at high doses of the compounds, to counteract inhibitory effects of such agonists at central inhibitory presynaptic and postsynaptic receptors of M2 type and to potentiate the release of acetylcholine via inhibition of inhibitory autoreceptors of M2 type at intact terminals; in combination with nicotinic agonists such as nicotine in order to stimulate both the nicotinic and muscarinic receptors in the brain; in combination with an adrenergic agonist (clonidine or quanfamicine) in order to alleviate both the cognitive and other impairments associated with a mixed cholinergic-noradrenergic deficiency in SDAT; in combination with inhibitors of neuronal serotonin reuptake such as alaproclate, zimelidine in order to alleviate both the cognitive and other emotional functions in SDAT; in combination with monoamine oxidase-B inhibitors like deprenyl in order to alleviate both cognitive and other motor impairments associated with mixed states such as SDAT/PD; in combination with Nerve Growth Factor (NGF, which is administered either by a nasal spray or intracerebroventricularly).

The spiro-compounds of the present invention, with or without the aforementioned other active substances, can be administered for example, by way of injection in a suitable diluent or carrier, per os, rectally in the form of suppositories, by way of insufflation or nasal spray, by infusion or transdermally in a suitable vehicle with or without physostigmine or tetrahydroaminoacridine, for example by using the device which is the subject of U.S. Patent No. 2163347 (issued November 29, 1988).

The present spiro-compounds, especially where R = methyl, are also of potential use for the treatment of disorders requiring the application of a long-lasting cholinergic agent of mild local activity. Such an agent is needed in disorders such as glaucoma, as the compound is not destroyed by the enzyme which deactivates acetylcholine, i.e. acetyl- and butyryl-cholinesterase, and may also be used for the treatment of peripheral cholinergic disorders such as myasthenia gravis, urinary bladder dysfunctions, Adi's disease and Eaton-Lambert disease. These compounds might also be used in disturbances where cholinergic underactivity is induced by drugs.

It appears that the present spiro-compounds, especially where R is C₃₋₆-alkyl or aryl, are anticholinergic agents and may potentially be used for treatment of disorders due to a cholinergic hyperfunction, whether this be spontaneous or drug-induced. These compounds are of potential use in the treatment of various diseases such as PD, pseudo-PD, mixed AD/PD, primary dystonias, spasmodic torticollis, cranial dystonia, depression, motion sickness, akathisia (after neuroleptic withdrawal), central hypertension, human head injury, mixed tardive dyskinesia and PD, manic-depression, as adjuncts in surgery instead of atropine, scopolamine, etc., in intoxication due to an excess of acetylcholine like inhibition of acetylcholinesterase. These may also be used in ophthalmology when either prolonged or short-term mydriasis is required.

The present spiro-compounds may also potentially be used in the treatment of disease characterized by excess peripheral-like activity such as asthma, chronic obstructive pulmonary disease, peptic ulcer disease. For these peripheral disorders it is recommended to use the quaternary salts of the formulae (Ia), (Ib) and (IIa) 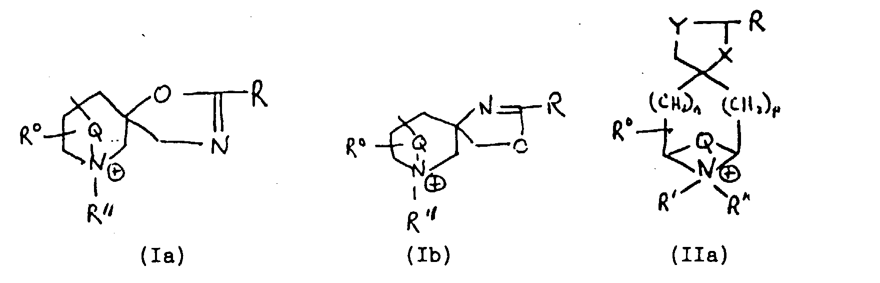 where the tertiary nitrogen is quaternized by R", where this is, for example, lower (C₁₋₆) alkyl, aryl such as phenyl, or aryl-substituted C₁₋₆ alkyl such as benzyl.

The invention will now be illustrated by the following non-limiting Examples.

### EXAMPLE 1: 2-Methyl-spiro(1,3 oxazoline-5,3')quinuclidine.

### (a) 3-Nitromethylquinuclidin-3-ol.

Quinuclidin-3-one (125 g., 1 mole) was dried by azeotropic distillation (40% w/w in toluene) and placed in a 3l. flask equipped with a mechanical stirrer, 1l. methanolic sodium ethoxide was added and the clear solution was stirred at 15-20°C. Nitromethane, 61 g. (1.0 mole), dissolved in 500 ml. absolute ethanol was then added over 0.5 hour while keeping the temperature below 20°C. The solution was acidified to pH=1 using HCl-isopropanol, filtered and dried to yield 160 g. crude product which was used as such for the next step.
¹H-NMR (D₂O, DSS) (HCl salt): δ 3.79 (d as part of an AB-type spectrum, one H of CH₂NO₂), 3.38-3.33 (m, 7H), 2.36 (m, 1H), 2.36-1.95 (m, 4H)

### (b) 3-Aminomethylquinuclidin-3-ol.

### (i) By reduction of 3-nitromethylquinuclidin-3-ol.

A solution of 3-nitromethylquinuclidin-3-ol hydrochloride in methanol was reduced by catalytic hydrogenation using 10% Pd on activated carbon to yield the HCl salt of 3-aminomethylquinuclidin-3-ol, which was recrystallized from methanol-isopropanol to give the product as white crystals.

### (ii) Via the azide.

3-Methylenequinuclidine epoxide (25 g., 0.18 mole) and sodium azide (20 g., 0.3 mole) were dissolved in 50 ml. water. The mixture was stirred overnight at room temperature, extracted with chloroform (2 x 200 ml.) and the extracts were concentrated by evaporation. The oily residue containing 3-azidomethylquinuclidin-3-ol was dissolved in water and wet active Raney nickel was added in portions with stirring until the evolution of nitrogen ceased (35 g.). The mixture was filtered, the filtrate was concentrated by evaporation and the residue was recrystallized from isopropanol to yield 9 g. pure 3-aminomethylquinuclidin-3-ol.
m/z M⁺ 156, base peak m/e 139 (M-NH₃), and m/z 96 which is typical of the quinuclidine skeleton.
¹H NMR (D₂O, DSS) (free base) : δ 1.3-2.0(5H); 2.3-3(8H). (di-HCl salt): δ 1.8-2.4(m,5H) ; 3.2-3.5(m,8H).
The ¹H-NMR of the dihydrochloride salt in comparison to the free base shows a downfield shift of six hydrogens attached to carbons adjacent to the nitrogens (e.g. δ 2.5-2.9 to 3.3-3.5ppm) in accordance with the expected structure.

### (c) 2-Methyl-spiro(1,3 oxazoline-5,3')quinuclidine (AF125).

### (i) Ethyl acetamidate method.

3-Aminomethylquinuclidin-3-ol (9.1 g., 0.058 mole) was dissolved in 500 ml. dichloromethane; ethyl acetamidate-HCl salt (14 g., 0.11 mole) was added and the mixture was stirred at 5°C for six hours. The solution was made alkaline with Dowex 1, filtered and the solvent was evaporated to yield an oily residue, which was distilled at 60-65°C/0.5 mm. Hg to yield AF125 as a colorless liquid.
m/z (M⁺)181 base peak, 139 (M-CH₃CN).
¹H-NMR (CDCl₃-TMS): δ 1.97(dd,3H)(J=1.3Hz); 2.8-2.9(m,1H); 3.14(d,1H)(J=12Hz); 3.5(dd,1H)(J=13Hz), 3.96(dd,1H)(J=13Hz, 1.3Hz).
¹H-NMR including spin decoupling experiments enable the assignment of the hydrogens adjacent to C₂, C₉ and the methyl group. Each of the hydrogens adjacent to C₉ shows a double doublet (J=13Hz geminal coupling) and a homo-allylic coupling with the methyl group (J=1.3Hz). Irradiation at 1.97 (methyl group) brings about the disappearance of the homo-allylic coupling at 3.5 and 3.9 ppm and vice versa. Irradiation at 3.5 effects the signal at 3.96 and vice versa which must be due to geminal coupling of H₉ₐ and H_{9b} (Table 1).
The ¹³C-NMR spectrum (in CDCl₃) is very informative and enables the assignment of most resonances to the appropriate carbons.

¹³C-NMR (CDCl₃-TMS): δ 13.2(CH₃); 20.5 (CH₂CH); 21.5(CH₂CH); 30.0 (CHCH₂); 45.3, 45.7(CH₂CH₂N); 62.2, 64.2(CH₂N); 84.0(COCH₂); 165(CCH₃).
GC (one peak): column, 25 m., diameter, 0.2mm., packing, 5% phenyl methyl silicone; detector type, FID; temperature: column, 125°C, injection port, 220°C, detector, 220°C, carrier gas: N₂, 0.7 ml./min.; retention time: 6.2 min.

### (ii) Acetic acid method.

3-Aminomethylquinuclidin-3-ol (10 g.) was dissolved in acetic acid (50 ml.), azeotropically distilled in xylene for 30 hours, and the solution was cooled and was made alkaline with

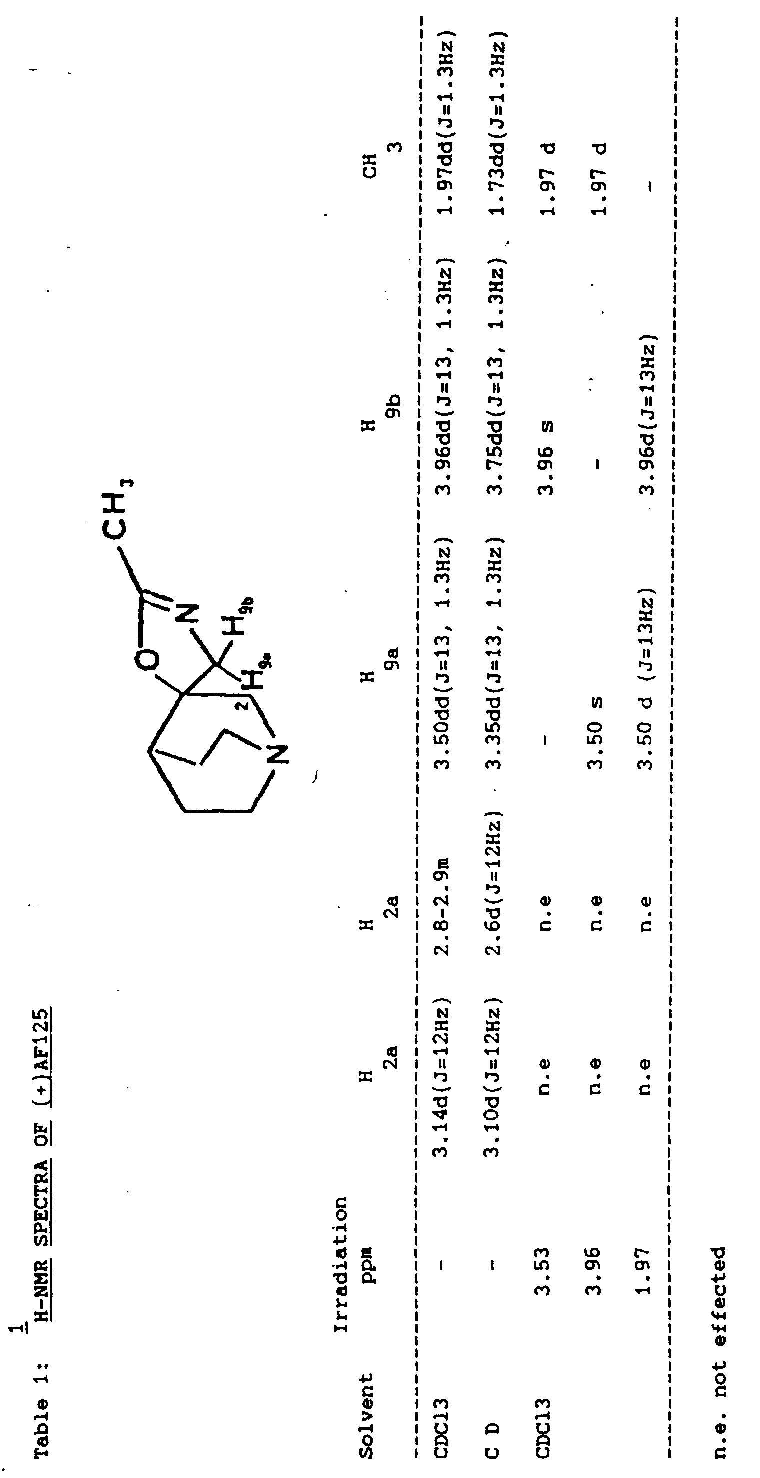

cold aqueous potassium carbonate. The organic phase was dried and evaporated to yield 5.2 g. crude product which was distilled under reduced pressure (60-65°C/0.5 mm. Hg) to yield a colorless liquid which solidified under refrigeration. The compound obtained was identical to AF125 obtained by method (c) (i).

### d) Optical resolution of AF125

(Preliminary note: owing to the possibility that the polarimeter used to determine the optical rotation data may not have been reliable, the purity of the optical isomers was evaluated using ¹H-NMR, as described below.)

To a stirred cold solution of AF125 (15.5 g., 0.085M) in acetone (150 ml.), there was added dropwise a solution of dibenzoyl-L-tartaric acid (18.8 g., 0.05M). A crystalline material separated immediately. The precipitated salt was collected and purified by dissolving in hot isopropanol, cooling and adding ether to the cold solution until turbid; one repetition of this procedure gave a product of constant optical rotation [α]_{D}²⁰ = -28°(MeOH); m.p. 145°.

In a similar manner, the corresponding salt of the other enantiomer was obtained, starting from AF125 (14.74 g., 0.08M) and dibenzoyl-D-tartaric acid (17.76 g., 0.047M) of the corresponding tartaric acid. After 3 crystallizations, the product had [α]_{D}²⁰ = +26°(MeOH); m.p. 145°.

The respective free bases were obtained by neutralization of the salts with a 10% solution of K₂CO₃ and several extractions with chloroform. The free base derived from the salt of dibenzoyl-D-tartaric acid) had [α]_{D}²⁰ = -43°(MeOH), while that derived from the salt of dibenzoyl-L-tartaric acid had [α]_{D}²⁰ = +36°(MeOH).

As indicated above, the optical purity of the enantiomers was shown by ¹H-NMR. Thus the ¹H-NMR spectrum of AF125 as a racemate in the presence of optically pure resolving agent (s)(+)2,2,2 trifluoro-1-(9-anthryl)-ethanol reveals two optical isomers. The difference in chemical shift (in C₆D₆) of one of the hydrogens which is attached to C₂ enables determination of the optical purity of AF125. Each separated enantiomer reveals only one doublet (half an AB-type spectrum) for one of these hydrogens on C₂.

### EXAMPLE 2: 2-Ethyl spiro(1,3 oxazoline 5,3')quinuclidine (AF123).

Similarly to Example 1(c), the title product was obtained by condensation of 3-aminomethylquinuclidin-3-ol with propanoic acid followed by azeotropic distillation in xylene. Purification of crude AF123 was effected by vacuum distillation under reduced pressure.
m/z: M 195, base peak.
¹H-NMR (CDCl₃-TMS): δ 4.0(d,1H)(J=13Hz); 3.55(d,1H)(J=13Hz); 3.1(d,1H); 2.7-2.9(m); 2.3(q,2H)(J=7.5Hz, CH₂CH₃); 1.1(t,3H)(J=7.5Hz,CH₃).
GC (same conditions as for AF125; one peak): retention time, 8.86 min.

### EXAMPLE 3: 2-Amino-spiro(1,3-oxazoline-5,3')quinuclidine AF125(N)

The amino-oxazoline derivative AF125(N) is an example of an electron rich oxazoline ring, since the amino nitrogen is conjugated to the double bond, enhancing the negative charge on the imino nitrogen.

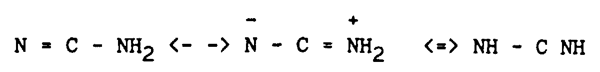

3-Aminomethylquinuclidin-3-ol (6 g.) and cyanogen bromide (3.5 g.) were dissolved in methanol (200 ml.). The reaction mixture was stirred at room temperature for 5 hours, and then concentrated under vacuum. The residue was purified on an alumina column (300 g.), using a multisystem solvent as an eluent (1.5:1.0:0.2:0.06 CHCl₃/ether/methanol/NH₄OH). An almost pure fraction of 450 mg. was obtained, which was finally purified by trituration with acetone to give a pure crystalline material. The first chromatographic separation gave also various products including 2 g. of crude desired product which was subjected to a further separation on a column of silica, using as eluent 2% NH₃ in methanol. The pure product has a m.p. 135-136°.
¹H-NMR (CDCl₃-TMS): δ 3.83, 3.43, 3.2 (3 doublets, 3H, Hb and Hc, J=0.048Hz); (m,5H); 1.9 (b, 2H); 1.5 (m, 3).
FAB-MS showed a molecular ion (M+1)⁺182.

### EXAMPLE 4: 2-Phenyl-spiro(1,3-oxazoline-5,3')quinuclidine AF125(Ph)

A suspension of 3-aminomethylquinuclidin-3-ol (2 g., 0.01 mole) and ethyl imidobenzoate hydrochloride (2g., 0.01 mole) in ethanol (200 ml.) was mixed for 24 hours at room temperature. After filtration and concentration of the filtrate by evaporation, the crude residue was basified with NaHCO₃ and extracted with dichloromethane. The organic extracts were concentrated by evaporation and the crude residue was separated on an alumina column (30 g., 1.5 cm. diameter) using a multi-solvent (1.0:1.0:0.2:0.026 CHCl₃/ether/methanol/ 32% aq. NH₄OH) as eluent. The product was rapidly eluted (after 40ml). The solvent was evaporated without heating and a white solid was obtained, m.p. 65-70°C.
¹H-NMR(CDCl₃): δ 7.955 (2H), 7,72 (2H), 4.16 (d,1H), 3.73 (d, 1H), 3.26 (d, 1H), 2.93 (d, 1H) and (t, 2H), 2.77 (t, 2H), 2.2 (m, 1H), 1.95 (m, 1H), 1.60-1.52 (m, 3H) ppm.
IR (CHCl₃) 2920, 2900,, 2850sh, 1640sh, 1630, 1625sh, 1440, 1337, 1265, 1250, 1070, 1050, 1035, 1005, 965 cm⁻¹.
m/z: 242 (60%) [M⁺], 198 (30%, 171 (12%), 149 (10%), 139 (50%0, 124 (17%), 122 (20%), 121 (20%), 117 (30%), 111 (12%), 105 (33%), 96 (100%), 82 (50%), 69 (42%), 55 (36%).

### CHEMICAL AND PHYSICAL PROPERTIES OF THE COMPOUNDS OF FORMULA (I)

Most of these compounds are relatively stable. AF125, for example, is thermally stable and can be distilled at 130° without decomposition. Its chemical behaviour resembles that of known 2-oxazoline derivatives. In aqueous solution the free base shows a slight decomposition after a few hours which increases gradually with time. The formation of the decomposition products can be monitored with ¹H-NMR by inspection at two regions δ 3.3-3.5 (appearance of two doublets) and 1.9-2.0 ppm (appearance of two additional singlets).

The expected decomposition pattern (when e.g., X is O and Y is N) involves opening of the oxazoline ring to form both amide and ester as shown in Scheme E, in which the quinuclidine-derived compounds are depicted illustratively. The formation of two new singlets at δ 1.9-2.0 ppm as expected for R = Me of these two structures are in agreement with this supposition.

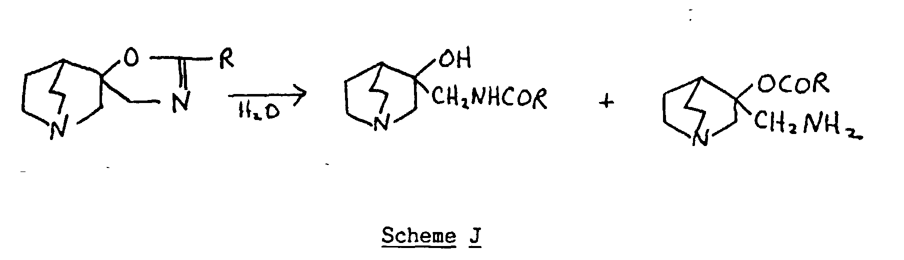

The same pattern of decomposition can be shown for R=Et (AF123). Ring opening is much faster in the presence of dilute hydrochloric acid. It is noted that heating of the tartrate and mandelate salts in tetrahydrofuran also resulted in partial decomposition. The tendency to decomposition of such salts complicates the optical separation of compounds such as AF125 and AF123. However, this problem can be overcome by using the method described for the optical separation of the enantiomers of AF125, or alternatively the amino-alcohols such as 3-aminomethylquinuclidin-3-ol can be resolved and the separated enantiomers can then be cyclized using an ester or imidate as already described. In yet another alternative the N-acyl derivatives of (e.g.) 3-aminomethylquinuclidin-3-ol can be resolved and the separated enantiomers can then be cyclized per se as shown in Scheme K: 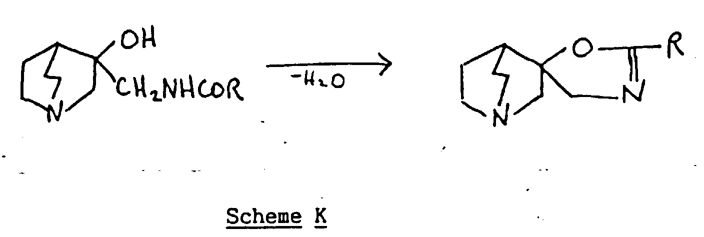 For this cyclization, there may be used, e.g., p-toluene sulfonic acid, or boron trifluoride etherate.

### EXAMPLE 5: 2-Methyl-spiro(1,3-oxazoline-5,4')-1'-methylpiperidine (II; R = R' = methyl - "AF150")

### (a) 1-Methyl-4-nitromethylpiperidin-4-ol hydrochloride

This starting material was prepared using a slight modification of the method of A.D. Cale (U.S. 4,746,655, 1988). A mixture of N-methylpiperidinone (142 g.**,** 1.28 mole) and nitromethane (78.1 g., 1.28 mole), was added to a well-stirred solution of sodium ethoxide (1.28 mole), 20% in ethanol, maintaining the internal temperature at 5-8°C. A white solid precipitates, the stirring is continued for 20 minutes and another 40 minutes at room temperature. The resulting solution was acidified with 500 ml. of 7.2N HCl in isopropyl alcohol. The hydrochloride and the inorganic salts were extracted with CH₃OH (3x200 ml) and the solvent removed in vacuo to give the title compound, m.p. 180-182°c (non hygroscopic).
m/z: 174 (M⁺ of free base, 100%), 157 (M-OH, 20%),
127 (M-H-NO₂, 25%), 113 (M-NO₂-CH₃, 40%).

### (b) 4-Aminomethyl-1-methylpiperidin-4-ol hydrochloride

Palladium on charcoal (10%, 4 g.) was added portionwise to a solution of 1-methyl-4-nitromethylpiperidin-4-ol (133.5 g.) in methanol (1500 ml). The compound was hydrogenated in a Paar at a pressure of 55 psi at room temperature for 48 hours. The solution was cautiously filtered, treated with active charcoal, the solvent removed and the residue was triturated with ethanol (200 ml.) to give the title compound, m.p. 177-179°C.
- m/z:: 144(M⁺ of free base, 15%), 127 (M-OH, 25%),
114 (M-CH₂NH₂, 100%).

### (c) 2-Methyl-spiro(1,3-oxazoline 5,4')-1'-methylpiperidine (AF150)

A solution of KOH (1.43 g. of 86%) in methanol (50 ml.) was added to a solution of 4-aminomethyl-1-methylpiperidin-4-ol hydrochloride (3.61 g., 0.02 mole) in absolute methanol (50 ml.). After stirring for 10 minutes, a solution of ethyl acetimidate hydrochloride (2.7 g.) in 20 ml. absolute methanol was added, and stirring continued for 30 minutes at room temperature. The solvent was removed, and the residual solid was dissolved in a solution of 2.8 g. Na₂CO₃ in 50 ml. water, which was concentrated to dryness in vacuo. The white solid was extracted with 2 x 50 ml. chloroform, treated with active charcoal, dried (Na₂SO₄) and the solvent removed to afford the title product (62.5% yield), m.p. 45°C (sublimed at 40°C/0.05 mm Hg), giving a single spot on silica TLC eluted with 2% NH₃ in CH₃OH, Rf=0.4.
m/z: 168 (M⁺ of free base, 100% at 7.5 ev).
¹H-NMR (300 MHz, CDCl₃): δ 3.56 (2H, q, J=1.5 Hz), 2.53 (4H, m), 2.34 (3H, s), 1.96 (3H, t, J=1.5 Hz), 1.82 (4H, m).

Replacement of the KOH used in this Example by the equivalent amount of NaOH or Et₃N, gave similar results.

### (d) 2-Methyl-spiro(1,3-oxazoline 5,4')-1'-methylpiperidine Dibenzoyl-D-tartrate

A hot solution of dibenzoyl-D-tartaric acid (5.4 g., 15 mmole) in 500 ml. toluene was added while stirring to AF150 (5.5 g., 32 mmole) dissolved in 200 ml. dry toluene. The precipitate was allowed to settle and the supernatant liquid was decanted off. The residual solid was washed with 3 x 100 ml. dry toluene and dried under reduced pressure to afford 8.4 g. (80% yield) of a white slightly hygroscopic solid.
TLC chloroform/alumina (Merck Art 5581) Rf=0.4.
m/z: 168 (M⁺)
¹H-NMR (300 MHz, D₂O containing 1.5 mg. Na₂CO₃/0.5 ml. D₂O):
δ 1.95 (s, 6H, CH₃-C), 2.35 (s, 6H, CH₃-N), 3.5 (s, 4H, CH₂), 5.7 (s, 2H), 7.5-8.2 (m, 10H, aromatic hydrogens).

### EXAMPLE 6: 2-Ethyl-spiro(1,3-oxazoline- 5,4')-1'-methylpiperidine (II; R = ethyl, R' = methyl)

This compound was prepared similarly to the compound of Example 5, using the equivalent amount of ethyl propionimidate hydrochloride, in place of ethyl acetimidate hydrochloride. The product was obtained as a liquid, b.p. 53°/0.03 mm Hg, in 60.5% yield.
¹H-NMR (300 MHz, CDCl₃): δ 3.52 (2H, t, J=1.5 Hz), 2.47 (4H, m), 2.30 (3H, s), 2.26 [2H, quartet (J=7 Hz), triplets (J=1.5 Hz)], 1.86 (2H, m), 1.72 (2H, m), 1.18 (3H, t).

### EXAMPLE 7: 1-Methylpiperidine-4-spiro -4'-(2'-methyl-1',3'-oxazoline (II; R = R' = methyl - "AF151")

### (a) 1-Methylpiperidine-4-spiro-5'-hydantoin

A mixture of solutions of 1-methylpiperidine-4-one (36.44 g., 0.322 mole) in ethanol (150 ml.), ammonium carbonate (93.0 g., 0.968 mole) in water (400 ml.) and potassium cyanide (25.8 g., 0.396 mole) in water (82 ml.), was heated at 60°C for 2.5 hours and then left at room temperature overnight, when 1-methylpiperidine-4-spiro-5'-hydantoin separated. It was filtered off and washed with small amounts of cold water, ethanol and ether, to give a crystalline powder (27.0 g.). Concentration of the filtrate and washings gave a second crop (20.0 g.). The product was crystallized from methanol: m.p. 265-276°(dec.).
IR (KBr) 3170 (NH); 1700 (C=O) cm⁻¹
m/z 183(M⁺, 38%); 71 (100%)
¹H-NMR (300 MHz, D₂O): δ 1.8 (2H), 2.06 (sextet, 2H), 2.49 (S, -CH₃), 2.58 (t, 2H), 3.14 (t, 1H), 3.20 (t, 1H).

### (b) 4 -Amino -1-methylpiperidine-4 -carboxylic acid

1-methylpiperidine-4-spiro-5'-hydantoin (9.75 g., 0.0533 mole) and barium hydroxide octahydrate (28.8 g., 0.00913 mole) in water (150 ml.) were heated at 160°C in an autoclave for three hours. The contents of four such batches were combined and the precipitated barium carbonate was filtered off. The filtrate was neutralized with solid carbon dioxide and the precipitate was removed by filtration. The filtrate was concentrated to a small volume to give 4-amino-1-methylpiperidine-4-carboxylic acid (32.0 g., 95% yield), m.p. 275-280°C (dec.).
IR (KBr) 3300, 1655, 1580 cm⁻¹
m/z 158(M⁺, 90%); 141 (98%, M-OH); 113 (12%, M-CO₂H); 96 (100%); 71 (52%)
¹H-NMR (300 MHz, C₅D₅N + D₂O): δ 1.2 (m, 2H), 1.48 (s, CH₃N-), 1.7 (m, 2H), 1.9 (m, 2H), 2.0 (m, 2H).

### (c) 4-Amino-4-hydroxymethyl-1-methylpiperidine

Lithium aluminum hydride powder (15.62 g., 0.412 mole) in dry tetrahydrofuran (THF) (600 ml.) was heated under reflux for 15 minutes, after which 4-amino-1-methylpiperidine-4-carboxylic acid (31.0 g., 0.196 mole) in the form of a dry powder was added portionwise under nitrogen, with efficient stirring. After the addition was completed, the reaction mixture was heated under reflux for four hours, cooled to 0°C under nitrogen with efficient stirring, worked up by careful slow addition of water (20 ml.), 15% aqueous NaOH (20 ml.) and again water (10 ml.). The reaction mixture was filtered and the precipitate was extracted with boiling THF (3 x 150 ml.). The THF filtrate and the extracts were combined and the solvent removed at 25 mm to give a yellow viscous oil (28.0 g., 98.9% yield).
IR (neat) 3320 (NH), 3200 (br. OH), 1587 (NH₂), 1468, 1448 cm⁻¹ m/z 144(M⁺, 15%); 127 (M-OH); 113 (M-CO₂H); 96 (100%); 70 (41%). ¹H-NMR (300 MHz, CDCl₃): δ 1.41 (m, 2H), 1.60 (m, 2H), 2.24 (s, CH₃-N), 2.29 (m, 2H), 2.48 (m, 2H), 2.50 (br., -NH₂), 3.29 (s, -CH₂OH).

### (d) 1-methylpiperidine-4-spiro-4'-(2'-methyl-1',3'-oxazoline) (AF151)

A mixture of 4-amino-4-hydroxymethyl-1-methylpiperidine (1.80 g.) with acetic acid (20 ml.) and xylene (20 ml.) was azeotropically distilled for 28 hours. The remaining acetic acid and xylene were removed at reduced pressure (25 mm Hg) to leave a residual viscous oil which was basified to pH 11 with an aqueous solution of K₂CO₃. Extraction with chloroform and evaporation of the extract gave a small amount of residual brown oil (0.27 g.). The aqueous solution remaining after chloroform extraction was evaporated to remove water, the residual solid was extracted with chloroform and the extract was dried (Na₂SO₄) and evaporated, to afford as residue a very hygroscopic solid (3.0 g.). TLC showed that the latter gave mainly one spot, which was more polar than the starting amino-alcohol.

A portion of the hygroscopic solid, which melted at 150-160°C, was heated under vacuum, and almost immediately began to distil as a colorless oil at 45°C/0.15 mm Hg. This oil, on keeping in the freezer, formed crystalline needles melting at room temperature. The distillate was the acetic acid salt of the title compound.
IR (neat) 1664 (-C=N); 1565 & 1398 (-CO₂⁻⁻); 1256 (C-O) cm⁻¹
m/z 168(M⁺ of free base); 109; 70.
¹H-NMR (300 MHz, CDCl₃): δ 1.77 (m, 2H), 1.96 (m, 2H), 1.98 (s, CH₃-), 2.0 (s, CH₃-), 2.49 (s, CH₃-N-), 2.91 (m, 4H), 3.95 (s, -CH₂O-), 9.30 (br. s, -CO₂H).
¹³C-NMR(300 MHz, CDCl₃): δ 14.0 (CH₃CO₂-), 22.9 (CH₃C=N-), 35.6 (C₃ and C₅), 44.4 (CH₃N⁺), 51.1 (C₂ and C₆), 67.0 (C₄), 77.4 (C_{5'}), 164.3 (C-=N), 176.7 (-CO₂-).
¹H-NMR of free base (300 MHz, CDCl₃): δ 1.64 (m, 2H), 1.84 (m, 2H), 1.98 (s, CH₃-), 2.26 (m, 2H), 2.30 (s, CH₃-), 2.69 (m, 2H), 3.94 (s, -CH₂-).

### EXAMPLE 8: 1-Methylpiperidine-4-spiro-4'-(2'-ethyl-1',3'-oxazoline)

A mixture of 4-amino-4-hydroxymethyl-1-methylpiperidine (3.0 g.) with propionic acid (50 ml.) and xylene (90 ml.) was azeotropically distilled for 5 hours. The residue (7 ml.) was basified to pH 11-12 with an aqueous solution of K₂CO₃. Extraction with chloroform and evaporation of the extract gave a mixture of non-polar compounds (0.80 g.). The aqueous solution remaining after chloroform extraction was evaporated to remove water, the residual solid was extracted with chloroform and the extract was dried (Na₂SO₄) and evaporated, to afford as residue a hygroscopic solid (3.6 g.). TLC showed that the latter gave mainly one spot, which was more polar than the starting amino-alcohol (silica gel, solvent 40:58:2 methanol-chloroform-aqueous ammonia).

A portion of the hygroscopic solid (1.5 g.) was heated under vacuum, and almost immediately began to distil as a viscous colorless oil at 50°C/0.1 mm Hg. The distillate is the propionic acid salt of the title compound.
m/z 182(M⁺ of free base, 14%); 167 (5%), 154 (71%), 125 (9%), 109 (100%), 96 (45%), 81 (30%), 74 (57%), 70 (89%), 57 (64%).
¹H-NMR (300 MHz, CDCl₃): δ 1.12 (t, J=7.5 Hz, CHCH₂-), 1.17 (t, J=7.6 Hz, CH₃CH₂-), 1.75 (m, 2H), 2.00 (m, 2H), 2.29 (q, J=7.5, CH₃CH₂-), 2.30 (q, J=7.6, CH₃CH₂-), 2.56 (s, CH₃N-), 3.02 (m, 2 - -CH₂-), 3.95 (s, -CH₂O-), 7.52 (br. -CO₂H).

To a stirred solution of the above propionic acid salt (700 mg.) in chloroform, a saturated aqueous solution of K₂CO₃ was added until evolution of CO₂ had ceased. The mixture was then stirred for 0.5 hour and the phases were separated. The aqueous phase was extracted with chloroform, the combined separated chloroform phase and the extracts were dried (Na₂SO₄), and the solvent was evaporated to afford the title compound in free base form as a residual colorless oil (550 mg.), which showed a single spot on TLC.
¹H-NMR (300 MHz, CDCl₃): δ 1.17 (t, J=7.6 Hz, CH₃CH₂-), 1.61 (m, -CH₂-), 1.86 (m, -CH₂-), 2.18 (m, -CH₂-), 2.29 (q, J=7.6, CH₃CH₂-), 2.30 (s, CH₃N-), 2.71 (m, -CH₂-), 3.94 (s, -CH₂O-).
m/z 182(M⁺ 25%), 167 (9%), 154 (78%), 125 (17%), 109 (100%), 96 (65%), 81 (54%), 70 (96%), 57 (77%).

An alternative route to compounds such as AF150 and AF151 depends on the cyclodehydration of the appropriate amides, as shown in the following illustrations: 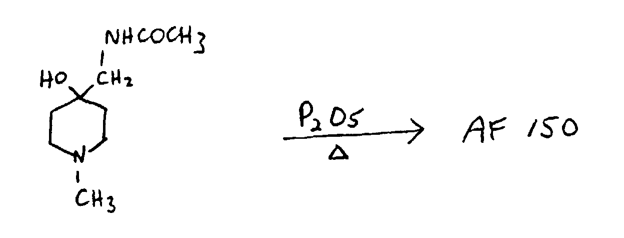 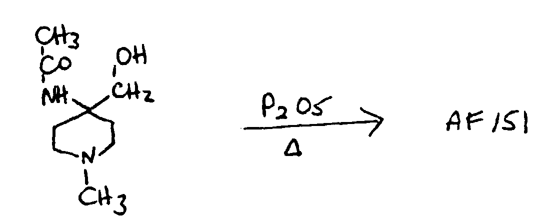 Dehydrating agents such as P₂O₅, sulfuric acid, BF₃-etherate, CaCl₂, and molecular sieves, can be used for the above reactions. Corresponding thiazolines instead of oxazolines can be obtained by analogous reactions using P₂S₅.

### EXAMPLE 9: 2-Methyl-spiro(1,3-thiazoline-5,4')-1'-methylpiperidine (II; R = R' = methyl - "AF150(S)")

### (a) 4-Acetamidomethyl-4-hydroxy-1-methylpiperidine

4-Aminomethyl-4-hydroxy-1-methylpiperidine (0.83 g., 5.7 mmole) was dissolved in 10 ml. chloroform, and acetic anhydride (0.58 g., 5.7 mmole) was added. The reaction mixture warmed spontaneously to 40-50°C. After 30 minutes, the solvent was evaporated and the crude residue was chromatographed on a silica gel column (Merck 7734), using 33:67 2% aqueous ammonia-methanol as eluent.
m/z 186 (M⁺)
¹H-NMR (300 MHz, CDCl₃): δ 1.60 (multiplet, 4H, H3 and H4), 2.01 (singlet, 3H, CH₃-C), 2.29 (singlet, 3H, CH₃-N), 2.38 (multiplet, 2H, H1), 2.55 (multiplet, 2H, H₂), 2.98 (multiplet, 1H, NH), 3.26 (doublet, 2H, H5) ppm.
¹H-NMR (300 MHz, D₂O): δ 1.42 (multiplet, 4H, H3 and H4), 1.81 (singlet, 3H, CH₃-C), 2.08 (singlet, 3H, CH₃-N), 2.27 (multiplet, 2H, H1), 2.46 (multiplet, 2H, H₂), 3.03 (singlet, 2H, H ) ppm. The impurity gives a peak at 3.44 ppm.

### (b) 2-Methyl-spiro(1,3-thiazoline-5,4')-1'-methylpiperidine

A mixture of 4-acetamidomethyl-4-hydroxy-1-methylpiperidine (6.5 g., 35 mmole) with phosphorus pentasulfide (10 g., 22 mole) was heated at 220°C for 30 minutes, cooled, and dissolved in 30 ml. concentrated hydrochloric acid. The acidic solution was transferred to 100 ml. cold concentrated aqueous sodium hydroxide, extracted with 2 x 100 ml. chloroform, and the combined extracts were dried and evaporated to afford 5 g. of a black oily residue, which was purified by distillation at 75°C/ 1 mm Hg to yield 1.8 g. clear liquid.
m/z: 184 (M⁺)
¹H-NMR (300 MHz, CDCl₃): δ 1.8-2.0 (m, 4H), 2.17 (t, 3H, CH₃-C), 2.2 (s, 3H, CH₃-N), 3.9 (q, 2H, CH₂-thiazoline ring).

### Biological Testing

The compounds according to the invention, exhibit pharmacological activity and are therefore useful as pharmaceuticals, e.g. for therapy. In particular the agonists show activity in the tests detailed below. In the Tables which report the results of such tests, certain compounds may be denoted by the following reference numerals:

| | | |
|---|---|---|
| (1) Carbachol | (6) AF151◆ | (11) Atropine |
| (2) Oxotremorine-M | (7) AF150(S)◆ | (12) Scopolamine |
| (3) Oxotremorine | (8) AF125◆ | (13) AF125(N)◆ |
| (4) AF102B* | (9) McN-A-343 | (14) AF123◆ |
| (5) AF150◆ | (10) Pirenzepine | (15) AF125(Ph)◆ |

| | | |
|---|---|---|
| *cis-2-methylspiro(1,3-oxathiolane-5',3)quinuclidine (US 4,855,290) | | |
| ◆exemplified compounds of the present invention | | |

### Test 1: Isolated Guinea-pig ileum preparation

Compounds of the invention were tested for their agonistic and antagonistic activities in the guinea-pig ileum preparation. Table 2 summarizes qualitatively the results obtained with some of the tested compounds, of which the most potent agonist was AF125. AF125 was found to be a full agonist in the guinea-pig ileum preparation and its EC₅₀ was calculated as 1.3 µM. The EPMR_{ACh} of AF125 was about 14 as compared to values ranging between 50-100 which were found for AF102B. This compound showed also high affinity toward muscarinic binding sites in the brain. In fact, comparison between AF102B and AF125 showed that AF125 is more active by an order of magnitude than AF102B in its ability to contract the guinea-pig ileum. Thus, AF125 induced much greater contractile response than AF102B at the same concentration.

**Table 2:**

| The effect of various putative cholinergic compounds on the guinea-pig ileum preparation | | | | | |
|---|---|---|---|---|---|
| Tested compd. # | Lowest conc. (µM) | * | Highest conc. (µM) | * | Remarks |
| 8 | 0.2 | + | 1.7 | +++ | Full agonist; contractions were blocked by atropine |
| 13 | 16.6 | + | 133.0 | +++ | Full agonist; contractions were blocked by atropine |
| 14 | 66 | - | 660.0 | - | Blocked reversibility ACh-induced contractions |
| 15 | 0.1 | - | 10 | - | Antagonist IC₅₀=0.9 µM |

| | | | | | |
|---|---|---|---|---|---|
| *intensity of contractions. The response is qualitatively described by the number of the plus signs, where + relates to a small contractile response and +++ relates to an intense one. No contractile response is marked by a - sign. | | | | | |

The amino analogue of AF125, [AF125(N)] was also active as an agonist, but higher concentrations of this compound were needed to contract the ileum. As can be seen, AF125(N) is active only at concentrations which are higher by one order of magnitude as compared to AF102B. AF125(Ph) is an antagonist (IC₅₀=0.7µM). In contrast to the amino analogue of AF125, AF123 was found to be an antagonist.

Similar tests showed that the compound of Example 5 (AF150) is a full muscarinic agonist - having an EC₅₀=0.8µM (blocked by atropine 10µM). When compared to acetylcholine (ACh), AF150 is capable of inducing a maximal contraction of the ileum of 130% (ACh=100%). Surprisingly, the shape of the curve of AF150 as compared with ACh is different and this curve can be analyzed by a two- or even a three-sites model. This can indicate that AF150 has different affinities to the subtypes of muscarinic receptors found in this preparation. Moreover, the compound of Example 7 (AF151) is also a full muscarinic agonist - having an EC₅₀=3µM (blocked by atropine 10µM).

### Test 2: Binding to muscarinic receptors in the brain

The binding of agonists to M1 muscarinic receptors and the effect of guanylyl imidophosphate (GppNHp). Agonists are known to be coupled with guanine nucleotide binding proteins (G proteins). While numerous studies of agonist binding to the M2 muscarinic receptor (low-affinity pirenzepine) have been done, relatively few studies have examined agonist binding to M1 muscarinic (high-affinity pirenzepine) receptors. The primary reason appears to be that most agonists (except AF102B for example, U.S. Patent No. 4,855,290) have a higher affinity for the M2-subtype than for M1 muscarinic receptors.

Non-hydrolyzable guanine nucleotides such as GppNHp dissociate the G protein from the receptor and decrease the affinity of the receptor for agonists but not for antagonists. Under special conditions it is possible to analyze both the affinity as well as the efficacy of agonist with M1 muscarinic receptors using low concentrations of ³H-pirenzepine (³H-PZ) bellow its K_{D} [Potter et al, Cell. Molec. Neurobiol 8:181-191 (1988); Potter and Ferrendelli, J. Pharmacol. Exp. Ther. 248: 974-978 (1989); Flynn et al, Eur. J. Pharmacol. 172:363-372 (1989)].

Under these conditions agonists of high efficacy ("full agonists") bind directly to the M1 receptor subtype with two affinities. The higher affinity state of the M1 receptor subtype is sensitive to guanine nucleotides and GppNHp, for example, converts the high-affinity state to a low-affinity state. The ratio of low and high affinities (K_{L}/K_{H}) of the M1 receptor may predict the relative efficacies of agonists to activate putative M1 receptor-mediated second messenger systems.

Table 3 summarizes the results obtained oxotremorine-M and carbachol (two full but non-selective M1 and M2 agonists), for the selective M1 agonist AF102B (U.S. Patent No. 4,855,290), AF150 and AF151. As expected, the quaternary full agonists, oxotremorine-M and carbachol show a two-affinity state and the relative efficacy of these compounds is predicted by the ratio of K_{L}/K_{H}. On the other hand, AF102B and AF125 show a mass-action curve.

**Table 3:**

| Apparent affinity states for M1 muscarinic receptor in rat cerebral cortex | | | | | | | | | |
|---|---|---|---|---|---|---|---|---|---|
| Compound # | K_{H}(µM) | K_{H}(µM) | %H | | K_{L}(µM) | K_{L}(µM) | %L | | K_{L-G} |
| | -G | +G | -G | +G | -G | +G | -G | +G | K_{H-G} |
| 1 | 0.23 | * | 36 | * | 12 | 20 | 64 | 100 | 52 |
| 2 | 0.11 | 0.35 | 53 | 2.7 | 6.7 | 4.2 | 47 | 73 | 61 |
| 4 | 0.7-1 | 0.7-1 | * | * | 0.7-1 | 0.7-1 | * | * | 1 |
| 5 | 0.11 | * | 21 | * | 11 | 21 | 83 | 100 | 100 |
| 6 | 3.7 | 4.6 | 39 | 17 | 4.8 | 59 | 61 | 83 | 13 |
| 8 | 9 | 9 | * | * | 12 | 12 | * | * | 1 |

| | | | | | | | | | |
|---|---|---|---|---|---|---|---|---|---|
| *Competition curves were significantly better fitted to a one site-model (mass-action curve). | | | | | | | | | |

Notes on Table 3: Ki=K_{H} or K_{L} = IC₅₀/1+C/K_{D} where C = 4.4nM is the concentration of the radioactive ligand (³H-PZ) and K_{D}=13.9nM is the dissociation constant thereof. K_{H}, K_{L}, %H and %L are the apparent inhibition constants of the tested ligands for the high and low affinity sites, respectively. G is GppNHp (guanylyl imidodiphosphate), and was used in 0.1 mM concentration. Displacement of ³H-PZ from cortical binding sites was performed in Tris/Mn buffer (pH 7.4) for one hour at 25°C; the buffer included 1 mM EDTA and 2mM Mn⁺⁺ ions. The method is similar to the procedure described by Potter and Ferrendelli (J. Pharmacol. Exptl. Therap. 248: 974-978, 1989). The binding data were analyzed using a GraphPAD program and were fitted to one or two components. Values are means from 1-3 experiments carried out in triplicates.

Surprisingly, AF150 and AF151 show a typical two-affinity states in presence of ³H-PZ with a very high K_{L}/K_{H} ratio for AF150. Moreover, GppNHp shifted the high-affinity state into the low-affinity state. AF150 and AF151 show both high affinity for M1 putative receptors and a high efficacy on the same receptor. This can be concluded because ³H-PZ at the concentrations used (1/3 of its K_{D}) binds only to M1 receptors. The data indicate that both AF150 and AF151 are highly efficacious agonists activating M1 receptors and are expected therefore to activate the transduction mechanism which is involved in secondary messengers coupled to M1 receptors, such as phosphatidyl inositol turnover and Ca²⁺ mobilization, for example. Simultaneous activation of both H and L sites may prove to be necessary for functional effects. In this regard, AF150 and AF151 may be particularly suited for the treatment of disorders with cholinergic hypofunction such as SDAT.

### Test 3

The two-affinity state of AF150 is preserved from homogenates of rat cerebral cortex also when the displacement of ³H-PZ is performed in a 10mM sodium-potassium buffer indicating that this is a unique agonist when compared with other putative muscarinic agonists (Table 4). AF150 shows an excellent selectivity and efficacy for M1 receptors as shown in Tables 4 and 5. In this regard AF150 may be an excellent drug for the treatment of disorders with cholinergic hypofunction such as SDAT since M1 selectivity and high efficacy are believed to be of particular therapeutic value in these type of disorders.

The potency of compounds of the invention (and for comparison other cholinergic compounds) in displacing from rat brain homogenates, cerebellum or frontal cortex, the following ³H-labelled compounds, namely, (-)³H-quinuclidinyl benzilate (³H-QNB; a non-selective M1 and M2 antagonist) and ³H-Pirenzepine (³H-PZ; a selective M1 antagonist), was investigated. For comparison purposes, oxotremorine (an M2>M1 tertiary agonist), oxotremorine-M and carbachol (mixed M2 and M1/M2 quaternary agonists), AF102B (see U.S. Patent No. 4,855,290) and McN-A-343 (an M1 quaternary agonist) were included. The results are shown in Tables 4 and 5.

**Table 4:**

| Apparent affinity states for M1 (displacement of ³H-PZ from rat cerebral cortex) and for M2 muscarinic receptors (displacement of ³H-QNB from rat cerebellum) | | | | | | | | | | | |
|---|---|---|---|---|---|---|---|---|---|---|---|
| COMPD. # | CORTEX | | | | CEREBELLUM | | | | RATIOS | | |
| | 3H-PZ-Binding | | % | | ³H-QNB-Binding | | % | | | | |
| . | K_{H} | K_{L} | | | K_{H} | K_{L} | | | | | |
| | | | H | L | | | H | L | | | |
| | (a) | (b) | | | (c) | (d) | | | c:a | d:b | b:a |
| | µM | | | | µM | | | | | | |
| 1 | 3.4 | 42 | 35 | 55 | 0.60 | 39 | 55 | 45 | 0.18 | 0.93 | 12 |
| 2 | 0.1 | 6.7 | 53 | 47 | 0.069 | 5.2 | 49 | 51 | 0.69 | 0.78 | 67 |
| 3 | | | | | 0.014 | 0.58 | 35 | 65 | | | |
| 4 | 1 | * | 100 | * | 6 | * | 100 | * | 6 | * | * |
| 5 | 0.02 | 18 | 24 | 76 | 4.5 | 79 | 49 | 51 | 225 | 4.4 | 900 |
| 8 | 4 | * | 100 | * | 3.8 | * | 100 | * | 0.95 | * | * |
| 9 | 3.8 | * | 100 | * | 24.4 | * | 100 | * | 6.4 | * | * |
| 13 | 0.6 | 3 | 28 | 72 | 5.0 | * | 100 | * | 83 | | |
| 14 | 1.3 | * | 100 | | 3.9 | * | 100 | * | 3.0 | * | * |
| 15 | 0.08 | * | 100 | * | 1.37 | * | 100 | * | 17.1 | * | * |

Displacements of ³H-PZ and ³H-QNB binding were performed in 10 and 50 mM phosphate buffers, respectively. Kᵢ = K_{H} or K_{L} were as expressed in Table 3. K_{D} values for ³H-PZ and ³H-QNB were 13.9 and 0.093 nM, respectively (Fisher et al, J. Pharmacol. Exptl. Therap., 1991, in the press).

**Table 5**

| Apparent affinity states for M1 (displacement of ³H-QNB from rat cerebral cortex) and for M2 muscarinic receptors (displacement of ³H-QNB from rat cerebellum) | | | | | | | | | | | | |
|---|---|---|---|---|---|---|---|---|---|---|---|---|
| COMPD. # | CORTEX | | | | CEREBELLUM | | | | | RATIOS | | |
| | ³H-QNB-Binding | | % | | ³H-QNB-Binding | | % | | | | | |
| | K_{H1} | K_{L1} | | | K_{H2} | K_{L2} | | | | | | |
| | | | H1 | L1 | | | H2 | L2 | | | | |
| | (a) | (b) | | | (c) | (d) | | | c:a | d:b | b:a | d:c |
| | µM | | | | µM | | | | | | | |
| 1 | 0.6 | 235 | 28 | 72 | 0.75 | 50 | 61 | 39 | 1.25 | 0.21 | 392 | 67 |
| 2 | 0.11 | 37 | 26 | 74 | 0.069 | 5.2 | 49 | 51 | 0.6 | 0.14 | 336 | 75 |
| 3 | 0.016 | 1.6 | 13 | 87 | 0.014 | 0.58 | 35 | 65 | 0.9 | 0.36 | 100 | 41 |
| 4 | 1.8 | * | 100 | * | 5.9 | * | 100 | * | 3.3 | * | * | * |
| 5 | 1.4 | 67 | 13 | 87 | 4.9 | 83 | 51 | 49 | 3.5 | 17 | 49 | 17 |
| 8 | 14 | * | 100 | * | 3.8 | * | 100 | * | 0.27 | * | * | * |
| 9 | 5.84 | * | 100 | * | 11 | * | 100 | * | 1.9 | * | * | * |
| 13 | 112 | * | 100 | * | 50 | * | 100 | * | 0.4 | * | * | * |
| 15 | 0.8 | * | 100 | * | 1.37 | * | 100 | * | 1.7 | * | * | * |

| | | | | | | | | | | | | |
|---|---|---|---|---|---|---|---|---|---|---|---|---|
| *Competition curves were significantly better fitted to a one site-model (mass-action curve). Note: the K values were generated by a non-linear, least-squares curve fitting of the data using the GRAPHPAD-2 two- and one- site analysis and statistics, as expressed in Table 1. | | | | | | | | | | | | |

Displacement of ³H-QNB binding (K_{D} value for ³H-QNB of 0.093nM) was performed in 50 mM phosphate buffer for one hour at 37°C (Fisher et al, J. Pharmacol. Exptl. Therap., 1991, in the press).

It may be seen from Tables 4 and 5 that AF150 shows high selectivity for M1 muscarinic receptors. In fact, where AF125 shows an apparent M2 selectivity, AF150 surprisingly shows a high selectivity for M1 receptors.

### Test 4: Modification of muscarinic agonist binding to cortical membranes by Cu⁺⁺ treatment.

Transition metal ions and guanine nucleotides exert strong effects on the affinity of agonists for muscarinic receptors, which probably result from modification in the proportions of the different agonist binding states (Gurwitz and Sokolovsky, Biochem. Biophys. Res. Commun. 96: 1296-1301, 1980; Aronstam et al, Mol. Pharmacol. 14: 575-582, 1978). Addition of Cu⁺⁺, which like certain other transition metal ions are known to form stable coordination complexes with protein sulfhydryl residues, has been shown to increase the apparent affinity of muscarinic agonists by increasing the proportion of high affinity sites and eliminating the sensitivity to guanine nucleotides (Farrar and Hoss, Trans. Am. Soc. Neurochem. 13: 250, 1982; Gurwitz et al, Biochem. Biophys. Res. Commun. 120: 271-276, 1984).

As shown in Table 6, Cu⁺⁺ treatment did not modify the affinity observed for the classical non-selective muscarinic antagonists atropine and scopolamine, as well as for the M1 selective antagonist pirenzepine. On the other hand, binding parameters of the classical non-selective agonists, carbachol and oxotremorine, showed that Cu⁺⁺ treatment increased their apparent affinity, i.e. the competition curves were shifted to lower agonist concentrations. Analysis of the competitive binding data according to a two-site model is summarized in Table 6, and indicates that Cu⁺⁺ treatment increased the proportion of high affinity binding sites from 32 to 51% for oxotremorine and from 25 to 44% for carbachol with no significant changes in their two affinity constants K_{H} and K_{L}.

The binding parameters for AF102B were also affected by Cu⁺⁺, in that, as shown by the data in Table 6, its interaction with untreated membranes represents binding to a single population of sites with Kᵢ = 3.3 µM, whereas following Cu⁺⁺ treatment a high affinity binding state was also observed, representing 26% of the total sites with K_{H} = 0.02 µM. No change was observed in the low affinity binding constant. Thus, it is shown that AF102B interacts with muscarinic receptors in the cerebral cortex membrane preparation in a fashion typical for agonists, i.e. the receptor-ligand complex may interact with G-protein(s) (c.f. discussion by Gurwitz et al, 1984).

AF150 also exhibited agonistic binding characteristics in these studies. In untreated membranes it showed multiple affinity states (0.32 µM and 33.5 µM for the high and the low affinity sites, respectively). The proportion of the high affinity sites was increased from 14 to 37% following Cu⁺⁺ treatment, suggesting that AF150 might behave as an agonist for cerebral cortex muscarinic receptors.

**Table 6:**

| Kᵢ values using (-)³H-QNB binding to rat cerebral cortex homogenates with and without Cu⁺⁺ treatment | | | | | | |
|---|---|---|---|---|---|---|
| Agonist # | without pretreatment | | | with Cu⁺⁺ pretreatment | | |
| | K_{H}(µM) | K_{L}(µM) | %H* | K_{H}(µM) | K_{L}(µM) | %H* |
| 1 | 0.893±0.07 | 27.0±0.7 | 25±1 | 0.11±0.010 | 17.5±3.2 | 44±3 |
| 3 | 0.02±0.005 | 13.3±1.4 | 32±3 | 0.03±0.801 | 7.2±1.7 | 51±7 |
| 4 | | 3.3±0.6 | 0 | 0.02±0.001 | 3.0±0.01 | 26±5 |
| 5 | 0.32±0.060 | 33.5±1.8 | 14±1 | 0.52±0.003 | 18.0±2.0 | 37±2 |
| 9 | 0.34±0.1 | 8.5±0.8 | 13±5 | 0.18±0.09 | 5.7±0.2 | 30±7 |

| | K_{H}(nM) | K_{L}(nM) | %H* | K_{H}(nM) | K_{L}(nM) | %H* |
|---|---|---|---|---|---|---|
| 10 | 19.5±0.5 | 695±5 | 72±3 | 12±2 | 560±60 | 64±6 |
| 11 | | 0.38±0.011 | 0 | | 0.2±0.04 | 0 |
| 12 | | 0.09±0.004 | 0 | | 0.1±0.005 | 0 |

| | | | | | | |
|---|---|---|---|---|---|---|
| *% of high affinity state. | | | | | | |

In the above Table, stated values are means ±SEM of at least 3 experiments; each experiment was performed in triplicate samples. The assay was performed in modified Krebs buffer at pH 7.4 for 2 hours at 25°C with and without pretreatment for 30 minutes if rat cerebral cortex membranes with Cu⁺⁺ (100 µM) (Fisher et al, J. Pharmacol. Exptl. Therap., 1991, in the press).

### Test 5: The effects of test compounds on the expression in cell lines of subtypes of muscarinic receptors

On the basis of pharmacological data, muscarinic receptors have been divided into three subtypes (M1, M2 and M3). Molecular cloning studies have identified five genetically distinct muscarinic receptor subtypes (m₁-m₅). Functional expression of these genes has indicated a correlation between the genetically and pharmacologically defined subtypes, where M1≡m₁, m₄ and m₅; M2≡m₂; and M3≡m₃ (Buckley et al, Mol. Pharmacol. 35: 469-476, 1989).

Stimulation of m₁ and m₃ receptors elicit dose-dependent increases in the hydrolysis of phosphoinositides (PI). Agonist activation of the m₁ receptor also elicits increases in basal and forskolin-stimulated cAMP, whereas the m₃ receptor has no effect on intracellular cAMP levels (Buck and Fraser, Biochem. Biophys. Res. Comm. 173: 662-672, 1990). Stimulation of the m₂ and m₄ receptors by muscarinic agonists leads to preferential inhibition of adenylate cyclase (AC) activity (Mei et al, Life Sciences, 45: 1831-1851, 1989).

The new compounds AF150, AF151, AF150(S) and AF125 were assayed for their ability to stimulate PI in CHO cells transfected with m₁ and m₃ receptors, respectively (Buck and Fraser, loc cit), and in cultured human neuroblastoma cells, line SK-N-SH, which express mainly the m₃ subtype, but not the m₁ subtype (Pinkas-Kramarski et al, Neurosci. Lett. 188: 335-340, 1990; Luthin et al Mol. Pharmacol. 34: 327-333, 1988).
These compounds were also assayed on cultured PC12 cells which express mainly m₄ subtype receptors and are coupled to inhibition of AC activity (Pinkas-Kramarski et al, loc cit).

Stimulation of PI breakdown was assayed using the method described by Stein et al (EMBO J. 7: 3031-3035, 1988), and modulation of AC activity was assayed by the method of Stein et al, loc cit and Pinkas-Kramarski et al (FEBS Lett., 239: 174-178, 1988). The results are shown in Table 7 and are compared with carbachol, a full muscarinic agonist (mixed M1/M2 type) and with AF102B.

**Table 7:**

| Effects of the test compounds on PI hydrolysis and AC activity in cell cultures expressing m₁, m₃ and m₄ receptor subtypes. | | | | | | | | |
|---|---|---|---|---|---|---|---|---|
| Compound # | CHO transfected with | | | | SK-N-SH | | PC12 | |
| | m₁ | | m₃ | | m₃ | | m₄ | |
| | PI | EC₅₀ | PI | EC₅₀ | PI | EC₅₀ | AC | EC₅₀ |
| 1 | +++ | 8 | +++ | 5 | +++ | 25 | +++ | 1.5 |
| 4 | +20%^{a} | 30^{b} | - | c | - | d | +40%^{a} | 0.1^{b} |
| 5 | +++ | 13 | +38%^{a} | 10^{b} | +40%^{a} | 3 | +++ | 1 |
| 6 | +++ | 54 | +45%^{a} | 10^{b} | NT | | +++ | 1 |
| 7 | +17%^{a} | NT | NT | | NT | | NT | |
| 8 | +14%^{a} | NT | NT | | +20%^{a} | | NT | |
| Notes to Table 7: EC₅₀: effective concentration (µM) at 50% activation +++ full agonist as compared with carbachol (full agonistic activity was tested for all compounds at 1mM) + partial agonist as compared with carbachol - no stimulation of PI or inhibition of AC PI phosphoinositides hydrolysis AC adenylate cyclase activity expressed by the change in cAMP concentration NT not tested a: % of carbachol effect b: effective concentration (µM) which induces the effect c: 22µM AF102B inhibited the effect of 1mM carbachol d: 1mM AF102B inhibited the effect of 1mM carbachol | | | | | | | | |

In Table 7, the agonistic effect on m₁ receptors is evidenced by increased PI hydrolysis and AC activity, whereas stimulation of m₄ receptors is reflected by inhibition of AC activity; m₃ stimulation leads to increased PI hydrolysis. From this Table, it is evident that surprisingly both AF150 and AF151 are full agonists on the m₁ and m₄ muscarinic receptor subtypes, but partial agonists on the m₃ subtypes. In addition, while AF150 and AF151 are full agonists on m₁ receptors as evidenced by PI hydrolysis, these compounds differ surprisingly from carbachol as evidenced from their very weak effect on potentiation of AC activity on the same receptors. Thus, carbachol-induced activation of m₁ muscarinic receptors elicits increases in basal and forskolin-stimulated cAMP (232-356% and 546-1382%, respectively), yet both AF150 and AF151 are equipotently very weak in this test (33% and 76%, respectively).

In a typical experiment with SK-A-SH neuroblastoma cells, it was found that the accumulation of inositol-1-phosphate (IP₁) was stimulated 7.46-fold over basal level by 10 mM carbachol, 5.24-fold by 1 mM oxotremorine-M, and 1.42-fold by 1 mM AF125. In contrast, the M1-selective agonist AF102B (US Patent No. 4,855,290) failed to stimulate phosphoinositide hydrolysis in these cells even at 1 mM, while at that concentration it could completely block the oxotremorine-M-induced signal. This inhibition was dependent on oxotremorine-M concentration; for example, 1 mM AF102B completely blocked phosphoinositide hydrolysis induced by 10 µM oxotremorine-M while inhibiting the signal of 1 mM oxotremorine-M by 34%.

In addition, the combined stimulation of phosphoinositides hydrolysis by carbachol and certain of the new compounds was checked: in the same experiment, the accumulation of IP₁ was stimulated 6.11-fold over basal level by a combination of 10 mM carbachol with 1 mM AF125. Hence, the new compound AF125 stimulates phosphoinositides hydrolysis in SK-N-SH human neuroblastoma cells to about 20-25% of the stimulation obtained with the non-selective full agonist cabachol, while only minimally (10-20%) interfering with the effect of carbachol. The significance of these results is that AF125 shows a unique activity as a muscarinic agonist in this cell line which expresses mainly m₃ muscarinic receptor subtype.

It is concluded that these compounds show a unique activity and selectivity and could therefore be particularly useful for the treatment of SDAT (Braan et al, FEBS Lett. 230: 90-94, 1988).

### Test 6: The effect of test compounds on synaptosomal acetylcholine (ACh) release.

The effects of AF150 (and for comparison purposes, AF125, MeN-A-343 and oxotremorine) on basal- and K^{**+**}-evoked release of ³H-ACh were studied on synaptosomes prepared from rat cerebral cortex using the methods described in detail by Pittel et al (J. Neurochem., 55: 665-672, 1990). Results are shown in Table 8. Surprisingly, the two closely related structures, AF150 and AF125, had differing effects on ³H-ACh release. Thus, while AF125(1mM) inhibited the K⁺-evoked release of ACh, AF150(1mM) surprisingly potentiated it. These results indicate that AF125 acts on presynaptic receptors like oxotremorine, a classical M2 agonist. On the other hand, the action of AF150 on presynaptic muscarinic receptors, causing an increase in K⁺-evoked ACh release, can be considered either as an M2 antagonistic or an M1 agonistic effect. Hadhazy and Szerb (Brain Res. 123: 311-322, 1977) and Gulya et al (Neurochem. Int. 15: 153-156, 1989) demonstrated that muscarinic antagonists stimulate the K⁺-evoked ACh release. Suzuki et al (Neurosci. Lett. 84: 209-212, 1988) and Pittel et al, loc cit, showed that muscarinic antagonists can also stimulate the basal ACh release. The latter study suggests that stimulatory muscarinic receptors that modulate ACh release are present in the CNS and they can be pharmacologically considered of the M1 subtype. The existence of muscarinic autoreceptors that increase ACh release was manifested also in the PNS in smooth muscle preparation (Kilbinger and Nafziger, Naunyn Schmiedebergs Arch. Pharmacol. 328: 304-309, 1985).

**Table 8:**

| Effects of test compounds on basal and K⁺-evoked release of acetylcholine (ACh) from rat cerebral cortex synaptosomes. | | | |
|---|---|---|---|
| Compound # | Concn. mM | Basal ³H-ACh release % | K⁺-evoked ³H-ACh release % |
| 3 | 0.1 | NT | 56 |
| 5 | 0.1 | 121 | 110 |
| 5 | 1.0 | NT | 115±4 (6)* p<0.01 |
| 5 | 0.1 | | |
| + | | | |
| 10 | 10◆ | 87 | 91 |
| 8 | 1.0 | NT | 78±9 (2)* |
| 9 | 0.1 | 144 | 172 |

| | | | |
|---|---|---|---|
| Notes to Table 8: results are from 1-3 experiments unless specified otherwise* NT = not tested ◆nM | | | |

The antagonistic effect of pirenzepine (an M1 antagonist) on the potentiating effects of AF150 on K⁺-evoked ³H-ACh release (as noted from Table 8), could indicate that AF150 can be considered an M1 agonist, which thus activated M1 excitatory autoreceptors. However, regardless of the mechanism of action of AF150, i.e. whether this can be attributed to, e.g., (a) M1 post- and pre-synaptic agonistic activity, or (b) M1 postsynaptic agonistic and an M2 pre-synaptic antagonistic effect, this compound could be especially promising for the control of SDAT and related disorders.

### Test 7: Electrophysiological evaluation of AF150 in rat hippocampal slices.

Acetylcholine (ACh) has a number of effects in mammalian brain. The main effects were observed in the hippocampal slice and include:
(1) a blockade of sustained outward potassium (K⁺) current activated by depolarization (I_{M});
(2) a blockade of Ca⁺⁺-dependent K⁺ current underlying the slow after hyperpolarization (AHP);
(3) a blockade of sustained K⁺ current at rest;
(4) a transient outward K⁺ current;
(5) reported effects of ACh on postsynaptic potentials (an increase in spontaneous and decrease in evoked ones) and on Ca⁺⁺ currents.
(Segal, Brain Res. 452: 79-98, 1988; Duttar and Nicoll, J Neurosci. 8: 4214-4224, 1988.)

The results using gallamine (an M2 antagonist) and pirenzepine (PZ, an M1 antagonist), suggest that an M2 receptor could mediate the depression of EPSP and blockade of I_{M}. In contrast, the depolarization and the blockade of the AHP, insensitive to gallamine and blocked by 0.3 µM of PZ, are likely to be mediated by M1 receptors (Duttar and Nicoll, loc cit).

In general terms the effects of microdrops of AF150, ACh and PZ were applied to rat hippocampal cells recorded intracellularly. The exact concentrations of the drug at the receptor sites are not known, using this method. However, this method has an advantage over the perfusion method, in that it gives a more correct picture from the electrophysiological point of view (Segal, loc cit).

Intracellular responses to AF150 of CA1 neurons in the hippocampal slice were recorded, using the method of Segal. Small hyperpolarizing current pulses (current traces not shown) were alternated with stimulation of the Schaffer-commissural afferents producing postsynaptic potentials. AF150 applied topically, by microdrops (100 µM) caused a potent depolarizing response. Hyperpolarizing the cell to control potentials, reveals a real increase in input resistance at rest. AF150 blocks the slow AHP mediated by a Ca⁺⁺-dependent K⁺ conductance. AF150 causes a small reduction in postsynaptic potentials (PSPs). Pirenzepine (10µM, an M1 muscarinic antagonist) markedly reduced the depolarization caused by AF150. The increase in input resistance at rest, was also prevented by pirenzepine. The blockade of the slow AHP by AF150 was markedly reduced in pirenzepine-treated slices. The reduction in PSPs cause by AF150 was unaffected by pirenzepine.

### Conclusions

(1) AF150 causes depolarization, increased input resistance, and blocks the slow AHP, which effects are antagonized by the M1-receptor antagonist pirenzepine; however, an effect on PSPs which was small compared to ACh was detected and was unaffected by pirenzepine.
(2) Surprisingly, AF150 appears to be a more specific agonist for M1 receptors than for M2 receptors.
(3) When compared with AF102B, it is clear that AF150 is far more efficacious and causes a depolarization (blocked by pirenzepine) which is not shown in the case of AF102B. Both AF150 and AF102B block the slow AHP (pirenzepine-sensitive M1 effect).

### Test 8: Pharmacodynamic Profile and General Toxicity

### PART A

### 1. Objective:

The objective of this study was to establish the primary pharmacodynamic profile and to evaluate the general toxicity of AF150, by observing mice after injection at 3 dose levels of AF150.

### 2. Materials and Methods:

a. Animals: Mice, Male CD strain, 20-30g.
b. Group size: n=5.
c. Route of administration: Intraperitoneal (i.p.).
d. Dose levels: 1, 5 and 10 mg./kg.
e. Volume of dosage: 10 ml./kg.
f. Preparation of Test Material: AF-150 was dissolved in saline solution.
g. AF150 at 3 dose levels was administered i.p. to mice (n=5). Behavioral changes up to two hours after dosing were recorded as well as additional observations up to 24 hours administration for death occurrence. Analgesic effects were evaluated by the tail pinch method at 15, 30, 60 and 120 minutes post dosing. Except for analgesia, animals were observed for the following effects: the occurrence of tremors, convulsions, respiratory distress, salivation, diarrhea, changes in pupil diameter, exaphthalamus, motor coordination, hypo- or hyperactivity and vocalization.

### 3. Experimental Results

1 mg./kg. i.p.: Very slight salivation and diarrhea was observed for about 30 minutes after injection. No analgesia was seen at any of the times of observation. No other central or autonomic effects were observed.
5 mg./kg i.p.: Decreased motor activity was observed up to 60 minutes after administration, then normal activity was regained. Partial analgesia was seen 15 minutes after injection. Motor coordination was reduced up to 30 minutes after injection. Mydriasis was observed 5 minutes after injection. Slight salivation and diarrhea was seen from 10 to 60 minutes after injection.
10 mg./kg. i.p.: Complete hypoactivity was observed from 5 to 75 minutes in 2 of the animals, the other 3 regained their activity after 120 minutes. Slight tremors were observed 10 minutes after injection for a short period. Motor coordination was markedly affected throughout the 2 hours of observation. Analgesia was complete at 15 and 30 minutes after injection; no analgesia was seen at ≥ 60 minutes. Moderate salivation, diarrhea, lacrimation and mydriasis were observed 5 minutes after injection and became severe 10 minutes after injection, but severity declined and all signs were absent 90 minutes after injection.

### PART B

Male white mice of the CD-1 strain (Charles River, UK), within the body weight range of 18-26 g, were used throughout this study, carried out on AF123, AF125 and AF125(N).

### AF125

Dose-range finding trials with AF125 injected i.p. into mice at dose levels ranging from 50 to 200 mg./kg., indicated that the toxic-lethal dose falls within the 100 to 200 mg./kg. range. Furthermore, these initial trials clearly established that the major signs of reactions to treatment consisted primarily of signs characteristic for potential cholinomimetic activity of the test material, such as tremors, salivation, lacrimation, diarrhea, analgesia, hypothermia and vasodilation. Survivors exhibited initial signs of recovery at about 2 to 3 hours post-injection. Results following p.os and i.v. injection of AF125 at non-lethal doses are listed in the following Table 9.

**Table 9**

| Dose | Route of Admin | Major Signs Observed* | | | | | | | | | | |
|---|---|---|---|---|---|---|---|---|---|---|---|---|
| mg/kg | | Tre | Con | Res | Sal | Lac | Dia | Ana | Myd | Mot | Pil | Hyp |
| 40 | i.v. | 5/5 | 1/5 | 3/5 | sev | sev | mod | 5/5 | mod | dec | mod | mod |
| 20 | i.v. | 2/5 | 0/5 | 1/5 | sev | sev | mod | 5/5 | mod | dec | mod | mod |
| 10 | i.v. | 0/5 | 0/5 | 0/5 | mod | sli | sli | 5/5 | sli | dec | sli | sli |
| 5 | i.v. | 0/5 | 0/5 | 0/5 | sli | sli | | 5/5 | sli | dec | | |
| 50 | p.os | 2/5 | 0/5 | 0/5 | mod | mod | sli | 5/5 | sli | dec | mod | sli |
| 40 | p.os | 0/5 | 0/5 | 0/5 | sli | mod | sli | 3/5 | | dec | sli | sli |
| 20 | p.os | 0/5 | 0/5 | 0/5 | sli | sli | | 0/5 | | | | |
| 10 | p.os | 0/5 | 0/5 | 0/5 | | | | | | | | |

| | | | | | | | | | | | | |
|---|---|---|---|---|---|---|---|---|---|---|---|---|
| *Numerical values of data presented refer to no. of animals affected out of the total no. of animals treated. For other abbreviations see details below: Tre = Tremors Con = Convulsive seizure, mostly of the clonic-tonic type Res = Respiratory distress, mostly hyperpnoea and tachypnoea Sal = Salivation: sli = slight; mod = moderate; sev = severe Lac = Lacrimation (sli; mod; sev); Dia = Diarrhea (sli; mod; sev) Ana = Analgesia, indicated by lack of response to tail pinching Myd = Mydriasis (sli; mod; sev) Mot = Altered spontaneous motor activity, either increased (inc) or decreased (dec) Pil = Piloerection (sli; mod; sev) Hyp = Hypothermia (sli; mod; sev) | | | | | | | | | | | | |

Median Lethal Dose (LD₅₀) of AF125

The following are the LD₅₀ (95% Confidence Limits) values obtained;
I. Intravenous Injection 68.9 mg./kg. (66.6 - 71.2)
II. Oral Administration 134.4 mg./kg. (118.4 - 152.5)

In view of the primary screening results obtained in mice, AF125 appears to possess primarily cholinomimetic activity. It should also be pointed out that with respect to duration of activity, onset of recovery occurred at about 1 to 2 hours after treatment, pending on the dose administered. Particularly under conditions of oral dosing, the comparative onset time of recovery appeared to be relatively rapid. The seemingly narrow ratio between the two respective LD₅₀ values, as well as the comparative "active" dose levels after either i.v. or p.os treatment, indicate that AF125 is fairly rapidly absorbed by the enteric route.

### AF125(N), AF123

The general pharmacological effects of these compounds are shown in Table 10.

**Table 10**

| Compound | Dose | Route of Admin | Mortality | Major Signs Observed* | | | | | | |
|---|---|---|---|---|---|---|---|---|---|---|
| | mg/kg | | no. time | Tre | Con | Res | Sal | Myd | Mot | P/H |
| AF125(N) | 100 | i.v. | 5/5 immed | | | | | | | |
| | 50 | i.v. | 5/5 immed | | | | | | | |
| | 20 | i.v. | 0/5 | 3/5 | | 4/5 | sli | sli | dec | |
| | 200 | p.os | 0/5 | | | | | sli | inc | sli |
| AF123 | 200 | i.v. | 5/5 immed | | | | | | | |
| | 100 | i.v. | 5/5 1 min. | | 5/5 | | | | | |
| | 50 | i.v. | 0/5 | | | | | | | |
| | 400 | p.os | 5/5 3 mins. | 5/5 | 5/5 | 5/5 | | | | |
| | 200 | p.os | 0/5 3 mins. | 4/5 | | | | | | |

| | | | | | | | | | | |
|---|---|---|---|---|---|---|---|---|---|---|
| *Numerical values of data presented refer to no. of animals affected out of the total no. of animals treated. For other abbreviations see details above, and additionally: P/H = Piloerection/Hypothermia (sli;mod;sev) | | | | | | | | | | |

### Test 9: The effects of test compounds in scopolamine-treated rats in a passive avoidance (PA) paradigm.

Naive male Sprague-Dawley rats, three months old (obtained from Charles River Breeding, U.K.), were used (weighing 200-300 g.). The passive avoidance (PA) test is according to Fisher et al, Neurosci. Lett. 102: 325 (1989), except that in these rats amnesia was induced by scopolamine.

Eight groups of 17-20 naive rats were used. Each group was divided into two subgroups of 7-10 rats each: subgroup 1 was injected with scopolamine HBr (0.5 mg./kg. in saline, s.c., 15 min. before the shock) and subgroup 2 was injected with saline (1 ml./kg., s.c., 15 min. before the shock). Seven of the groups were treated within one minute after the shock with one of the following doses of the tested compound: 0.1, 0.5, 1, 3, 5, 8, 10 mg./kg. (in saline, i.p.) and one group received saline (1 ml./kg., i.p.).

### AF125

The initial latency and retention latency measures were analyzed by a two-way ANOVA (pre-shock scopolamine treatment/post-shock AF125 treatment). Tables 11 and 12 present the means +S.E.M. of the initial and retention latencies, respectively.

**Table 11:**

| Initial latency measures of preshock scopolamine-postshock AF125 treated rats | | | | |
|---|---|---|---|---|
| | Pre-shock | | | |
| Post-shock AF125 | Saline | | Scopolamine | |
| Dose (mg/kg) | Mean | S.E.M. | Mean | S.E.M. |
| Saline | 28.7 | 6.1 | 23.5 | 9.8 |
| 0.1 | 49.5 | 10.9 | 33.6 | 15.8 |
| 0.5 | 42.0 | 9.0 | 47.4 | 19.0 |
| 1.0 | 36.0 | 14.5 | 23.3 | 4.9 |
| 3.0 | 51.9 | 12.9 | 47.3 | 11.8 |
| 5.0 | 34.7 | 11.4 | 51.3 | 14.6 |
| 8.0 | 41.2 | 6.8 | 53.9 | 12.2 |
| 10.0 | 46.3 | 14.0 | 45.9 | 12.6 |

**Table 12:**

| Retention latency measure of preshock scopolamine-postshock AF125 treated rats | | | | |
|---|---|---|---|---|
| | Pre-shock | | | |
| Post-shock AF125 | Saline | | Scopolamine | |
| Dose (mg/kg) | Mean | S.E.M. | Mean | S.E.M. |
| Saline | 600.0 | 0.0 | 97.9 | 42.5 |
| 0.1 | 572.8 | 27.2 | 128.8 | 69.4 |
| 0.5 | 600.0 | 0.0 | 222.7 | 76.5 |
| 1.0 | 555.8 | 41.1 | 261.2 | 82.7 |
| 3.0 | 530.4 | 49.5 | 296.0 | 84.1 |
| 5.0 | 426.7 | 53.4 | 227.3 | 81.4 |
| 8.0 | 594.1 | 6.0 | 294.9 | 98.0 |
| 10.0 | 498.3 | 59.0 | 288.3 | 103.2 |

### CONCLUSIONS

The reversal screening experiment using scopolamine as a pre-shock treatment and AF125 as a post-shock treatment yielded a significant dose response curve. The doses of 3, 5, 8 and 10 mg./kg. had an obvious advantage as compared to all other doses. Rats treated with the above doses exhibited the longest retention latencies when compared to the saline treated rats. The lower doses exhibited short retention latencies indicating no improvement in cognitive functions. No significant differences were found in the initial latencies between all the groups tested.

The effect of the post shock AF125 treatment was found significant (F=104.77, df=1/126, p<0.001), 10 mg./kg. (p<0.05), increased significantly the retention latency of the scopolamine-treated rats. In addition, AF125 (5mg./kg.) significantly (p<0.05) decreased the retention latency of the pre-shock saline treated rats. Finally, a significant difference was found between the retention latency of the preshock scopolamine/postshock saline treated rats and the pre-shock saline/post-shock saline treated rats (p<0.001). All pre-shock saline treated rats who received AF125 post-shock in the doses: 3, 5, 8 and 10 mg./kg. had side effects. The group which received 3 mg./kg. had slight diarrhea, the 5 mg./kg. treated group exhibited stronger diarrhea and the 8 and 10 mg./kg. treated groups had severe diarrhea with lacrimation. It is important to note that the pre-shock scopolamine treated rats did not exhibit any side effects following AF125 treatment.

Compared to AF102B (US Patent No. 4,855,290), AF125 showed a wider dose-response curve; AF102B had a significant enhancing effect on retention-behavior, using the doses of 3 and 5 mg./kg., while the enhancing effect of AF125 continued along a wider range from 3 to 10 mg./kg. All these results would indicate that the beneficial effect of AF125 in this model are due to its potent central agonistic effect on muscarinic receptors which are involved in cognitive functions.

### AF125(N)

### Subjects

Naive male Sprague-Dawley rats, three months old (obtained from Charles-River Breeding, U.K.), were used (weighing 200-300 g.).

### Behavioral test

Eight groups of 18-20 naive rats each were used. Each group was divided into two subgroups of 9-10 rats each: subgroup 1 was injected with scopolamine HBr (0.5 mg./kg. in saline, s.c., 15 min. before the shock) and subgroup 2 was injected with saline (1 ml./kg., s.c., 15 min. before the shock). Seven of the groups were treated, within one minute after the shock, with one of the following doses of AF125(N): 0.1, 0.5, 1, 3, 5, 8 or 10 mg./kg. (in saline, i.p.) and one group received saline (1 ml./kg., i.p.).

The initial latency and retention latency measures were analyzed by a two way ANOVA (pre-shock scopolamine treatment/post-shock AF125(N) (N) (treatment). Tables 13 and 14 present the mean ±S.E.M. of the initial and retention latencies, respectively.

**Table 13:**

| Initial latency measures of preshock scopolamine-postshock AF125(N) treated rats | | | | |
|---|---|---|---|---|
| | Pre-shock | | | |
| Post-shock AF125N | Saline | | Scopolamine | |
| Dose (mg/kg) | Mean | S.E.M. | Mean | S.E.M. |
| Saline | 26.7 | 4.7 | 39.1 | 17.6 |
| 0.1 | 21.0 | 2.7 | 22.3 | 5.6 |
| 0.5 | 28.1 | 7.7 | 62.9 | 17.4 |
| 1.0 | 22.0 | 4.2 | 29.1 | 14.5 |
| 3.0 | 44.1 | 14.1 | 23.1 | 4.0 |
| 5.0 | 65.1 | 18.5 | 24.6 | 5.3 |
| 8.0 | 19.3 | 5.4 | 16.3 | 2.7 |
| 10.0 | 4.9 | 2.2 | 21.5 | 5.3 |

A significant difference (F(7/140)=2.37, p<0.05) in the initial latency was found between the groups treated with AF125(N) (Table 13). An interaction between AF125(N) treatments and the preshock treatment (saline vs scopolamine) was also found (F(7/140)=22.43, p<0.05). A Scheffe test showed, more specifically, that the initial latency for the preshock scopolamine-postshock AF125(N) 0.5 mg./kg. treated rats (62.9±17.4 sec.) was significantly (p<0.025) longer than the initial latency for the control group (preshock scopolamine-postshock saline, 39.1±17.6 sec.). The preshock saline-postshock AF125(N) 5 mg./kg. treated group also had a significantly longer initial latency when compared to the control group: preshock saline-postshock saline treated group (65.1±18.5 vs 26.7±4.7 sec., p<0.001). The effect of the post-shock AF125(N) treatment was found to be significant (F(7/140)=3.51, p<0.05) and Table 14). A Scheffe test showed specifically that the dose of 0.5 mg./kg. of AF125(N) increased significantly the retention latency of the scopolamine-treated rats when compared to the saline treated rats (332.7±80.9 vs 141.1±5.5 sec, p<0.005).

**Table 14:**

| Retention latency measure of preshock scopolamine-postshock AF125(N) treated rats | | | | |
|---|---|---|---|---|
| | Pre-shock | | | |
| Post-shock AF125N | saline | | Scopolamine | |
| Dose (mg/kg) | Mean | S.E.M. | Mean | S.E.M. |
| Saline | 483.6 | 64.9 | 141.1 | 55.5 |
| 0.1 | 554.7 | 22.5 | 199.7 | 71.7 |
| 0.5 | 600.0 | 0.0 | 332.7* | 80.9 |
| 1.0 | 448.9 | 56.7 | 114.8 | 56.2 |
| 3.0 | 500.1 | 44.0 | 243.4 | 69.2 |
| 5.0 | 290.3 | 52.1 | 181.6 | 58.2 |
| 8.0 | 552.0 | 28.3 | 210.2 | 61.5 |
| 10.0 | 417.5 | 64.9 | 129.7 | 42.2 |

| | | | | |
|---|---|---|---|---|
| *p<.005, compared to saline | | | | |

In addition, AF125(N) (5 mg./kg., i.p.) significantly decreased the retention latency of the pre-shock saline treated rats when compared to their control group (290.3±52.1 vs 483.6±64.9 sec., p<0.005). Finally, a significant difference was found between the retention latency of the pre-shock scopolamine/post-shock saline treated rats and the pre-shock saline/post-shock saline treated rats (141.1±55.5 vs 483.6±64.9, p<0.001). The treatment with AF125(N) did not cause obvious side effects in any of the doses given either post-scopolamine or post-saline administration. The reversal screening experiment using scopolamine as a pre-shock treatment and AF125(N) as a post-shock treatment yielded a positive significant response only following one dose: 0.5 mg./kg., i.p. Rats treated with this dose exhibited longer retention latency when compared to the saline treated rats.

### Test 10: The effects of AF150 in AF64A-treated rats in a passive avoidance (PA) paradigm.

This animal model mimics to a certain extent the cholinergic hypofunction in SDAT (Fisher and Hanin, Ann. Rev. Pharmacol. Toxicol. 26: 161-181, 1986). The method described in Fisher et al (J. Pharmacol. Exptl. Therap., 1991, in the press) and in Fisher et al (Neurosci. Lett. 102: 325-331, 1989) was used here. The results are shown in Table 15.

As may be seen from the Table, AF150 was beneficial in this animal model at all three doses tested, by i.p. injection. In addition, AF150 (in free base form) improved significantly the retention latency of A64A-treated rats when it was administered orally at 0.2 and 1.0 mg./kg. (not shown). The latter finding indicates that the compound is well absorbed and effective when administered orally, at very low doses. Notably, the low effective doses in the PA test are free of overt side effects. Thus, AF150 has a wide safety margin, as evidenced in learning and memory tests like PA.

No significant differences were found among the different groups of animals tested in the initial latency test; the initial latency ranged from 20 and 31 seconds. In all the experiments using AF150, the compound as free base was dissolved in 10 mM phosphate buffer having pH 7.35.

**Table 15:**

| Passive avoidance studies on AF150 | | | | |
|---|---|---|---|---|
| | Retention latency (secs.) after 72 hours | | | |
| | 10 mM phosphate buffer | | AF150 (mg./kg., i.p.) | |
| | | 0.1 | 0.2 | 1.0 |
| Rats treated with saline | 438.1±57.0 | 511.8±45.7 | 479.1±57.2 | 571.6±28.4 |
| Rats treated with AF64A* | 174.8±44.2 | 449.5±52.1 | 481.1±54.3 | 600.0±0 |

| | | | | |
|---|---|---|---|---|
| *3 nmole/2µl./side, icv | | | | |

Notes on Table 15. Significant interaction when F(2.54)=6.33, p<0.005. By analyzing the foregoing data according to Scheffe's main contrasts, the following significant results were found:

| | | |
|---|---|---|
| (p<0.005) | AF150 (0.1 mg./kg.) | vs. AF64A-buffer |
| (p<0.025) | A64A-AF150(0.2 mg./kg.) | vs. AF64A-buffer |
| (p<0.001) | A64A-AF150(1.0 mg./kg.) | vs. AF64A-buffer |
| (p<0.05) | A64A-AF150(1.0 mg./kg.) | vs. AF64A-AF150(0.2 mg./kg.) |
| (p<0.05) | saline-buffer | vs. AF64A-buffer |
| (p<0.005) | saline-buffer | vs. AF64A-AF150(1.0 mg./kg.) |
| (p<0.025) | saline-buffer | vs. saline-AF150(1.8 mg./kg.) |

### Test 11: The effects of AF125 and AF150 in AF64A-treated rats in an 8-arm radial maze (RAM) paradigm.

This animal model mimics the cholinergic hypofunction and cognitive dysfunction in SDAT (Fisher and Hanin, loc cit; Fisher et al, Neurosci. Lett. 102: 325-331, 1989); the method described in the latter publication was used here.

### PART A - AF125

The potential effect of AF125 in reversing memory deficits was evaluated on the performance of AF64A-injected mature rats, in the RAM.

### Methods

### a) Subjects

Forty eight mature male (11-12 months) rats which were used in this study. They were injected icv with AF64A (3 nmole/2 µl./side) 8 months earlier, at the age of three months. One week before the behavioral testing, rats were transferred to individual cages and were food restricted until reaching 90% of their free feeding weight (the rats had free access to water). The room was illuminated 12 hrs a day (6:00 to 18:00) and behavioral testing sessions were carried out during the mornings. After reaching 90% of free feeding weight, rats received about 4 food pellets per day (Altromin, 15 g.) in order to further reduce the rats' weights. Two days before behavioral testing the rats were fed with precision pellets (Bioserv Inc.) which were later used for reinforcement in the maze.

### b ) Apparatus

Behavioral tests were conducted in an elevated (70cm) 8-arms radial maze, made of PVC. The arms (75cm long and 10cm wide) extended from an octagonal central arena (40cm wide). At the end of each arm a self feeder was placed (45 mg pellet dispenser Model 8000. (Lafayette Instrument Company).

### c) Behavioral testing

### 1) Pretraining

Before starting the actual test the rats were familiarized with the RAM. Pellets were scattered in the whole area of the maze. Rats were placed in the central arena, one at a time, facing always the same direction, and were permitted to run from arm to arm until visiting all 8 arms or until 10 minutes had elapsed. Pretraining lasted two weeks (five days a week).

### 2) Training

Due to the size of the batch (48 rats) the experiment was conducted in two phases. Twelve AF64A-injected rats and 12 saline-injected rats participated in each phase. The rats were randomly assigned to the different phases. The rats in each phase were further divided into two subgroups - drug treated and controls. In each of the training days each rat was placed in the central arena 30 minutes following injection of either AF125 (0.5 mg./kg. solution in saline, i.p.) or saline (1 mg./kg., i.p.). The rats were allowed to run from arm to arm until 8 pellets were collected or until 15 minutes had elapsed. For each rat the correct choices, number of errors and total time of the five training days were grouped into 1 block. The correct choices, number of errors and total time scores were analyzed by a 2-way ANOVA (2x2) (Injection-AF64A/saline and Treatment-AF125 0.5 mg./kg./saline).

Correct Choices: A significant difference was found in the average number of correct choices out of the first eight entries between the AF64A-injected rats' performance (6.0±0.24) and that of the saline-injected rats (6.8±0.15) (F(1/36)=9.29, p<0.005). In addition a significant interaction between injection and treatment was found (F(1/36)=6.35, p<0.025). Scheffe' tests showed that AF125 improved the performance of the AF64A-injected rats (6.53±0.36) compared to that of the saline treated rats (5.49±0.26), (p<0.025). Furthermore no significant difference was found between the performance of the AF64A-injected rats treated with AF125 and that of saline-injected rats treated with saline.

Number of errors: The AF64A-injected rats made significantly more errors (7.69±1.1) than the saline-injected rats (3.5±0.59) (F=(1/36)=10.11, p<0.001). AF125 treatment did not have any effect on this parameter.

Total time: No significant difference was found between any of the groups in the total time measure.

Body weight: Comparing the mean weights of the rats before behavioral testing by a t-test revealed an overall significant difference (t=(38)=4.05, p<0.001) between the AF64A-injected rats (489.5 g.) and the saline injected rats (579 g.). The effect of AF125 on the body weights was not tested because rats were food restricted.

Side-effects: The dose of 0.5 mg./kg. used in this study caused less side-effects than the higher doses (1 and 3 mg./kg.) which were used in the MWM experiment. Only two saline-injected rats treated with AF125 had diarrhoea. Other side-effects were not found.

### CONCLUSIONS

1. AF64A-injected rats showed a significant decrease in performance which was expressed in both parameters: number of correct choices out of the first eight entries and number of errors.
2. AF125 (0.5 mg./kg., i.p.) significantly improved the performance of AF64A injected rats. This result was illustrated only by the parameter of correct out of the first eight entries.
3. The side-effects of the test-compound AF125 were minimal probably because of the low dose - 0.5 mg./kg., compared to the higher doses (1 and 3 mg./kg.) used in the MWM experiment.

### PART B - AF150

In brief, saline- and A64A-pretreated rats (3 nmole/2 µl./side, icv) were used in these experiments, 2.5 months after the lesion. The experiment was carried out over a three-week period. During the first week the rats were trained to find food pellets in all eight arms of the maze. After learning the task, the rats were trained using the delay procedure. During a predelay session, four of the arms of the maze were closed and the rats collected the food pellets from the remaining four opened arms. During this period working memory errors were recorded. Following a two hour delay, the rats were returned to the maze. All of the arms of the maze were now open but only four of them, those which were closed during the pre-delay session, were baited. The rats had to collect the food pellets without entering any previously visited arms, whether during the predelay or the post-delay session. After reaching a baseline level the rats were tested for their performance during the third week of the experiment in which either 10mM phosphate buffer (pH 7.3), or AF150 (0.07 or 1.0 mg./kg. free base dissolved in 10 mM phosphate buffer, pH 7.3), were administered i.p., immediately after the pre-delay session, once a day for five days. The results were listed according to the week (II or III), and are shown in Table 16.

**Table 16:**

| Working memory errors (session I) | | | | | | |
|---|---|---|---|---|---|---|
| | phosphate buffer | | AF150 (mg./kg., i.p.) | | | |
| | | | 0.07 | | 1.0 | |
| | (pre)II | III | (pre)II | III | (pre)II | III |
| Sal. | 0.27±0.11 | 0.27±0.09 | 0.33±0.12 | 0.18±0.07 | 0.11±0.05 | 0.13±0.07 |
| AF64A | 3.51±0.74 | 9.02±2.59 | 3.33±1.18 | 3.75±1.04 | 3.00±0.94 | 4.22±1.76 |
| Sal. = saline | | | | | | |

The working memory errors increased during the pre-delay session in the group of AF64A + buffer treated rats; it seems that as the learning process progresses, such rats became more confused, perhaps because a previous experience does not facilitate, but rather interferes with, the following one. AF150 at the tested doses (0.07 or 1.0 mg./kg., i.p.) was significantly beneficial in consolidating the working memory (in the pre-delay session only) of the AF64A treated rats; in fact, the lower dose of 0.07 mg./kg., i.p., is slightly better than the higher tested dose. The beneficial effect of AF150 in this experiment is the prevention of deterioration in AF64A treated rats, rather than simply improvement. AF150 had no improving effect on the working memory errors during the post-delay session, although those errors also increased during that period. The reasons for this effect could be: (a) the pre-delay session is a more simple task than the post-delay session, therefore demanding less cognitive ability and being easier to improve; (b) AF150 was administered after the pre-delay session, therefore improving the consolidation of the memory traces of the four first entries. It is possible that this post-administration of the drug had no effect on the post-delay session, namely on the learning of the other four entries, which can be a different cognitive process.

These results, taken together with the results of AF150 in the PA test, would seem to indicate that this compound is a very potent cognitive activator, surprisingly more potent than AF102B. Therefore, AF150 can be especially promising for the control of SDAT.

### Test 12: The effects of AF151 in AF64A-treated rats in the Morris water maze (MWM) paradigm.

This animal model, which mimics the cholinergic hypofunction and cognitive impairments in SDAT was used as described in Fisher et al (J. Pharmacol. Exptl. Therap, 1991, in the press).

Rats were injected with AF64A (3 nmole/2 µl./side, icv) or saline (2 µl., icv), in presence of phosphate buffer or AF151. The results, in terms of the path length (cm.) travelled by the rats are shown in Table 17.

AF151 at all three dosage rates showed a significant dose dependent beneficial effect in restoring cognitive dysfunction in the A64A treated rats. Thus, AF151 can be a promising drug in the treatment of SDAT, since it is effective at very low doses without exhibiting overt side effects.

The statistical significance of the data recorded in Table 17 is as follows:
AF151 (1.0 mg./kg., i.p.) vs. AF64A + phosphate buffer: p < 0.01
AF151 (0.3 mg./kg., i.p.) vs. AF64A + phosphate buffer: p < 0.05
AF151 (0.07mg./kg., i.p.) vs. AF64A + phosphate buffer: p < 0.07.

**Table 17:**

| Path length (cm.) travelled by rats in MWM | | | | | |
|---|---|---|---|---|---|
| | | Block | | | |
| icv | Treatment | 1 | 2 | 3 | 4 |
| AF64A | phosphate buffer* | 831.5±100.8 | 1040.2±188.7 | 340.6± 41.6 | 582.6±84.7 |
| | AF151◆ | | | | |
| | 0.07 | 729.0± 98.2 | 918.1±124.3 | 743.1±151.1 | 574.9±101.1 |
| | 0.3 | 862.3±151.0 | 843.5±197.5 | 539.9±146.2 | 297.9± 60.4 |
| | 1.0 | 656.6±107.6 | 802.7±111.1 | 795.0±131.3 | 434.4± 33.0 |
| Sal. | phosphate buffer* | 622.2± 71.1 | 558.7± 69.6 | 314.3± 41.7 | 282.1±41.4 |
| | AF151◆ | | | | |
| | 0.07 | 490.6± 74.0 | 562.9± 92.7 | 429.2± 76.5 | 278.0± 44.0 |
| | 0.3 | 517.8± 45.3 | 365.1± 58.4 | 424.6± 92.3 | 346.7± 64.4 |
| | 1.0 | 607.4± 90.6 | 440.2± 90.1 | 408.1± 97.1 | 233.8± 30.6 |
| icv | Treatment | **Reversal** | | | |
| AF64A | phosphate buffer* | 606.6± 78.9 | | | |
| | AF151◆ | | | | |
| | 0.07 | 602.9± 97.9 | | | |
| | 0.3 | 390.5± 77.0 | | | |
| | 1.0 | 606.0± 60.0 | | | |
| Sal. | phosphate buffer* | 433.2± 93.3 | | | |
| | AF151◆ | | | | |
| | 0.07 | 453.9± 76.0 | | | |
| | 0.3 | 306.8± 43.3 | | | |
| | 1.0 | 450.0± 93.5 | | | |

| | | | | | |
|---|---|---|---|---|---|
| Sal. = saline *0.2 ml./kg., i.p. | | | | | |
| ◆mg./kg., i.p. Notes to Table 17: In brief (c.f. Fisher et al, J. Pharmacol. Exptl. Therap, 1991, in the press), training was continued for five consecutive days, with each rat receiving four trials on each day; results are expressed as blocks (e.g. of four trials/day). During trials 1-16 (days 1-4, training stage) the platform was located in the center of the north-west quadrant of the pool. During trial no. 17, on the fifth day, the platform was removed from the pool entirely (a transfer test). In this trial the rat was placed in the water for a limited period (60 secs.) and its spacial bias was measured. During trials 18-21 on the fifth day the platform was moved to the center of the southeast quadrant (a reversal test). Four starting locations were used: north, south, east or west around the pool's perimeter. The sequence of the locations was semirandomly changed each day. AF151 and phosphate buffer were administered once a day for five days, 30 minutes before testing. The experiment was performed using a tracking system consisting of an image analyzer (cis-2) coupled to a microcomputer (8 MZHz - IBM AT, Galai Laboratories, Ltd.) | | | | | |

## Claims (Claims for the following Contracting State(s): AT, BE, DK, FR, DE, IT, LU, NL, SE, CH, GB, LI)

1. Compounds having the structural formulae (I) and (II) 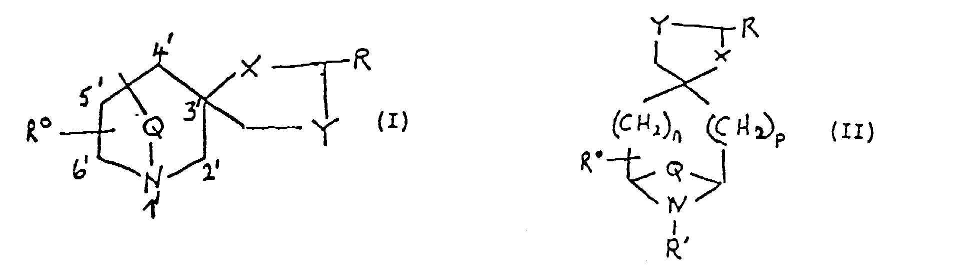 including enantiomers, racemates and acid addition and quaternary salts thereof, wherein in formula(I) Q is -CH₂CH₂- attached to the 1',4' or 1',5' positions of the six-membered ring, and in formula (II) Q is two hydrogen atoms, (CH₂)ₘ or C(CH₃)₂ where m is 1, 2 or 3 and n and p are each independently 0, 1, 2 or 3, provided that n + p = 1-3; R^{o} is hydrogen, methyl or hydroxyl;
the moiety 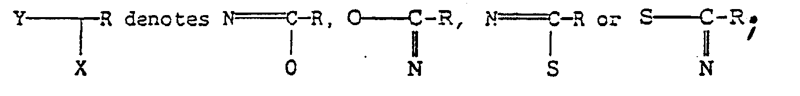 R is selected from hydrogen,
C₁₋₆-alkyl, C₂₋₆-alkenyl, C₂₋₆-alkynyl, C₃₋₇- cycloalkyl, C₁₋₆-alkyl substituted by 1-6 halogen atoms, hydroxy- C₁₋₆-alkyl, C₁₋₆-alkoxy, C₁₋₆-alkylthio, C₁₋₆-alkoxy-C₁₋₆-alkyl, carboxy-C₁₋₆-alkyl, (C₁₋₆-alkoxy)carbonyl-C₁₋₆-alkyl, amino-C₁₋₆-alkyl, mono-(C₁₋₆-alkyl)amino-C₁₋₆-alkyl, di-(C₁₋₆-alkyl)amino-C₁₋₆-alkyl, 2-oxo-pyrrolidin-1-yl-methyl, aryl and C₁₋₆-alkyl substituted by one or two aryl groups, with R being additionally selected from NH₂, NHC₁₋₆ alkyl and N(C₁₋₆alkyl)₂ when R forms part of formula (I); R' is independently selected from the group from which R is selected when R forms part of formula (I), C₁₋₆-alkanoyl and arylcarbonyl; and aryl denotes unsubstituted phenyl or phenyl substituted by 1-3 substituents selected from halogen, C₁₋₆-alkyl, C₁₋₆-alkoxy and CF₃, subject to the provisos (i), (ii) and (iii), namely: (i) in formula II, when Q is two hydrogen atoms, n =p = 1, R^{o} is hydrogen, and R is phenyl, then R' is not tertiary butyl, (ii) in formula II, when Q is two hydrogen atoms, n = p = 1, R⁰ is hydrogen, R is p-chlorophenyl, and R' is tert. butyl, then
the moiety 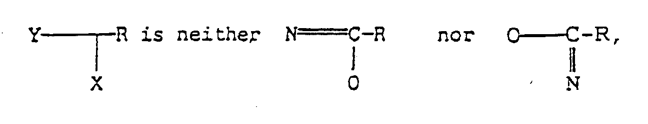 (iii) in formula II, when Q is two hydrogen atoms, n = p = 1, R^{o} is hydrogen, R is 3,5-dichlorophenyl, and R' is tertiary butyl, then the moiety

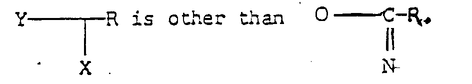

2. Compounds according to claim 1, wherein each of the symbols in formulae (I) and (II) has the meaning defined in claim 1 with the following exceptions, namely, that
the moiety 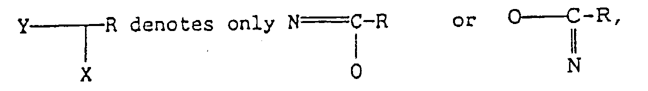 and R may not be C₁₋₆-alkoxy or C₁₋₆-alkylthio.

3. A compound according to claim 2, which is selected from the group consisting of:
2-aminospiro(1,3-oxazoline-5-3')quinuclidine,
2-methylspiro(1,3-oxazoline-5,3')quinuclidine,
2-ethylspiro(1,3-oxazoline-5,3')quinuclidine,
2-phenylspiro(1,3-oxazoline-5,3')quinuclidine,
1-methylpiperidine-4-spiro-4'-(2'-methyl-1',3'-oxazoline),
1-methylpiperidine-4-spiro-4'-(2'-ethyl-1',3'-oxazoline),
2-methylspiro(1,3-oxazoline-5,4')-1'-methylpiperidine,
2-methylspiro(1,3-thiazoline-5,4')-1'-methylpiperidine,
2-ethylspiro(1,3-oxazoline-5,4')-1'-methylpiperidine,
including enantiomers, racemates and acid addition and quaternary salts thereof.

4. A pharmaceutical composition for use in treating diseases of the central and peripheral nervous system in mammals, which comprises an amount effective for use in treating said diseases, of at least one member of the group consisting of compounds of the formulae (I) and (II) as defined in claim 1 except that provisos (i), (ii) and (iii) do not apply, and including enantiomers, racemates and pharmaceutically compatible addition and quaternary salts thereof.

5. A pharmaceutical composition according to claim 4, wherein compounds of formulae (I) and (II) are as defined in claim 2, except that provisos (i), (ii) and (iii) do not apply.

6. A pharmaceutical composition according to claim 4, wherein compounds of formulae (I) and (II) are as defined in claim 3.

7. A pharmaceutical composition according to any of claims 4-6, which is in a form suitable for oral, rectal, parenteral or transdermal administration, or for administration by insufflation or nasal spray.

8. A pharmaceutical composition according to claim 7, which is in a form suitable for transdermal administration and which comprises as an additional component, a low molecular weight fatty acid.

9. A pharmaceutical composition according to claim 7, which is in unit dosage form.

10. A pharmaceutical composition according to claim 9, wherein said at least one member is present in an amount in the range of about 0.5 to about 100 mg., together with an inert carrier or diluent.

11. A pharmaceutical composition according to any one of claims 4 to 10, which additionally contains at least one compound selected from the group consisting of physostigmine, tetrahydroaminoacridine, choline, lecithin, piracetam, aniracetam, pramiracetam, oxiracetam, 4-aminopyridine, 3,4-diaminopyridine, somatostatin, pirenzepine, N-methylatropine, N-butylscopolamine, scopolamine, clonidine, quanfamicine, propantheline, methantheline, glycopyrrolate, tropenzilium, nortriptyline, amitriptyline, imipramine, minaprine, secoverine, AFDX-116, nicotine, alaproclate, zimelidine, deprenyl and Nerve Growth Factor; and which additionally contains an inert carrier or diluent.

## Claims (Claims for the following Contracting State(s): ES)

1. A method for the preparation of compounds having the structural formulae (I) and (II) 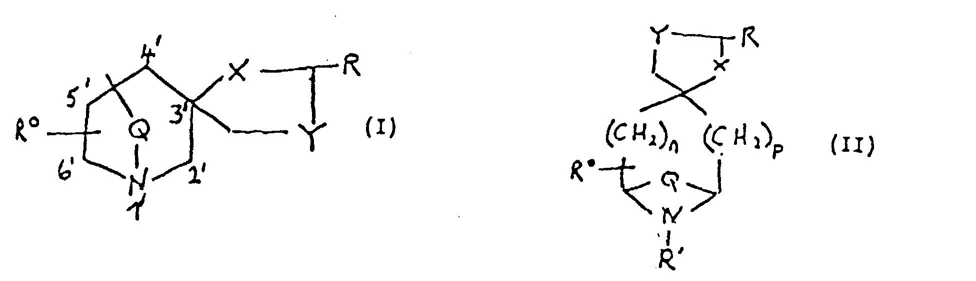 including enantiomers, racemates and acid addition and quaternary salts thereof, wherein in formula (I) Q is -CH₂CH₂- attached to the 1',4' or 1',5' positions of the six-membered ring, and in formula (II) Q is two hydrogen atoms, (CH₂)ₘ or C(CH₃)₂ where m is 1, 2 or 3 and n and p are each independently 0, 1, 2 or 3, provided that n + p = 1-3; R^{o} is hydrogen, methyl or hydroxyl;
the moiety 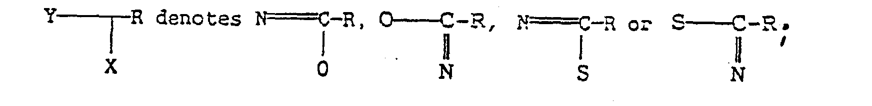 R is selected from hydrogen,
C₁₋₆-alkyl, C₂₋₆-alkenyl, C₂₋₆-alkynyl, C₃₋₇- cycloalkyl, C₁₋₆-alkyl substituted by 1-6 halogen atoms, hydroxy- C₁₋₆-alkyl, C₁₋₆-aikoxy, C₁₋₆-alkylthio, C₁₋₆-alkoxy-C₁₋₆-alkyl, carboxy-C₁₋₆-alkyl, (C₁₋₆-alkoxy)carbonyl-C₁₋₆-alkyl, amino-C₁₋₆-alkyl, mono-(C₁₋₆-alkyl)amino-C₁₋₆-alkyl, di- (C₁₋₆-alkyl)amino-C₁₋₆-alkyl, 2-oxo-pyrrolidin-1-yl-methyl, aryl and C₁₋₆-alkyl substituted by one or two aryl groups, with R being additionally selected from NH₂, NH-C₁₋₆ alkyl and N(C₁₋₆alkyl)₂ when R forms part of formula (I); R' is independently selected from the group from which R is selected when R forms part of formula (I), C₁₋₆-alkanoyl and arylcarbonyl; and aryl denotes unsubstituted phenyl or phenyl substituted by 1-3 substituents selected from halogen, C₁₋₆-alkyl, C₁₋₆-alkoxy and CF₃, subject to the provisos (i), (ii) and (iii), namely: (i) in formula II, when Q is two hydrogen atoms, n =p = 1, R^{o} is hydrogen, and R is phenyl, then R' is not tertiary butyl, (ii) in formula II, when Q is two hydrogen atoms, n = p = 1, R^{o} is hydrogen, R is p-chlorophenyl, and R' is tert. butyl, then
the moiety 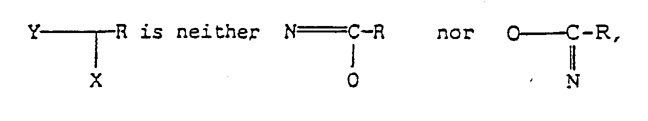 (iii) in formula II, when Q is two hydrogen atoms, n = p = 1, R^{o} is hydrogen, R is 3,5-dichlorophenyl, and R' is tertiary butyl, then the moiety 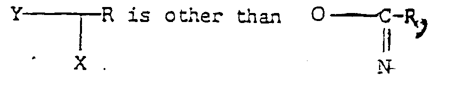 wherein, to produce a compound having the structural formula (I), a compound having the general formula 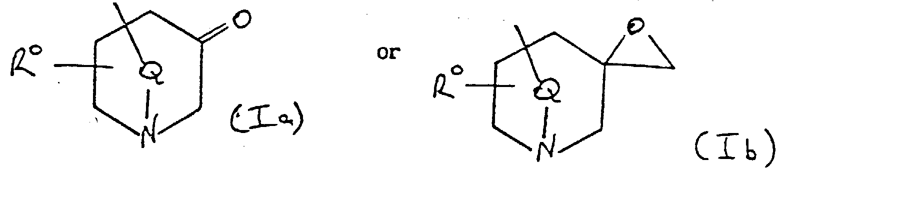 wherein R^{o} and Q are as above defined is subjected to oxazoline or thiazoline ring forming reaction at the carbonyl group or epoxide group of general formula (Ia) or (Ib) respectively to produce a compound of general formula (I) wherein X, R and Y are as above defined; and wherein, to produce a compound having the structural formula (II), a compound having the general formula 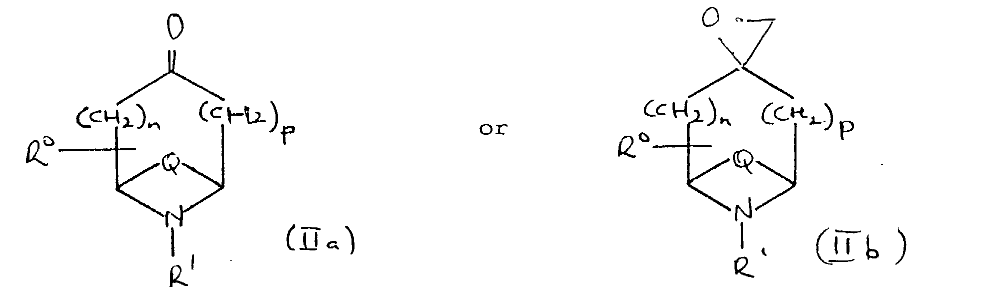 wherein R^{o}, R¹, Q, n and p are as above defined is subjected to oxazoline or thiazoline ring forming reaction at the carbonyl group or epoxide group of general formula (IIa) or (IIb) respectively to produce a compound of formula (II) wherein X, R and Y are as above defined.

2. A method as outlined in claim 1, wherein the compound of general formula (Ia) or (Ib) or of general formula (IIa) or (IIb) is initially converted to a 3-aminomethyl compound or 3-hydroxymethyl compound of general formula 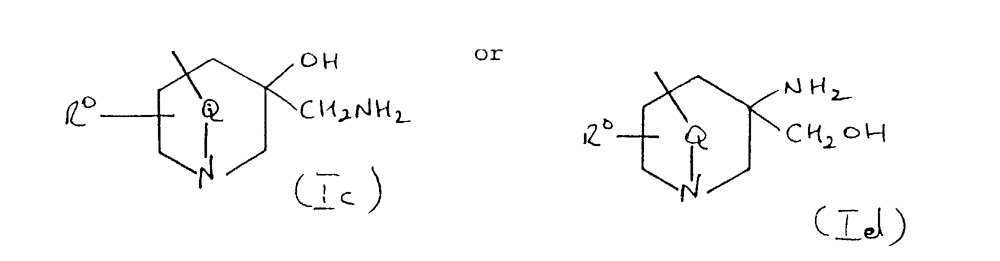 or 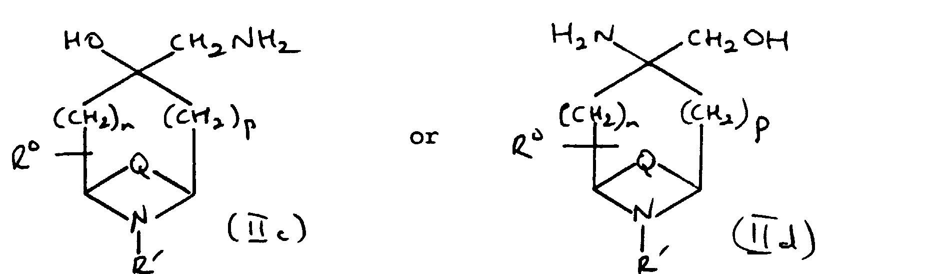 respectively before formation of an oxazoline compound is effected.

3. A method as claimed in claim 2 wherein conversion from a compound of general formula (Ib) or (IIb) is effected by reaction with alkali metal azide followed by contacting with wet active Raney Nickel.

4. A method as claimed in claims 2 or 3 wherein, when the moiety 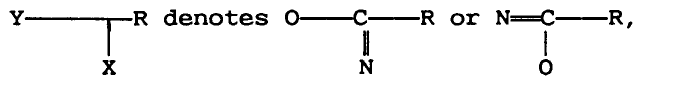 the 3-aminomethyl compound or 3-hydroxymethyl compound respectively is condensed with a carboxylic acid RCOOH or an amidate of formula RC(:NH)-O-alkyl wherein R has the meaning set out in claim 1 or with cyanogen bromide when R = NH₂ and wherein when the moiety 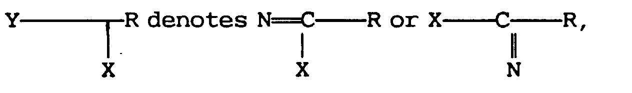 the 3-aminomethyl compound or 3-hydroxymethyl compound respectively is condensed with an acid anhydride (RCO)₂O and the resulting reaction product is reacted with P₂X₅, wherein X is O or S.

5. A method according to any preceding claim, wherein each of the symbols in formulae (I) and (II) have the meanings defined in claim 1 with the following exceptions, namely, that the moiety 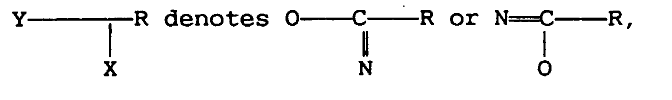 and R may not be C₁₋₆-alkoxy or C₁₋₆-alkylthio.

6. A method according to claim 5, which is carried out to produce a compound selected from the group consisting of:
2-aminospiro (1,3-oxazoline-5,3')quinuclidine,
2-methylspiro(1,3-oxazoline-5,3')quinuclidine,
2-ethylspiro(1,3-oxazoline-5,3')quinuclidine,
2-phenylspiro(1,3-oxazoline-5,3')quinuclidine,
1-methylpiperidine-4-spiro-4'-(2'-methyl-1',3'-oxazoline),
1-methylpiperidine-4-spiro-4'-(2'-ethyl-1',3'-oxazoline),
2-methylspiro(1,3-oxazoline-5,4')-1'-methylpiperidine,
2-methylspiro(1,3-thiazoline-5,4')-1'-methylpiperidine,
2-methylspiro(1,3-oxazoline-5,4')-1'-methylpiperidine
including enantiomers, racemates and acid addition and quaternary salts thereof.

7. A method for the production of a pharmaceutical composition for use in treating diseases of the central and peripheral nervous system in mammals, which comprises mixing at least one member of the group consisting of compounds of the formulae (I) and (II) as defined in claim 1 except that provisos (i), (ii) and (iii) do not apply, and including enantiomers, racemates and pharmaceutically compatible addition and quaternary salts thereof with a pharmaceutically acceptable carrier therefor.

8. A method as claimed in claim 7, wherein compounds of formulae (I) and (II) are as defined in claim 5 except that provisos (i), (ii) and (iii) do not apply.

9. A method according to claim 7, wherein compounds of formulae (I) and (II) are as defined in claim 6.

10. A method according to any of claims 7 to 9, wherein the pharmaceutical composition is made up in a form suitable for oral, rectal, parenteral or transdermal administration, or for administration by insufflation or nasal spray.

11. A method according to claim 7, wherein the pharmaceutical composition is made up in a form suitable for transdermal administration and which comprises as an additional component, a low molecular weight fatty acid.

12. A method according to claim 10, wherein the pharmaceutical composition is made up in unit dosage form.

13. A method according to claim 12, wherein, in each dosage unit of said pharmaceutical composition, said at least one member is present in an amount in the range of about 0.5 to about 100 mg, together with an inert carrier or diluent.

14. A method according to any one of claims 7 to 13, wherein there is additionally included in the pharmaceutical composition at least one compound selected from the group consisting of physostigmine, tetrahydroaminoacridine, choline, lecithin, piracetam, aniracetam, pramiracetam, oxiracetam, 4-aminopyridine, 3,4-diaminopyridine, somatostatin, pirenzepine, N-methylatropine, N-butylscopolamine, scopolamine, clonidine, quanfamicine, proantheline, metantheline, glycopyrrolate, tropenzilium, nortriptyline, amitriptyline, imipramine, minaprine, secoverine, AFDX-116, nicotine, alaproclate, zimelidine, deprenyl and Nerve Growth Factor.

## Claims (Claims for the following Contracting State(s): GR)

1. Compounds having the structural formulae (I) and (II) 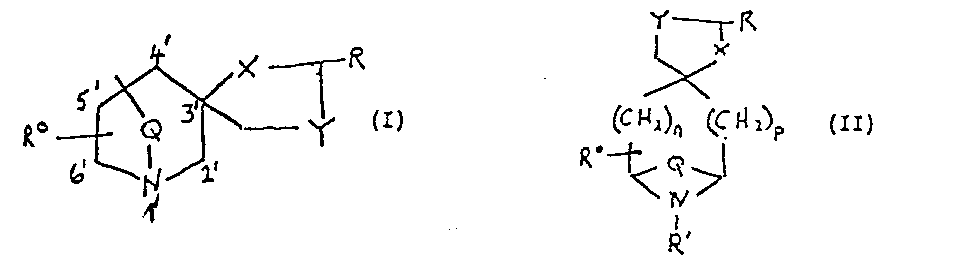 including enantiomers, racemates and acid addition and quaternary salts thereof, wherein in formula (I) Q is -CH₂CH₂- attached to the 1',4' or 1',5' positions of the six-membered ring, and in formula (II) Q is two hydrogen atoms, (CH₂)ₘ or C(CH₃)₂ where m is 1, 2 or 3 and n and p are each independently 0, 1, 2 or 3, provided that n + p = 1-3; R^{o} is hydrogen, methyl or hydroxyl;
the moiety 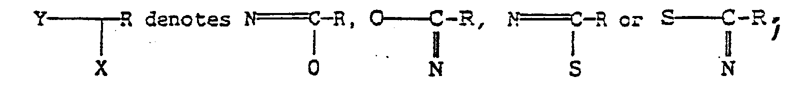 R is selected from hydrogen,
C₁₋₆-alkyl, C₂₋₆-alkenyl, C₂₋₆-alkynyl, C₃₋₇- cycloalkyl, C₁₋₆-alkyl substituted by 1-6 halogen atoms, hydroxy- C₁₋₆-alkyl, C₁₋₆-alkoxy, C₁₋₆-alkylthio, C₁₋₆-alkoxy-C₁₋₆-alkyl, C₁₋₆-alkoxy-C₁₋₆-alkyl, (C₁₋₆-alkoxy)carbonyl-C₁₋₆-alkyl, amino-C₁₋₆-alkyl, mono-(C₁₋₆-alkyl)amino-C₁₋₆-alkyl, di-(C₁₋₆-alkyl)amino-C₁₋₆-alkyl, 2-oxo-pyrrolidin-1-yl-methyl, aryl and C₁₋₆-alkyl substituted by one or two aryl groups, with R being additionally selected from NH₂, NH-C₁₋₆ alkyl and N(C₁₋₆alkyl)₂ when R forms part of formula (I); R' is independently selected from the group from which R is selected when R forms part of formula (I), C₁₋₆-alkanoyl and arylcarbonyl; and aryl denotes unsubstituted phenyl or phenyl substituted by 1-3 substituents selected from halogen, C₁₋₆-alkyl, C₁₋₆-alkoxy and CF₃, subject to the provisos (i), (ii) and (iii), namely: (i) in formula II, when Q is two hydrogen atoms, n =p = 1, R^{o} is hydrogen, and R is phenyl, then R' is not tertiary butyl, (ii) in formula II, when Q is two hydrogen atoms, n = p = 1, R^{o} is hydrogen, R is p-chlorophenyl, and R' is tert. butyl, then
the moiety 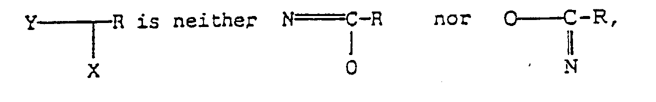 (iii) in formula II, when Q is two hydrogen atoms, n = p = 1, R^{o} is hydrogen, R is 3,5-dichlorophenyl, and R' is tertiary butyl, then the moiety

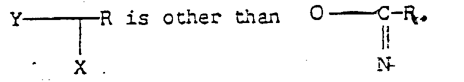

2. Compounds according to claim 1, wherein each of the symbols in formulae (I) and (II) has the meaning defined in claim 1 with the following exceptions, namely, that
the moiety 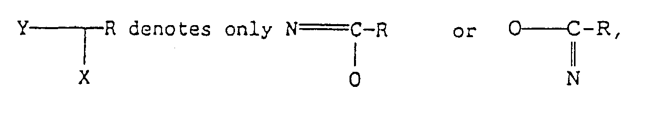 and R may not be C₁₋₆-alkoxy or C₁₋₆-alkylthio.

3. A compound according to claim 2, which is selected from the group consisting of:
2-aminospiro (1,3-oxazoline-5,3')quinuclidine,
2-methylspiro(1,3-oxazoline-5,3')quinuclidine,
2-ethylspiro(1,3-oxazoline-5,3')quinuclidine,
2-phenylspiro(1,3-oxazoline-5,3')quinuclidine,
1-methylpiperidine-4-spiro-4'-(2'-methyl-1',3'-oxazoline),
1-methylpiperidine-4-spiro-4'-(2'-ethyl-1',3'-oxazoline),
2-methylspiro(1,3-oxazoline-5,4')-1'-methylpiperidine,
2-methylspiro(1,3-thiazoline-5,4')-1'-methylpiperidine,
2-methylspiro(1,3-oxazoline-5,4')-1'-methylpiperidine
including enantiomers, racemates and acid addition and quaternary salts thereof.

4. A method for the production of a pharmaceutical composition for use in treating diseases of the central and peripheral nervous system in mammals, which comprises mixing at least one member of the group consisting of compounds of the formulae (I) and (II) as defined in claim 1 except that provisos (i), (ii) and (iii) do not apply, and including enantiomers, racemates and pharmaceutically compatible addition and quaternary salts thereof with a pharmaceutically acceptable carrier therefor.

5. A method as claimed in claim 4, wherein compounds of formulae (I) and (II) are as defined in claim 2 except that provisos (i), (ii) and (iii) do not apply.

6. A method according to claim 4, wherein compounds of formulae (I) and (II) are as defined in claim 3.

7. A method according to any of claims 4 to 6, wherein the pharmaceutical composition is made up in a form suitable for oral, rectal, parenteral or transdermal administration, or for administration by insufflation or nasal spray.

8. A method according to claim 4, wherein the pharmaceutical composition is made up in a form suitable for transdermal administration and which comprises as an additional component, a low molecular weight fatty acid.

9. A method according to claim 7, wherein the pharmaceutical composition is made up in unit dosage form.

10. A method according to claim 9, wherein, in said pharmaceutical composition, said at least one member is present in an amount in the range of about 0.5 to about 100 mg, together with an inert carrier or diluent.

11. A method according to any one of claims 4 to 10, wherein there is additionally included in the pharmaceutical composition at least one compound selected from the group consisting of physostigmine, tetrahydroaminoacridine, choline, lecithin, piracetam, aniracetam, pramiracetam, oxiracetam, 4-aminopyridine, 3,4-diaminopyridine, somatostatin, pirenzepine, N-methylatropine, N-butylscopolamine, scopolamine, clonidine, quanfamicine, proantheline, metantheline, glycopyrrolate, tropenzilium, nortriptyline, amitriptyline, imipramine, minaprine, secoverine, AFDX-116, nicotine, alaproclate, zimelidine, deprenyl and Nerve Growth Factor.

## Patentansprüche (Patentansprüche für folgende(n) Vertragsstaat(en): AT, BE, DK, FR, DE, IT, LU, NL, SE, CH, LI, GB)

1. Verbindungen der Strukturformeln (I) und (II) 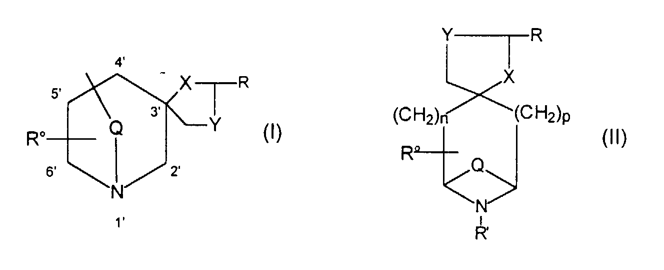 einschließlich deren Enantiomere, Racemate, Säureadditionen und quaternären Salze,
worin ist:
Q in Formel (I) gleich -CH₂CH₂-, verknüpft mit dem Sechsring in 1',4'- oder 1',5'-Stellung, und
in Formel (II) gleich zwei Wasserstoffatome, (CH₂)ₘ oder C(CH₃)_{2,} wobei m gleich 1, 2 oder 3 ist und n und p jeweils unabhängig voneinander 0, 1, 2 oder 3 sind, vorausgesetzt n + p = 1 - 3;
R⁰ Wasserstoff, Methyl oder Hydroxyl;
der Rest 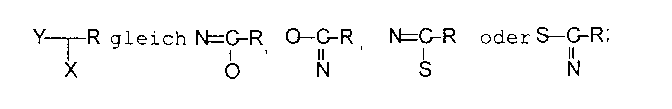
R ausgewählt aus Wasserstoff, C₁₋₆-Alkyl, C₂₋₆-Alkenyl, C₂₋₆-Alkynyl, C₃₋₇-cycloalkyl, C₁₋₆-Alkyl, substituiert mit 1-6 Halogenatomen, Hydroxy-C₁₋₆-Alkyl, C₁₋₆-Alkoxy, C₁₋₆-Alkylthio, C₁₋₆-Alkoxy-C₁₋₆-Alkyl, Carboxy-C₁₋₆-alkyl, (C₁₋₆-Alkoxy)carbonyl-C₁₋₆-alkyl, Amino-C₁₋₆alkyl, Mono-(C₁₋₆-alkyl)amino-C₁₋₆-alkyl, Di-(C₁₋₆-alkyl)amino-C₁₋₆-alkyl, 2-Oxopyrrolidin-1-yl-methyl, Aryl und C₁₋₆-Alkyl, substituiert von ein oder zwei Arylgruppen, wobei R zusätzlich ausgewählt ist aus NH₂, NH-C₁₋₆-Alkyl und N(C₁₋₆-alkyl)₂, wenn R Teil der Formel (I) ist;
R' unabhängig ausgewählt aus der Gruppe, aus der R ausgewählt ist, wenn R ein Teil der Formel (I) ist, C₁₋₆-Alkanoyl und Arylcarbonyl;
wobei Aryl steht für ein unsubstituiertes Phenyl oder ein Phenyl, substituiert mit ein bis drei Substituenten, ausgewählt aus Halogen, C₁₋₆-Alkyl, C₁₋₆-Alkoxy und CF₃;
mit den Maßgaben (i), (ii) und (iii), nämlich:
(i) in Formel II, ist Q zwei Wasserstoffatome, n = p = 1, R⁰ Wasserstoff und R Phenyl, dann ist R' kein *tert*-Butyl;
(ii) in Formel II, ist Q zwei Wasserstoffatome, n = p = 1, R⁰ Wasserstoff, R p-Chlorphenyl und R' *tert*-Butyl, dann ist der
Rest 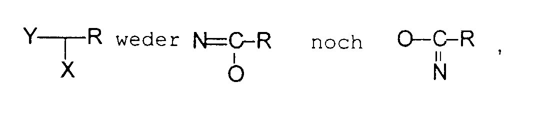
(iii) in Formel II, ist Q zwei Wasserstoffatome, n = p = 1, R⁰ Wasserstoff, R 3,5-Dichlorphenyl, und R' *tert*-Butyl, dann ist der
Rest

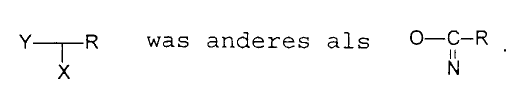

2. Verbindungen nach Anspruch 1, worin jeweils die Symbole in den Formeln (I) und (II) die in Anspruch 1 angegebenen Bedeutungen besitzen, mit folgenden Ausnahmen, nämlich dass der
Rest 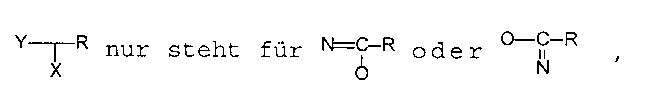 und R nicht C₁₋₆-Alkoxy oder C₁₋₆-Alkylthio ist.

3. Verbindung nach Anspruch 2, ausgewählt aus der Gruppe:
2-Aminospiro(1,3-oxazolin-5,3')chinuclidin,
2-Methylspiro(1,3-oxazolin-5,3')chinuclidin,
2-Ethylspiro(1,3-oxazolin-5,3')chinuclidin,
2-Phenylspiro(1,3-oxazolin-5,3')chinuclidin,
1-Methylpiperidin-4-spiro-4'-(2'-methyl-1',3'-oxazolin),
1-Methylpiperidin-4-spiro-4'-(2'-ethyl-1',3'-oxazolin),
2-Methylspiro(1,3-oxazolin-5,4')-1'-methylpiperidin,
2-Methylspiro(1,3-thiazolin-5,4')-1'-methylpiperidin,
2-Ethylspiro(1,3-oxazolin-5,4')-1'-methylpiperidin,
einschließlich deren Enantiomere, Racemate, Säureadditionen und quaternären Salze.

4. Pharmazeutische Zusammensetzung zur Verwendung in der Behandlung von Krankheiten des zentralen und peripheren Nervensystems in Säugetieren, umfassend eine Menge, die wirksam ist zur Verwendung in der Behandlung dieser Krankheiten, von mindestens einem Vertreter der Gruppe, bestehend aus den Verbindungen der Formeln (I) und (II), wie in Anspruch 1 definiert, ausgenommen, dass die Maßgaben (i), (ii) und (iii) nicht greifen, einschließlich deren Enantiomere, Racemate, pharmazeutisch kompatiblen Additionen und quaternären Salze.

5. Pharmazeutische Zusammensetzung nach Anspruch 4, wobei die Verbindungen der Formeln (I) und (II) wie in Anspruch 2 definiert sind, mit der Ausnahme, dass die Maßgaben (i), (ii) und (iii) nicht greifen.

6. Pharmazeutische Zusammensetzung nach Anspruch 4, wobei die Verbindungen der Formeln (I) und (II) wie in Anspruch 3 definiert sind.

7. Pharmazeutische Zusammensetzung nach einem der Ansprüche 4 bis 6, welche in einer Form vorliegt, geeignet zur oralen, rektalen, parenteralen oder transdermalen Verabreichung oder zur Verabreichung durch Insufflation oder als Nasenspray.

8. Pharmazeutische Zusammensetzung nach Anspruch 7, welche in einer Form vorliegt, geeignet zur transdermalen Verabreichung und umfassend als weitere Komponente eine niedermolekulare Fettsäure.

9. Pharmazeutische Zusammensetzung nach Anspruch 7, welche in Form von Doseneinheiten vorliegt.

10. Pharmazeutische Zusammensetzung nach Anspruch 9, wobei mindestens ein Vertreter zugegen ist in einer Menge im Bereich von 0,5 bis etwa 100 mg zusammen mit einem inerten Träger oder Verdünnungsmittel.

11. Pharmazeutische Zusammensetzung nach einem der Ansprüche 4 bis 10, die weiterhin enthält mindestens eine Verbindung aus der Gruppe Physostigmin, Tetrahydroaminoacridin, Cholin, Lecithin, Piracetam, Aniracetam, Pramiracetam, Oxiracetam, 4-Aminopyridin, 3,4-Diaminopyridin, Somatostatin, Pirenzepin, N-Methylatropin, N-Butylscopolamin, Scopolamin, Clonidin, Quanfamicin, Propanthelin, Metanthelin, Glycopyrrolat, Tropenzilium, Nortriptylin, Amitriptylin, Imipramin, Minaprin, Secoverin, AFDX-116, Nikotin, Alaproclat, Zimelidin, Deprenyl und Nervenwachstumsfaktor und desweiteren einen inerten Träger oder ein inertes Verdünnungsmittel.

## Patentansprüche (Patentansprüche für folgende(n) Vertragsstaat(en): ES)

1. Verfahren zur Herstellung von Verbindungen der Strukturformeln (I) und (II) 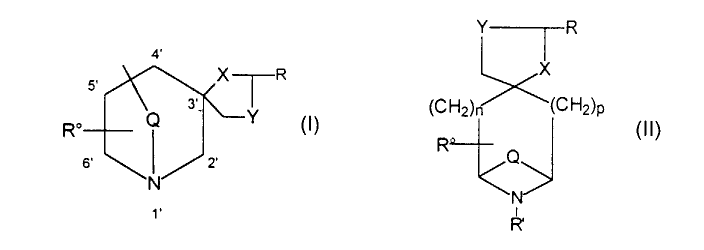 einschließlich deren Enantiomere, Racemate, Säureadditionen und quaternären Salze,
worin ist:
Q in Formel (I) gleich -CH₂CH₂-, verknüpft mit dem Sechsring in 1',4'- oder 1',5'-Stellung, und in Formel (II) gleich zwei Wasserstoffatome, (CH₂)ₘ oder C(CH₃)_{2,} wobei m gleich 1, 2 oder 3 ist und n und p jeweils unabhängig voneinander 0, 1, 2 oder 3 sind, vorausgesetzt n + p = 1 - 3;
R⁰ Wasserstoff, Methyl oder Hydroxyl;
der Rest 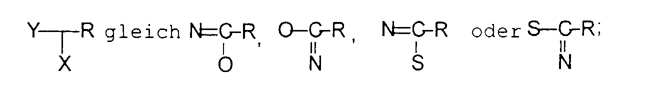
R ausgewählt aus Wasserstoff, C₁₋₆-Alkyl, C₂₋₆-Alkenyl, C₂₋₆-Alkynyl, C₃₋₇-Cycloalkyl, C₁₋₆-Alkyl, substituiert mit 1-6 Halogenatomen, Hydroxy-C₁₋₆-Alkyl, C₁₋₆-Alkoxy, C₁₋₆-Alkylthio, C₁₋₆-Alkoxy-C₁₋₆-Alkyl, Carboxy-C₁₋₆-alkyl, (C₁₋₆-Alkoxy)carbonyl-C₁₋₆-alkyl, Amino-C₁₋₆-alkyl, Mono-(C₁₋₆-alkyl)amino-C₁₋₆-alkyl, Di-(C₁₋₆-alkyl)amino-C₁₋₆-alkyl, 2-Oxopyrrolidin-1-yl-methyl, Aryl und C₁₋₆-Alkyl, substituiert von ein oder zwei Arylgruppen, wobei R zusätzlich ausgewählt ist aus NH₂, NH-C₁₋₆-Alkyl und N(C₁₋₆-alkyl)₂, wenn R Teil der Formel (I) ist;
R' unabhängig ausgewählt aus der Gruppe, aus der R ausgewählt ist, wenn R ein Teil der Formel (I) ist, C₁₋₆-Alkanoyl und Arylcarbonyl;
wobei Aryl steht für ein unsubstituiertes Phenyl oder ein Phenyl, substituiert mit ein bis drei Substituenten, ausgewählt aus Halogen, C₁₋₆-Alkyl, C₁₋₆-Alkoxy und CF₃;
mit den Maßgaben (i), (ii) und (iii), nämlich:
(i) in Formel II, ist Q zwei Wasserstoffatome, n = p = 1, R⁰ Wasserstoff und R Phenyl, dann ist R' kein *tert*-Butyl;
(ii) in Formel II, ist Q zwei Wasserstoffatome, n = p = 1, R⁰ Wasserstoff, R p-Chlorphenyl und R' *tert*-Butyl, dann ist der
Rest 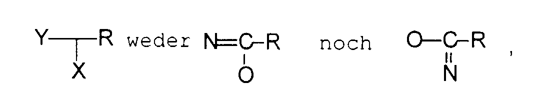
(iii) in Formel II, ist Q zwei Wasserstoffatome, n = p = 1, R⁰ Wasserstoff, R 3,5-Dichlorphenyl, und R' *tert*-Butyl, dann ist der
Rest 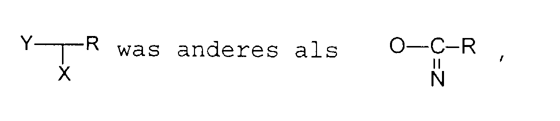 wobei zur Herstellung einer Verbindung der Strukturformel (I) eine Verbindung der allgemeinen Formel 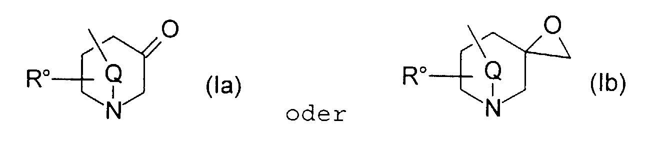 worin R^{o} und Q wie oben definiert sind, unterworfen wird einer Oxazolin- oder Thiazolin-Ringbildungsreaktion an der Carbonyl- oder Epoxy-Gruppe der allgemeinen Formel (Ia) bzw. (Ib), so dass man eine Verbindung der allgemeinen Formel (I) erhält, worin X, R und Y wie oben definiert ist; und wobei, zur Herstellung einer Verbindung der Strukturformel (II), eine Verbindung der allgemeinen Formel 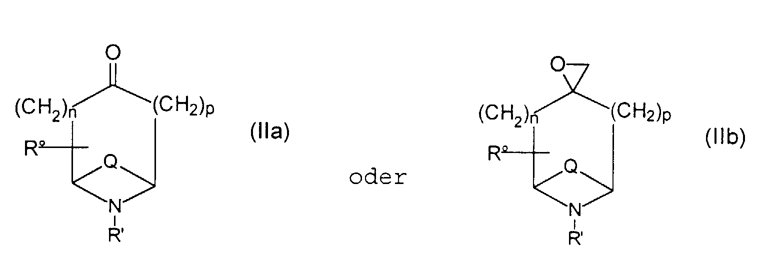 worin R^{o}, R¹, Q n und p wie oben definiert sind, unterworfen wird einer Oxazolin- oder Thiazolin-Ringbildungsreaktion an der Carbonyl- oder Epoxy-Gruppe der allgemeinen Formel (IIa) bzw. (IIb), so dass man eine Verbindung der Formel (II) erhält, worin X, R und Y wie oben definiert ist.

2. Verfahren nach Anspruch 1, wobei die Verbindung der allgemeinen Formel (Ia) bzw. (Ib) oder der allgemeinen Formel (IIa) bzw. (IIb) zunächst umgewandelt wird in eine 3-Aminomethyl- oder eine 3-Hydroxymethyl-Verbindung der allgemeinen Formel 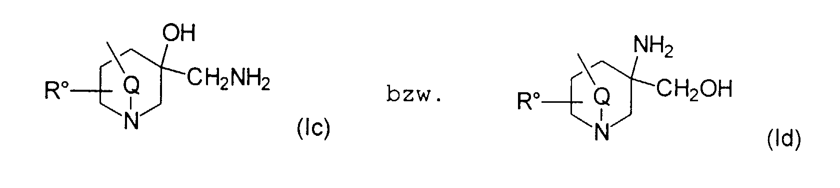 oder 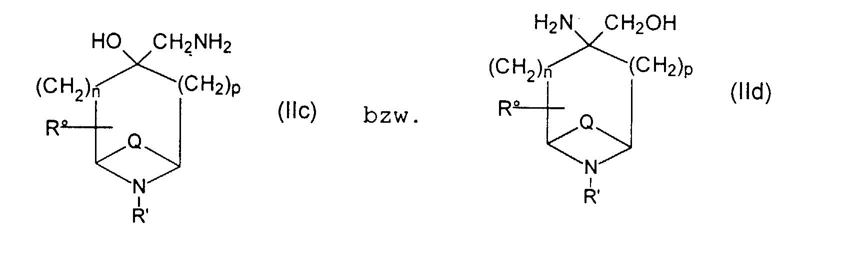 bevor die Bildung der Oxazolin-Verbindung erfolgt.

3. Verfahren nach Anspruch 2, wobei die Umwandlung von einer Verbindung der allgemeinen Formel (Ib) oder (IIb) erfolgt durch Umsetzung mit Alkalimetallazid, gefolgt von einem Zusammenbringen mit feuchtem aktiven Raney-Nickel.

4. Verfahren nach Anspruch 2 oder 3, wobei, wenn der Rest 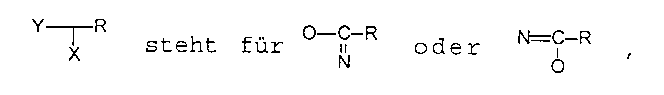 die 3-Aminomethyl- bzw. 3-Hydroxymethyl-Verbindung kondensiert wird mit einer Carbonsäure RCOOH oder einem Amidat der Formel RC(:NH)-O-alkyl, worin R die in Anspruch 1 angegebene Bedeutung hat, oder mit Cyanbromid, wenn R gleich NH₂ ist, und wobei, wenn
der Rest 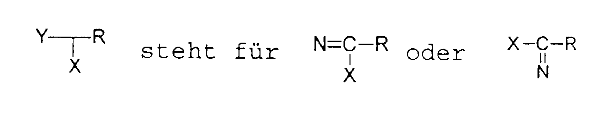 die 3-Aminomethyl- bzw. 3-Hydroxymethyl-Verbindung kondensiert wird mit einem Säureanhydrid (RCO)₂O und das resultierende Umsetzungsprodukt umgesetzt wird mit P₂X₅, worin X gleich O oder S ist.

5. Verfahren nach einem vorhergehenden Anspruch, wobei die Symbole in den Formeln (I) und (II) die in Anspruch 1 angegebenen Bedeutungen besitzen, mit folgenden Ausnahmen, nämlich, dass der
Rest 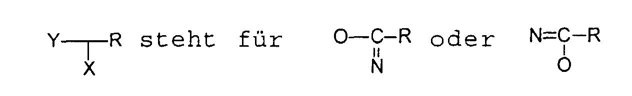 und R nicht C₁₋₆-Alkoxy oder C₁₋₆-Alkylthio ist.

6. Verfahren nach Anspruch 5, das so erfolgt, dass eine Verbindung erhalten wird aus der Gruppe:
2-Aminospiro(1,3-oxazolin-5,3')chinuclidin,
2-Methylspiro(1,3-oxazolin-5,3')chinuclidin,
2-Ethylspiro(1,3-oxazolin-5,3')chinuclidin,
2-Phenylspiro(1,3-oxazolin-5,3')chinuclidin,
1-Methylpiperidin-4-spiro-4'-(2'-methyl-1',3'-oxazolin),
1-Methylpiperidin-4-spiro-4'-(2'-ethyl-1',3'-oxazolin),
2-Methylspiro(1,3-oxazolin-5,4')-1'-methylpiperidin,
2-Methylspiro(1,3-thiazolin-5,4')-1'-methylpiperidin,
2-Ethylspiro(1,3-oxazolin-5,4')-1'-methylpiperidin,
einschließlich deren Enantiomere, Racemate, Säureadditionen und quaternären Salze.

7. Verfahren zur Herstellung einer pharmazeutischen-Zusammensetzung zur Verwendung in der Behandlung von Krankheiten des zentralen und peripheren Nervensystems in Säugetieren, umfassend das Mischen mindestens eines Vertreters der Gruppe, bestehend aus den Verbindungen der Formeln (I) und (II), wie in Anspruch 1 definiert, ausgenommen, dass die Maßgaben (i), (ii) und (iii) nicht greifen, einschließlich deren Enantiomere, Racemate, pharmazeutisch kompatiblen Additionen und quaternären Salze, mit einem pharmazeutisch akzeptablen Träger dafür.

8. Verfahren nach Anspruch 7, wobei die Verbindungen der Formeln (I) und (II) wie in Anspruch 5 definiert sind, mit der Ausnahme, dass die Maßgaben (i), (ii) und (iii) nicht greifen.

9. Verfahren nach Anspruch 7, wobei die Verbindungen der Formeln (I) und (II) wie in Anspruch 6 definiert sind.

10. Verfahren nach einem der Ansprüche 7 bis 9, wobei die pharmazeutische Zusammensetzung zubereitet wird in einer Form, geeignet zur oralen, rektalen, parenteralen oder transdermalen Verabreichung oder zur Verabreichung durch Insufflation oder als Nasenspray.

11. Verfahren nach Anspruch 7, wobei die pharmazeutische Zusammensetzung zubereitet wird in einer Form geeignet zur transdermalen Verabreichung, und die als weitere Komponente eine niedermolekulare Fettsäure umfasst.

12. Verfahren nach Anspruch 10, wobei die pharmazeutische Zusammensetzung in Form von Doseneinheiten zubereitet wird.

13. Verfahren nach Anspruch 12, wobei in jeder Doseneinheit der pharmazeutische Zusammensetzung mindestens ein Vertreter zugegen ist in einer Menge im Bereich von 0,5 bis etwa 100 mg zusammen mit einem inerten Träger oder Verdünnungsmittel.

14. Verfahren nach einem der Ansprüche 7 bis 13, wobei die pharmazeutische Zusammensetzung desweiteren mindestens eine Verbindung enthält aus der Gruppe Physostigmin, Tetrahydroaminoacridin, Cholin, Lecithin, Piracetam, Aniracetam, Pramiracetam, Oxiracetam, 4-Aminopyridin, 3,4-Diaminopyridin, Somatostatin, Pirenzepin, N-Methylatropin, N-Butylscopolamin, Scopolamin, Clonidin, Quanfamicin, Propanthelin, Metanthelin, Glycopyrrolat, Tropenzilium, Nortriptylin, Amitriptylin, Imipramin, Minaprin, Secoverin, AFDX-116, Nikotin, Alaproclat, Zimelidin, Deprenyl und Nervenwachstumsfaktor.

## Patentansprüche (Patentansprüche für folgende(n) Vertragsstaat(en): GR)

1. Verbindungen der Strukturformeln (I) und (II) 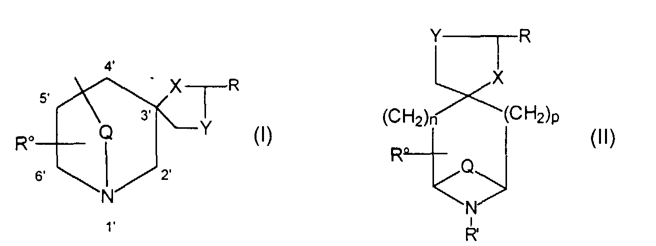 einschließlich deren Enantiomere, Racemate, Säureadditionen und quaternären Salze,
worin ist:
Q in Formel (I) gleich -CH₂CH₂-, verknüpft mit dem Sechsring in 1',4'- oder 1',5'-Stellung, und in Formel (II) gleich zwei Wasserstoffatome, (CH₂)ₘ oder C(CH₃)_{2,} wobei m gleich 1, 2 oder 3 ist und n und p jeweils unabhängig voneinander 0, 1, 2 oder 3 sind, vorausgesetzt n + p = 1 - 3;
R⁰ Wasserstoff, Methyl oder Hydroxyl;
der Rest 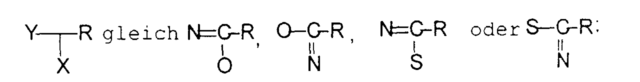
R ausgewählt aus Wasserstoff, C₁₋₆-Alkyl, C₂₋₆-Alkenyl, C₂₋₆-Alkynyl, C₃₋₇-Cycloalkyl, C₁₋₆-Alkyl, substituiert mit 1-6 Halogenatomen, Hydroxy-C₁₋₆-Alkyl, C₁₋₆-Alkoxy, C₁₋₆-Alkylthio, C₁₋₆-Alkoxy-C₁₋₆-Alkyl, Carboxy-C₁₋₆-alkyl, (C₁₋₆-Alkoxy)carbonyl-C₁₋₆-alkyl, Amino-C₁₋₆-alkyl, Mono-(C₁₋₆-alkyl)amino-C₁₋₆-alkyl, Di-(C₁₋₆-alkyl)amino-C₁₋₆-alkyl, 2-Oxopyrrolidin-1-yl-methyl, Aryl und C₁₋₆-Alkyl, substituiert von ein oder zwei Arylgruppen, wobei R zusätzlich ausgewählt ist aus NH₂, NH-C₁₋₆-Alkyl und N(C₁₋₆-alkyl)₂, wenn R Teil der Formel (I) ist;
R' unabhängig ausgewählt aus der Gruppe, aus der R ausgewählt ist, wenn R ein Teil der Formel (I) ist, C₁₋₆-Alkanoyl und Arylcarbonyl;
wobei Aryl steht für ein unsubstituiertes Phenyl oder ein Phenyl, substituiert mit ein bis drei Substituenten, ausgewählt aus Halogen, C₁₋₆-Alkyl, C₁₋₆-Alkoxy und CF₃;
mit den Maßgaben (i), (ii) und (iii), nämlich:
(i) in Formel II, ist Q zwei Wasserstoffatome, n = p = 1, R⁰ Wasserstoff und R Phenyl, dann ist R' kein *tert*-Butyl;
(ii) in Formel II, ist Q zwei Wasserstoffatome, n = p = 1, R⁰ Wasserstoff, R p-Chlorphenyl und R' *tert*-Butyl, dann ist der
Rest 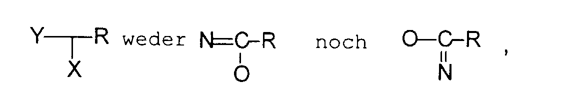
(iii) in Formel II, ist Q zwei Wasserstoffatome, n = p = 1, R⁰ Wasserstoff, R 3,5-Dichlorphenyl, und R' *tert*-Butyl, dann ist der
Rest

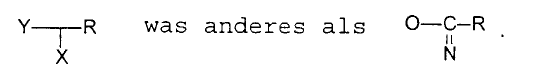

2. Verbindungen nach Anspruch 1, worin jeweils die Symbole in den Formeln (I) und (II) die in Anspruch 1 angegebenen Bedeutungen besitzen, mit folgenden Ausnahmen, nämlich dass der
Rest 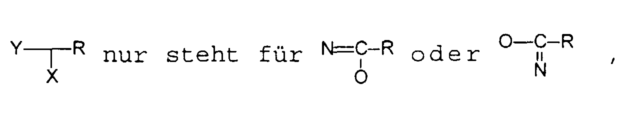 und R nicht C₁₋₆-Alkoxy oder C₁₋₆-Alkylthio ist.

3. Verbindung nach Anspruch 2, ausgewählt aus der Gruppe:
2-Aminospiro(1,3-oxazolin-5,3')chinuclidin,
2-Methylspiro(1,3-oxazolin-5,3')chinuclidin,
2-Ethylspiro(1,3-oxazolin-5,3')chinuclidin,
2-Phenylspiro(1,3-oxazolin-5,3')chinuclidin,
1-Methylpiperidin-4-spiro-4'-(2'-methyl-1',3'-oxazolin),
1-Methylpiperidin-4-spiro-4'-(2'-ethyl-1',3'-oxazolin),
2-Methylspiro(1,3-oxazolin-5,4')-1'-methylpiperidin,
2-Methylspiro(1,3-thiazolin-5,4')-1'-methylpiperidin,
2-Ethylspiro(1,3-oxazolin-5,4')-1'-methylpiperidin,
einschließlich deren Enantiomere, Racemate, Säureadditionen und quaternären Salze.

4. Verfahren zur Herstellung einer pharmazeutische Zusammensetzung zur Verwendung in der Behandlung von Krankheiten des zentralen und peripheren Nervensystems in Säugetieren, umfassend das Mischen mindestens eines Vertreters der Gruppe, bestehend aus den Verbindungen der Formeln (I) und (II), wie in Anspruch 1 definiert, ausgenommen, dass die Maßgaben (i), (ii) und (iii) nicht greifen, einschließlich deren Enantiomere, Racemate, pharmazeutisch kompatiblen Additionen und quaternären Salze, mit einem pharmazeutisch akzeptablen Träger dafür.

5. Verfahren nach Anspruch 4, wobei die Verbindungen der Formeln (I) und (II) wie in Anspruch 2 definiert sind, mit der Ausnahme, dass die Maßgaben (i), (ii) und (iii) nicht greifen.

6. Verfahren nach Anspruch 4, wobei die Verbindungen der Formeln (I) und (II) wie in Anspruch 3 definiert sind.

7. Verfahren nach einem der Ansprüche 4 bis 6, wobei die pharmazeutische Zusammensetzung in einer Form zubereitet wird, geeignet zur oralen, rektalen, parenteralen oder transdermalen Verabreichung oder zur Verabreichung durch Insufflation oder als Nasenspray.

8. Verfahren nach Anspruch 4, wobei die pharmazeutische Zusammensetzung in einer Form zubereitet wird, geeignet zur transdermalen Verabreichung, und umfassend als weitere Komponente eine niedermolekulare Fettsäure.

9. Verfahren nach Anspruch 7, wobei die pharmazeutische Zusammensetzung in Form von Doseneinheiten zubereitet wird.

10. Verfahren nach Anspruch 9, wobei in der pharmazeutischen Zusammensetzung mindestens ein Vertreter zugegen ist in einer Menge im Bereich von 0,5 bis etwa 100 mg zusammen mit einem inerten Träger oder Verdünnungsmittel.

11. Verfahren nach einem der Ansprüche 4 bis 10, wobei der pharmazeutischen Zusammensetzung weiterhin zugefügt wird mindestens eine Verbindung aus der Gruppe Physostigmin, Tetrahydroaminoacridin, Cholin, Lecithin, Piracetam, Aniracetam, Pramiracetam, Oxiracetam, 4-Aminopyridin, 3,4-Diaminopyridin, Somatostatin, Pirenzepin, N-Methylatropin, N-Butylscopolamin, Scopolamin, Clonidin, Quanfamicin, Propanthelin, Metanthelin, Glycopyrrolat, Tropenzilium, Nortriptylin, Amitriptylin, Imipramin, Minaprin, Secoverin, AFDX-116, Nikotin, Alaproclat, Zimelidin, Deprenyl und Nervenwachstumsfaktor.

## Revendications (Revendications pour l'(les) Etat(s) contractant(s) suivant(s): AT, BE, CH, DE, DK, FR, GB, IT, LU, NL, SE)

1. Composés correspondant à l'une des formules structurales (I) et (II) : 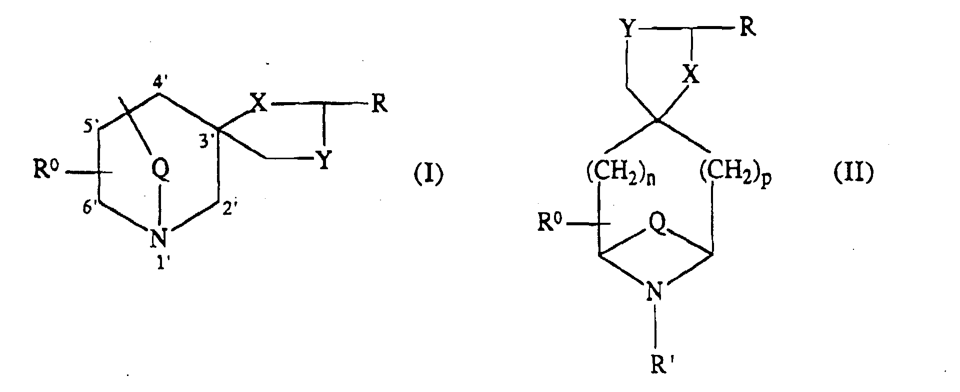 y compris leurs énantiomères, leurs racémiques, leurs sels d'addition d'acide et leurs sels quaternaires,
formules dans lesquelles :
dans la formule (I), Q représente un groupe -CH₂-CH₂- lié aux atomes situés en positions 1' et 4' ou en positions 1' et 5' du cycle à six chaînons, et dans la formule (II), Q représente une paire d'atomes d'hydrogène, un groupe -C(CH₃)₂- ou -(CH₂)ₘ- où m vaut 1, 2 ou 3, et n et p représentent chacun, indépendamment, le nombre 0, 1, 2 ou 3, pourvu que la somme n+p vaille de 1 à 3 ;
R⁰ représente un atome d'hydrogène, un groupe méthyle ou un groupe hydroxyle ;
le fragment 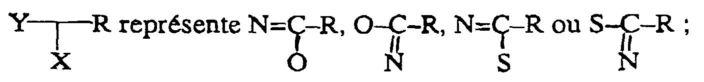
R est choisi dans l'ensemble que constiutent un atome d'hydrogène et les groupes alkyle en C₁₋₆, alcényle en C₂₋₆, alcynyle en C₂₋₆, cycloalkyle en C₃₋₇, alkyle en C₁₋₆ portant de 1 à 6 substituants halogéno, hydroxyalkyle en C₁₋₆, alcoxy en C₁₋₆, alkylthio en C₁₋₆, (alcoxy en C₁₋₆)-alkyle en C₁₋₆, carboxy-alkyle en C₁₋₆, (alcoxy en C₁₋₆)-carbonylalkyle en C₁₋₆, aminoalkyle en C₁₋₆, mono(alkyle en C₁₋₆)-aminoalkyle en C₁₋₆, di(alkyle en C₁₋₆)-aminoalkyle en C₁₋₆, 2-oxo-pyrrolidine-1-yl-méthyle, aryle et alkyle en C₁₋₆ portant un ou deux substituants aryle, R pouvant en outre être choisi, dans le cas de la formule (I), parmi les groupes amine, (alkyle en C₁₋₆)-amino et di(alkyle en C₁₋₆)-amino ;
R' est indépendamment choisi dans l'ensemble dans lequel R est choisi dans le cas de la formule (I), augmenté des groupes alcanoyle en C₁₋₆ et arylcarbonyle ;
le terme "aryle" désignant un groupe phényle, sans substituant ou portant de 1 à 3 substituants choisis parmi les atomes d'halogène et les groupes alkyle en C₁₋₆, alcoxy en C₁₋₆ et trifluorométhyle ;
sous réserve que
a) dans la formule (II), R' ne représente pas un groupe tertiobutyle si, simultanément, Q représente une paire d'atomes d'hydrogène, n et p valent 1, R⁰ représente un atome d'hydrogène et R représente un groupe phényle;
b) dans la formule II, le fragment 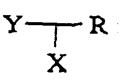 ne représente ni 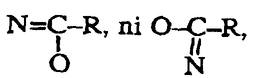 si, simultanément, Q représente une paire d'atomes d'hydrogène, n et p valent 1, R⁰ représente un atome d'hydrogène, R représente un groupe p-chlorophényle et R' représente un groupe tertiobutyle ;
c) dans la formule II, le fragment 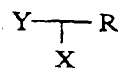 représente autre chose que ni 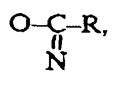 si, simultanément, Q représente une paire d'atomes d'hydrogène, n et p valent 1, R⁰ représente un atome d'hydrogène,
R représente un groupe 3,5-dichlorophényle et R' représente un groupe tertiobutyle.

2. Composés conformes à la revendication 1, dans lesquels chacun des symboles présents dans les formules (I) et (II) a les significations indiquées dans la revendication 1, sauf que le fragment 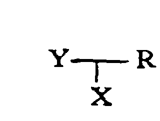 ne peut représenter que 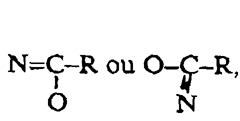 et que R ne peut pas représenter un groupe alcoxy en C₁₋₆ ou alkylthio en C₁₋₆.

3. Composé conforme à la revendication 2, qui est choisi dans l'ensemble que constituent les composés suivants :
2-amino-spiro[1,3-oxazoline-5,3'-quinuclidine],
2-méthyl-spiro[1,3-oxazoline-5,3'-quinuclidine],
2-éthyl-spiro[1,3-oxazoline-5,3'-quinuclidine],
2-phényl-spiro[1,3-oxazoline-5,3'-quinuclidine],
(1-méthyl-pipéridine)-4-spiro-4'-(2'-méthyl-1',3'-oxazoline),
(1-méthyl-pipéridine)-4-spiro-4'-(2'-éthyl-1',3'-oxazoline),
(2-méthyl-1,3-oxazoline)-5-spiro-4'-(1'-méthyl-pipéridine),
(2-méthyl-1,3-thiazoline)-5-spiro-4'-(1'-méthyl-pipéridine),
(2-éthyl-1,3-oxazoline)-5-spiro-4'-(1'-méthyl-pipéridine),
y compris leurs énantiomères, leurs racémiques, leurs sels d'addition d'acide et leurs sels quaternaires.

4. Composition pharmaceutique destinée à être employée pour traiter des maladies du système nerveux central ou périphérique, qui contient, en une quantité suffisante pour traiter efficacement ces maladies, au moins un élément de l'ensemble des composés de formules (I) et (II), définis dans la revendication 1 (sauf que les réserves (a), (b) et (c) ne s'appliquent plus), y compris leurs énantiomères, leurs racémiques, et leurs sels d'addition d'acide et leurs sels quaternaires admissibles en pharmacie.

5. Composition pharmaceutique conforme à la revendication 4, qui contient des composés de formules (I) et (II) qui sont conformes à la revendication 2 (sauf que les réserves (a), (b) et (c) ne s'appliquent plus).

6. Composition pharmaceutique conforme à la revendication 4, qui contient des composés de formules (I) et (II) qui sont conformes à la revendication 3.

7. Composition pharmaceutique conforme à l'une des revendications 4 à 6, qui se présente sous une forme appropriée pour pouvoir être administrée par voie orale, rectale, parentérale ou percutanée, ou par insufflation ou en nébulisations nasales.

8. Composition pharmaceutique conforme à la revendication 7, qui se présente sous une forme appropriée pour pouvoir être administrée par voie percutanée, et qui contient, en tant que composant supplémentaire, un acide gras de faible masse moléculaire.

9. Composition pharmaceutique conforme à la revendication 7, qui se présente sous une forme administrée par doses unitaires.

10. Composition pharmaceutique conforme à la revendication 9, dans laquelle ledit élément, au nombre d'au moins un, se trouve en une quantité d'environ 0,5 à environ 100 mg et en compagnie d'un diluant ou d'un véhicule inerte.

11. Composition pharmaceutique conforme à l'une des revendications 4 à 10, qui contient en outre au moins un composé choisi dans l'ensemble que constituent les physostigmine, tétrahydroaminoacridine, choline, lécithine, piracétam, aniracétam, pramiracétam, oxiracétam, 4-aminopyridine, 3,4-diaminopyridine, somatostatine, pirenzépine, N-méthyl-atropine, N-butyl-scopolamine, scopolamine, clonidine, quanfamicine, proanthéline, métanthéline, glycopyrrolate, tropenzilium, nortriptyline, amitriptyline, imiprarnine, minaprine, sécovérine, AFDX-116, nicotine, alaproclate, zimélidine, déprényl et facteur de croissance des nerfs.

## Revendications (Revendications pour l'(les) Etat(s) contractant(s) suivant(s): ES)

1. Procédé de préparation de composés correspondant à l'une des formules structurales (I) et (II) : 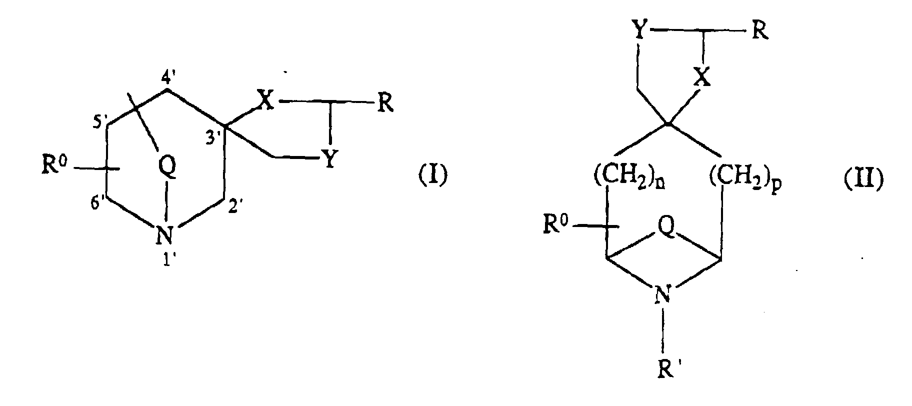 y compris leurs énantiomères, leurs racémiques, leurs sels d'addition d'acide et leurs sels quaternaires,
formules dans lesquelles :
dans la formule (I), Q représente un groupe -CH₂-CH₂- lié aux atomes situés en positions 1' et 4' ou en positions 1' et 5' du cycle à six chaînons, et dans la formule (II), Q représente une paire d'atomes d'hydrogène, un groupe -C(CH₃)₂- ou (CH₂)ₘ- où m vaut 1, 2 ou 3, et n et p représentent chacun, indépendamment, le nombre 0, 1, 2 ou 3, pourvu que la somme n+p vaille de 1 à 3 ;
R⁰ représente un atome d'hydrogène, un groupe méthyle ou un groupe hydroxyle ;
le fragment 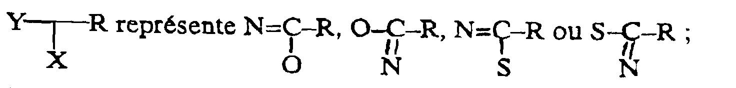
R est choisi dans l'ensemble que constituent un atome d'hydrogène et les groupes alkyle en C₁₋₆, alcényle en C₂₋₆, alcynyle en C₂₋₆, cycloalkyle en C₃₋₇, alkyle en C₁₋₆ portant de 1 à 6 substituants halogéno, hydroxyalkyle en C₁₋₆, alcoxy en C₁₋₆, alkylthio en C₁₋₆, (alcoxy en C₁₋₆)-alkyle en C₁₋₆, carboxy-alkyle en C₁₋₆, (alcoxy en C₁₋₆)-carbonyl-alkyle en C₁₋₆, aminoakyle en C₁₋₆, mono(alkyle en C₁₋₆)-aminoalkyle en C₁₋₆, di(alkyle en C₁₋₆)-aminoalkyle en C₁₋₆, 2-oxo-pyrrolidine-1-yl-méthyle, aryle et alkyle en C₁₋₆ portant un ou deux substituants aryle, R pouvant en outre être choisi, dans le cas de la formule (I), parmi les groupes amino, (alkyle en C₁₋₆-amino et di(alkyle en C₁₋₆)-amino ;
R' est indépendamment choisi dans l'ensemble dans lequel R est choisi dans le cas de la formule (I), augmenté des groupes alcanoyle en C₁₋₆ et arylcarbonyle ;
le terme "aryle" désignant un groupe phényle, sans substituant ou portant de 1 à 3 substituants choisis parmi les atomes d'halogène et les groupes alkyle en C₁₋₆, alcoxy en C₁₋₆ et trifluorométhyle ;
sous réserve que
a) dans la formule (II), R' ne représente pas un groupe tertiobutyle si, simultanément, Q représente une paire d'atomes d'hydrogène, n et p valent 1, R⁰ représente un atome d'hydrogène et R représente un groupe phényle ;
b) dans la formule II, le fragment 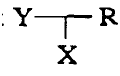 ne représente
ni 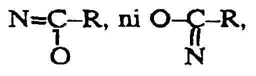 si, simultanément, Q représente une paire d'atomes d'hydrogène, n et p valent 1, R⁰ représente un atome d'hydrogène, R représente un groupe p-chlorophényle et R' représente un groupe tertiobutyle;
c) dans la formule II, le fragment 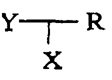 représente autre chose que 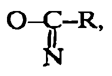 si, simultanément, Q représente une paire d'atomes d'hydrogène, n et p valent 1, R⁰ représente un atome d'hydrogène,
R représente un groupe 3,5-dichlorophényle et R' représente un groupe tertiobutyle ;
procédé dans lequel :
pour préparer un composé de formule (I), on soumet un composé de formule générale 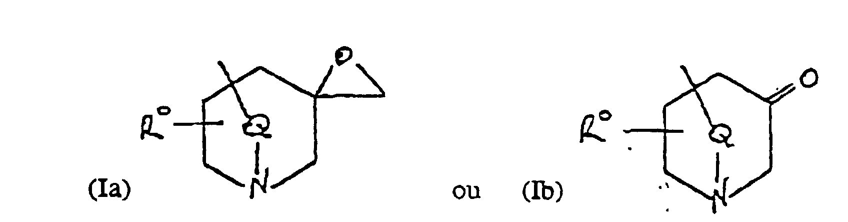 où R⁰ et Q ont les significations indiquées plus haut,
à une réaction au cours de laquelle il se forme un cycle oxazoline ou thiazoline, au niveau du groupe carbonyle de la formule générale (Ia) ou au niveau du groupe époxyde de la formule générale (Ib), ce qui donne un composé de formule générale (I) où X, R et Y ont les significations indiquées plus haut ;
et pour préparer un composé de formule (II), on soumet un composé de formule générale 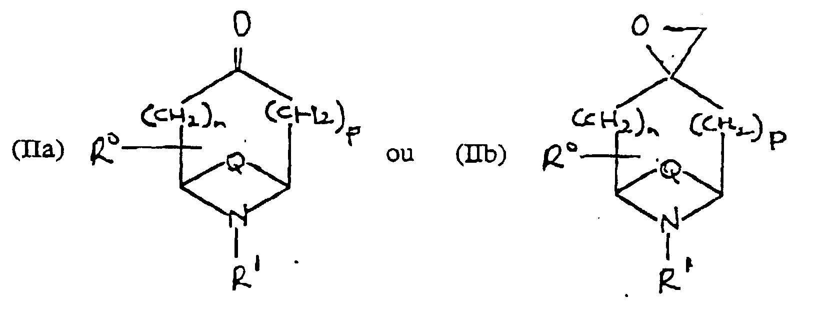 où R⁰, R¹, Q, n et p ont les significations indiquées plus haut,
à une réaction au cours de laquelle il se forme un cycle oxazoline ou thiazoline, au niveau du groupe carbonyle de la formule générale (IIa) ou au niveau du groupe époxyde de la formule générale (IIb), ce qui donne un composé de formule générale (II) où X, R et Y ont les significations indiquées plus haut.

2. Procédé conforme à la revendication 1, dans lequel on commence par convertir le composé de formule générale (Ia) ou (Ib), ou le composé de formule générale (IIa) ou (IIb), en un composé 3-aminométhylé ou 3-hydroxyméthylé, de formule générale 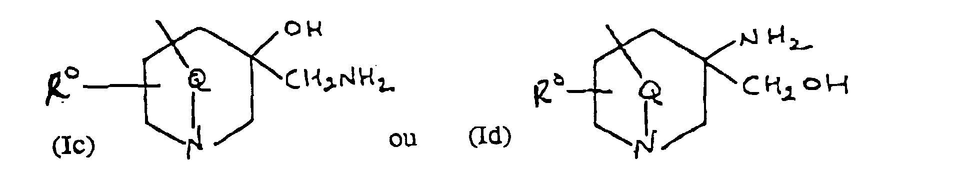 ou 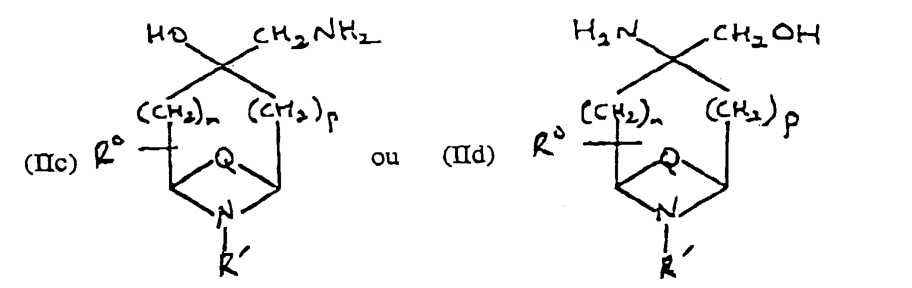 respectivement, avant de réaliser la préparation d'un composé à cycle oxazoline.

3. Procédé conforme à la revendication 2, dans lequel on réalise ladite conversion, à partir d'un composé de formule générale (Ib) ou (IIb), en faisant réagir un tel composé avec un azoture de métal alcalin, et en mettant ensuite le produit ainsi obtenu en contact avec du nickel de Raney actif et mouillé.

4. Procédé conforme à la revendication 2 ou 3, dans lequel :
quand le fragment 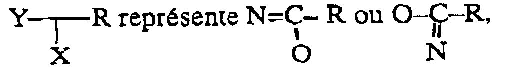 on condense respectivement le composé 3-aminométhylé ou le composé 3-hydroxyméthylé avec un acide carboxylique de formule R-COOH ou avec un amidate de formule R-C(=NH)-O-alkyle, où R a la signification indiquée dans la revendication 1, ou encore avec du bromure de cyanogène si R représente un groupe amino,
et quand le fragment 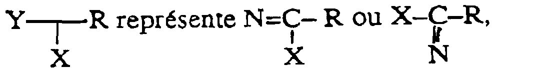 on condense respectivement le composé 3-aminométhylé ou le composé 3-hydroxyméthylé avec un anhydride d'acide de formule (RCO)₂O et l'on fait réagir le produit de réaction ainsi obtenu avec du composé de formule P₂X₅ où X représente O ou S.

5. Procédé conforme à l'une des revendications précédentes, dans lequel chacun des symboles présents dans les formules (I) et (II) a les significations indiquées dans la revendication 1, sauf que le fragment 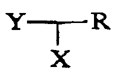 ne peut représenter que 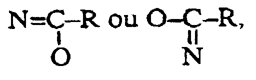 et que R ne peut pas représenter un groupe alcoxy en C₁₋₆ ou alkylthio en C₁₋₆.

6. Procédé conforme à la revendication 5, que l'on met en oeuvre pour préparer un composé choisi dans l'ensemble que constituent les suivants :
2-amino-spiro[1,3-oxazoline-5,3'-quinuclidine],
2-méthyl-spiro[1,3-oxazoline-5,3'-quinuclidine],
2-éthyl-spiro[1,3-oxazoline-5,3'-quinuclidine],
2-phényl-spiro[1,3-oxazoline-5,3'-quinuclidine],
(1-méthyl-pipéridine)-4-spiro-4'-(2'-méthyl-1',3'-oxazoline),
(1-méthyl-pipéridine)-4-spiro-4'-(2'-éthyl-1',3'-oxazoline),
(2-méthyl-1,3-oxazoline)-5-spiro-4'-(1'-méthyl-pipéridine),
(2-méthyl-1,3-thiazoline)-5-spiro-4'-(1'-méthyl-pipéridine),
(2-éthyl-1,3-oxazoline)-5-spiro-4'-(1'-méthyl-pipéridine),
y compris leurs énantiomères, leurs racémiques, leurs sels d'addition d'acide et leurs sels quaternaires.

7. Procédé de préparation d'une composition pharmaceutique destinée à être employée pour traiter des maladies du système nerveux central ou périphérique, lequel procédé comporte le fait de mélanger, avec un véhicule admissible en pharmacie, au moins un élément de l'ensemble des composés de formules (I) et (II), définis dans la revendication 1 (sauf que les réserves (a), (b) et (c) ne s'appliquent plus), y compris leurs énantiomères, leurs racémiques, et leurs sels d'addition d'acide et leurs sels quaternaires admissibles en pharmacie.

8. Procédé conforme à la revendication 7, dans lequel on emploie des composés de formules (I) et (II) qui sont définis dans la revendication 5, (sauf que les réserves (a), (b) et (c) ne s'appliquent plus).

9. Procédé conforme à la revendication 7, dans lequel on emploie des composés de formules (I) et (II) qui sont définis dans la revendication 6.

10. Procédé conforme à l'une des revendications 7 à 9, dans lequel on met la composition pharmaceutique sous une forme appropriée pour pouvoir l'administrer par voie orale, rectale, parentérale ou percutanée, ou par insufflation ou en nébulisations nasales.

11. Procédé conforme à la revendication 7, dans lequel on met la composition pharmaceutique sous une forme appropriée pour pouvoir l'administrer par voie percutanée, et l'on y ajoute, en tant que composant supplémentaire, un acide gras de faible masse moléculaire.

12. Procédé conforme à la revendication 10, dans lequel on met la composition pharmaceutique sous une forme administrée par doses unitaires.

13. Procédé conforme à la revendication 12, dans lequel ledit élément, au nombre d'au moins un, se trouve, dans chaque dose unitaire de ladite composition pharmaceutique, en une quantité d'environ 0,5 à environ 100 mg et en compagnie d'un diluant ou d'un véhicule inerte.

14. Procédé conforme à l'une des revendications 7 à 13, dans lequel on met en plus, dans la composition pharmaceutique, au moins un composé choisi dans l'ensemble que constituent les physostigmine, tétrahydroaminoacridine, choline, lécithine, piracétam, aniracétam, pramiracétam, oxiracétam, 4-aminopyridine, 3 ,4-diaminopyridine, somatostatine, pirenzépine, N-méthyl-atropine, N-butyl-scopolamine, scopolamine, clonidine, quanfamicine, proanthéline, métanthéline, glycopyrrolate, tropenzilium, nortriptyline, amitriptyline, imipramine, minaprine, sécovérine, AFDX-116, nicotine, alaproclate, zimélidine, déprényl et facteur de croissance des nerfs.

## Revendications (Revendications pour l'(les) Etat(s) contractant(s) suivant(s): GR)

1. Composés correspondant à l'une des formules structurales (I) et (II) : 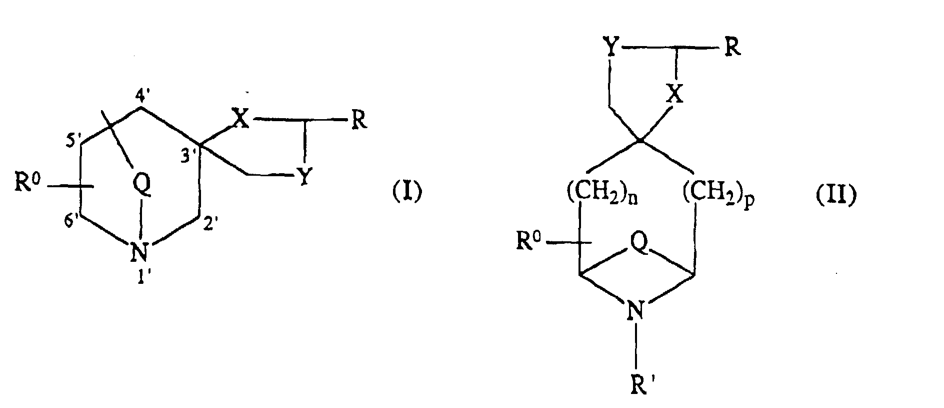 y compris leurs énantiomères, leurs racémiques, leurs sels d'addition d'acide et leurs sels quaternaires,
formules dans lesquelles :
dans la formule (I), Q représente un groupe -CH₂-CH₂- lié aux atomes situés en positions 1' et 4' ou en positions 1' et 5' du cycle à six chaînons, et dans la formule (II), Q représente une paire d'atomes d'hydrogène, un groupe -C(CH₃)₂- ou -(CH₂)ₘ- où m vaut 1, 2 ou 3, et n et p représentent chacun, indépendamment, le nombre 0, 1, 2 ou 3, pourvu que la somme n+p vaille de 1 à 3 ;
R⁰ représente un atome d'hydrogène, un groupe méthyle ou un groupe hydroxyle ;
le fragment 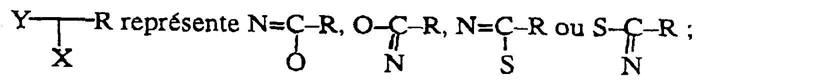
R est choisi dans l'ensemble que constituent un atome d'hydrogène et les groupes alkyle en C₁₋₆, alcényle en C₂₋₆, alcynyle en C₂₋₆, cycloalkyle en C₃₋₇, alkyle en C₁₋₆ portant de 1 à 6 substituants halogéno, hydroxyalkyle en C₁₋₆, alcoxy en C₁₋₆, alkylthio en C₁₋₆, (alcoxy en C₁₋₆)-alkyle en C₁₋₆, carboxy-alkyle en C₁₋₆, (alcoxy en C₁₋₆)-carbonylalkyle en C₁₋₆, aminoalkyle en C₁₋₆, mono(alkyle en C₁₋₆)-aminoalkyle en C₁₋₆, di(alkyle en C₁₋₆)-aminoalkyle en C₁₋₆, 2-oxo-pyrrolidine-1-yl-méthyle, aryle et alkyle en C₁₋₆ portant un ou deux substituants aryle, R pouvant en outre être choisi, dans le cas de la formule (I), parmi les groupes amino, (alkyle en C₁₋₆)-amino et di(alkyle en C₁₋₆)-amino ;
R' est indépendamment choisi dans l'ensemble dans lequel R est choisi dans le cas de la formule (I), augmenté des groupes alcanoyle en C₁₋₆ et arylcarbonyle ;
le terme "aryle" désignant un groupe phényle, sans substituant ou portant de 1 à 3 substituants choisis parmi les atomes d'halogène et les groupes alkyle en C₁₋₆, alcoxy en C₁₋₆ et trifluorométhyle ;
sous réserve que
a) dans la formule (II), R' ne représente pas un groupe tertiobutyle si, simultanément, Q représente une paire d'atomes d'hydrogène, n et p valent 1, R⁰ représente un atome d'hydrogène et R représente un groupe phényle ;
b) dans la formule II, le fragment 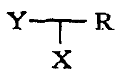 ne représente
ni 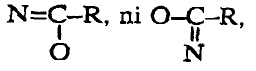 si, simultanément, Q représente une paire d'atomes d'hydrogène, n et p valent 1, R⁰ représente un atome d'hydrogène, R représente un groupe p-chlorophényle et R' représente un groupe tertiobutyle;
c) dans la formule II, le fragment 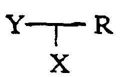 représente autre chose que 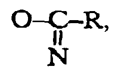 si, simultanément, Q représente une paire d'atomes d'hydrogène, n et p valent 1, R⁰ représente un atome d'hydrogène, R représente un groupe 3,5-dichlorophényle et R' représente un groupe tertiobutyle.

2. Composés conformes à la revendication 1, dans lesquels chacun des symboles présents dans les formules (I) et (II) a les significations indiquées dans la revendication 1, sauf que le fragment 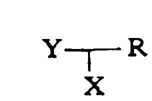 ne peut représenter que 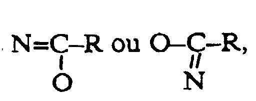 et que R ne peut pas représenter un groupe alcoxy en C₁₋₆ ou alkylthio en C₁₋₆.

3. Composé conforme à la revendication 2, qui est choisi dans l'ensemble que constituent les composés suivants :
2-amino-spiro[1,3-oxazoline-5,3'-quinuclidine],
2-méthyl-spiro[1,3-oxazoline-5,3'-quinuclidine],
2-éthyl-spiro[1,3-oxazoline-5,3'-quinuclidine],
2-phényl-spiro[1,3-oxazoline-5,3'-quinuclidine],
(1-méthyl-pipéridine)-4-spiro-4'-(2'-méthyl-1',3'-oxazoline),
(1-méthyl-pipéridine)-4-spiro-4'-(2'-éthyl-1',3'-oxazoline),
(2-méthyl-1,3-oxazohne)-5-spiro-4'-(1'-méthyl-pipéridine),
(2-méthyl-1,3-thiazoline)-5-spiro-4'-(1'-méthyl-pipéridine),
(2-éthyl-1,3-oxazoline)-5-spiro-4'-(1'-méthyl-pipéridine),
y compris leurs énantiomères, leurs racémiques, leurs sels d'addition d'acide et leurs sels quaternaires.

4. Procédé de préparation d'une composition pharmaceutique destinée à être employée pour traiter des maladies du système nerveux central ou périphérique, lequel procédé comporte le fait de mélanger, avec un véhicule admissible en pharmacie, au moins un élément de l'ensemble des composés de formules (I) et (II), définis dans la revendication 1 (sauf que les réserves (a), (b) et (c) ne s'appliquent plus), y compris leurs énantiomères, leurs racémiques, et leurs sels d'addition d'acide et leurs sels quaternaires admissibles en pharmacie.

5. Procédé conforme à la revendication 4, dans lequel on emploie des composés de formules (I) et (II) qui sont conformes à la revendication 2 (sauf que les réserves (a), (b) et (c) ne s'appliquent plus).

6. Procédé conforme à la revendication 4, dans lequel on emploie des composés de formules (I) et (II) qui sont conformes à la revendication 3.

7. Procédé conforme à l'une des revendications 4 à 6, dans lequel on met la composition pharmaceutique sous une forme appropriée pour pouvoir l'administrer par voie orale, rectale, parentérale ou percutanée, ou par insufflation ou en nébulisations nasales.

8. Procédé conforme à la revendication 4, dans lequel on met la composition pharmaceutique sous une forme appropriée pour pouvoir l'administrer par voie percutanée, et l'on y ajoute, en tant que composant supplémentaire, un acide gras de faible masse moléculaire.

9. Procédé conforme à la revendication 7, dans lequel on met la composition pharmaceutique sous une forme administrée par doses unitaires.

10. Procédé conforme à la revendication 9, dans lequel ledit élément, au nombre d'au moins un, se trouve, au sein de la composition pharmaceutique, en une quantité d'environ 0,5 à environ 100 mg et en compagnie d'un diluant ou d'un véhicule inerte.

11. Procédé conforme à l'une des revendications 4 à 10, dans lequel on met en plus, dans la composition pharmaceutique, au moins un composé choisi dans l'ensemble que constituent les physostigmine, tétrahydroaminoacridine, choline, lécithine, piracétam, aniracétam, pramiracétam, oxiracétam, 4-aminopyridine, 3,4-diaminopyridine, somatostatine, pirenzépine, N-méthyl-atropine, N-butyl-scopolamine, scopolamine, clonidine, quanfarnicine, proanthéline, métanthéline, glycopyrrolate, tropenzilium, nortriptyline, amitriptyline, imipramine, minaprine, sécovérine, AFDX-116, nicotine, alaproclate, zimélidine, déprényl et facteur de croissance des nerfs.
